# EUROPEAN PATENT APPLICATION

(11) **EP 1 142 857 A1**
(43) Date of publication of application: **10.10.2001**
(21) Application number: 99961471.2
(22) Date of filing: 28.12.1999
(51) Int. Cl.: C07C 45/68, C07C 45/64, C07C 49/248, C07C 49/258, C07C 67/31, C07C 67/343, C07C 69/73, C07C 69/732, C07C 69/734, C07D 239/52, C07D 311/76, C07F 7/18, C07B 41/06, C07B 41/04, C07B 37/00

(54) **PROCESSES FOR PRODUCING ACRYLIC ACID DERIVATIVE**

(30) Priority: 29.12.1998 JP 37735398; 22.01.1999 JP 1375999; 22.01.1999 JP 1431999; 12.03.1999 JP 6665699; 20.10.1999 JP 29825799; 08.12.1999 JP 34830299; 08.12.1999 JP 34856499; 08.12.1999 JP 34875299
(71) Applicant: NIPPON SODA CO., LTD., Chiyoda-ku, Tokyo 100-8165 (JP)
(72) Inventor: MIYAZAWA, Yasuyuki, Nihongi, Nippon Soda Co., Ltd, Niigata 949-2392 (JP); SAGAE, Takahiro, Nihongi, Nippon Soda Co., Ltd, Niigata 949-2392 (JP); ISHII, Hiroshi, Nihongi, Nippon Soda Co., Ltd, Niigata 949-2392 (JP); YAZAKI, Hiroyuki, Nihongi, Nippon Soda Co., Ltd, Niigata 949-2392 (JP); FUNABORA, Makoto, Nihongi, Nippon Soda Co., Ltd, Niigata 949-2392 (JP); TAKASE, Mitsuru, Nihongi, Nippon Soda Co., Ltd, Niigata 949-2392 (JP); IIYOSHI, Yoshiyuki, Nihongi, Nippon Soda Co.,Ltd, Niigata 949-2392 (JP); YAMAZAKI, Satoru, Nihongi, Nippon Soda Co., Ltd, Niigata 949-2392 (JP); KAWAHARA, Noriaki, Nihongi, Nippon Soda Co., Ltd, Niigata 949-2392 (JP)
(74) Representative: Schmitz, Jean-Marie
(86) International application number: JP9907397
(87) International publication number: WO0040537

(57) **Abstract**

Processes for producing a compound (1) represented by formula (1), which includes an acrylic acid derivative and is useful as an agricultural chemical or medicine. One of the processes comprises the step of formylating a compound (3) (step (1)) and the step of converting the OH of the resultant compound (2) into OR" (step (2)). The step (1) comprises reacting a formic or orthoformic ester in the presence of a Lewis acid and a base. The step (2) comprises [1] reacting the compound (2) with R" OH or with R" OH and CH(OR")₃ under acidic conditions or [2] using a phase-transfer catalyst in a two-phase system and regulating the base and the concentration thereof to stereoselectively synthesize the target compound. In another process, the compound (1) is efficiently produced without isolating the compound (2). In still another process, the compound (1) is directly produced without via the compound (2).

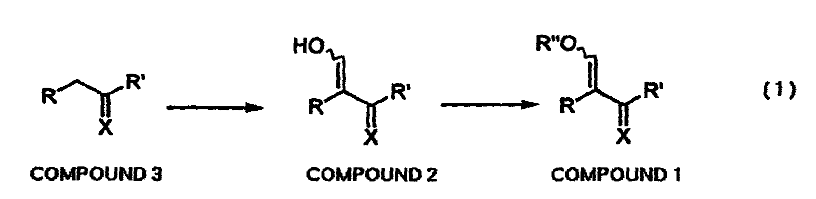

## Description

### Field of Invention

The present invention is related to a process to formylate the active methylene part in the presence of a Lewis acid and a base, a process to convert a formylated compound into an acrylic acid derivative, and a process to produce alkoxyacrylic acid derivatives useful as an agricultural chemical by employing said formylating process and said converting process.

### Background Art

There are several processes for producing α -alkoxy methylene carbonyl compounds and the derivatives (compound 1 shown below), and as one of the processes, a process containing a step to formylate an α -methylene carbonyl compound or the derivative (compound 3 shown below) to obtain a compound 2 shown below and then to alkylate the -OH part thereof as shown in the reaction formula (1) described below is considered as an advantageous process in an industrial scale since the materials and the reagents used in the process are commercially available and easy to obtain.

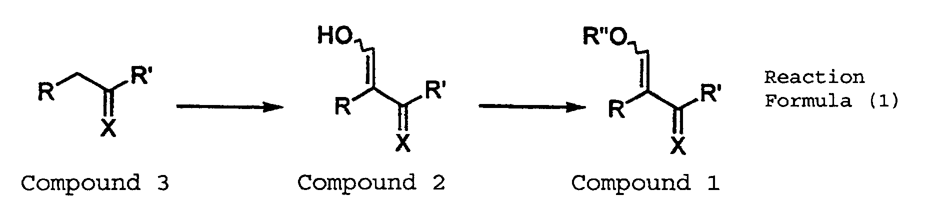

As the process to produce the compound 1 shown in the reaction formula (1), a process ① to formylate the active methylene part of phenylacetate, etc. with a formic acid ester or the like under an alkaline condition and then to alkylate the formylated product under an alkaline condition to obtain the compound 1 is known (JP laid-open 63-216848 Gazette).

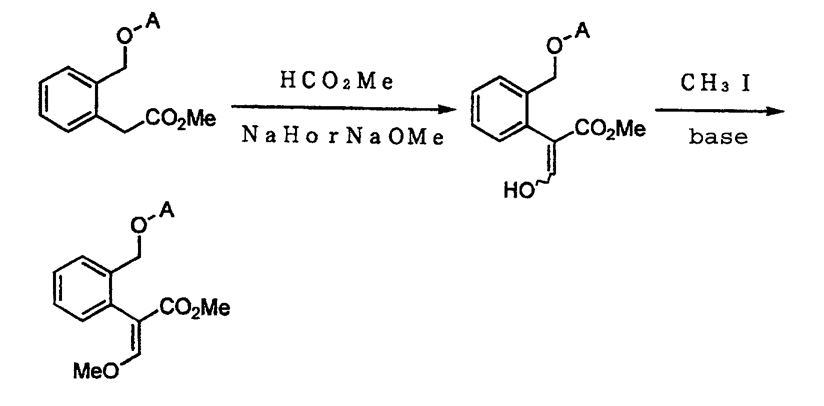

In the reaction formula above,
A represents optionally substituted alkyl, aryl or heteroaryl,

However, the process ① has a problem such that, in case of using a compound containing a substituent which causes side reaction in the molecule due to an existing base, such as methyl 2-[(2-isopropoxy-6-trifluoromethylpyridine-4-yl)oxymethyl] phenylacetate and methyl 2-chloromethylphenylacetate, the elimination or the dimerization of a functional group at the side chain start ahead, thereby disturbing reactions for such formylation and alkylation.

As a process for the formylation under no basic condition, a method ② to use Vilsmeier reagent prepared from dimethyl formamide and phosphorus oxychloride is known. However, the method ② has a problem in applying it as an industrial process that it requires to use the remarkably excess amount of the reagent to obtain the satisfactory yield.

As a process to obtain the compound 1 by using the compound 3 as a starting material without via the compound 2, a process ③ to react ketene silyl acetate with orthoformic acid ester and titanium tetrachloride to produce an acetal and then to remove the moiety of alcohol from the acetal to produce a methoxyacrylic acid derivative is known (JP laid-open 63-216848).

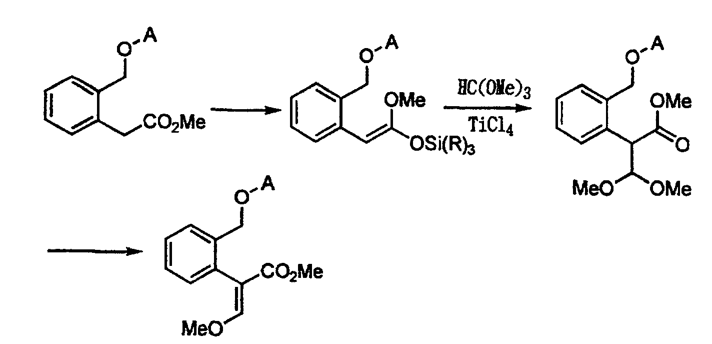

In the reaction formula above, A represents optionally substituted alkyl, aryl or heteroaryl.

However, the process ③ requires to use an expensive silylating agent to synthesize ketene silyl acetal as the starting material and it has problems of difficulty in the production due to steric hindrance caused by the adjacent substituents and less stability of the ketene silyl acetal due to its high sensitivity to moisture.

Whereas, as an acylation reaction to acylate an active methylene compound with an ester in combination with a Lewis acid or a base, such as titanium tetrachloride and triethylamine, the following reaction has been known. 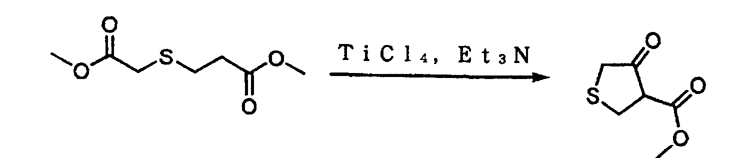 Deshmukh. M. N. et al., Synth. Commun., 1996, 26(9), 1657

However, this reaction is an intramolecular cycloacylation reaction, and no formylation reaction using a formic acid ester or the like has been known up till now.

In most case, compounds having pharmacological activity useful as an agricultural chemical or a pharmaceutical ingredient have a particular spatial configuration. For example, α -alkoxymethylene carbonyl compound has Z isomer and E isomer those which are derived on the double bond contained in the molecule, however, a compound useful as a fungicide or an insecticide for agricultural use is the E isomer (See JP 9-176136 gazette, etc.).

For producing such α-alkoxymethylene carbonyl compound, a process to alkylate the hydroxy group in an α-hydroxymethylene carbonyl compound may be proposed, for example.

However, when the alkylation reaction is operated under an ordinary condition, it is difficult in the past to selectively obtain just either one of the Z isomer or the E isomer.

M. G. Hutchings et al. have reported a process to produce an alkoxymethylene compound corresponding to the compound 1 by subjecting a phenylacetate derivative to silylation reaction in ether with methanesulfonic trimethylsilyl ester and triethylamine and then subjecting the silylated product to a reaction with titanium tetrachloride and methyl orthoformate.

However, the yield of the desired alkoxymethylene compound according to this process was unsatisfactory as low as 33.6%. Although it is disclosed in the report that the yield may be improved by replacing the reaction solvent from ether to methylene chloride, it is found by the inventor's resits that the reaction became very complicate by the replacement of the reaction solvent and no improvement in the yield was observed.

Therefore, it is an object of the present invention to provide a condition for the formylation reaction even applicable for compounds which are unstable under a basic condition and process to selectively and efficiently produce the compound 1 including acrylic acid derivatives and the like useful as an agricultural chemical by obtaining the compound 2 from the compound 3 and then converting the compound 2 into the alkoxymethylene compound.

### Disclosure of the Invention

The inventors of the present invention achieved to selectively produce the desired α-alkoxymethylene carbonyl compound (1) by reacting with either a formic acid ester or an orthoformic acid ester in the presence of either Lewis acid or a base in the step to obtain the compound 2 from the compound 3, or (1) by reacting with an alcohol represented by either R"OH or R"OH and a compound represented by CH(OR")₃ under an acidic condition or (2) by using a phase-transfer catalyst in a bilayer solvent system to fix the base and the concentration of the base in the step to obtain the compound 1 from the compound 2. Furthermore, the inventors also found that a step to directly obtain the compound 1 without isolating the compound 2 and a step to directly obtain the compound 1 from the compound 3 without via the compound 2.

Therefore, the present invention is directed to the following constitutions.
(Constitution 1) The process for producing compounds represented by a general formula (II); 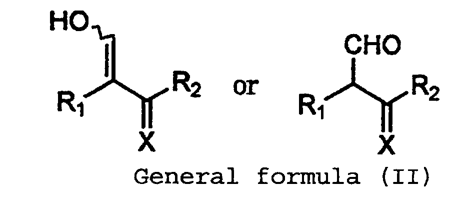 wherein R₁ represents hydrogen, halogeno, optionally substituted alkyl, optionally substituted alkoxy, a group having an alicyclic structure, R₃S(O)_{q}, R₄R₅N, R₆C(=O), nitrile, R₇C(=NR₈), optionally substituted aryl, optionally substituted aryoxy, optionally substituted heterocyclic group, optionally substituted heteroaryloxy or optionally substituted aralkyl, R₂ represents optionally substituted alkyl, optionally substituted alkoxy, a group having an alicyclic structure, optionally substituted amino, optionally substituted aryl, optionally substituted heterocyclic group or optionally substituted aralkyl, R₃, R₄ and R₅ each independently represent optionally substituted alkyl, optionally substituted aryl, optionally substituted heterocyclic group or optionally substituted aralkyl, R₆ and R₇ each independently represents optionally substituted alkyl, optionally substituted alkoxy, a group having an alicyclic structure, optionally substituted amino, optionally substituted aryl, an optionally substituted heterocyclic group or optionally substituted aralkyl, R₈ represents optionally substituted alkyl, optionally substituted alkoxy, nitrile, nitro, oprionally-substituted aryl, optionally substituted heterocyclic group or optionally substituted aralkyl, R₉ and R₁₀ each independently represents hydrogen, lower alkyl or optionally substituted aryl, provided R₁ and R₂ may bond to jointly form a ring, and X represents oxygen or NR₉R₁₀, characterized in that the compounds is produced by reacting a methylene compound represented by a general formula (I); 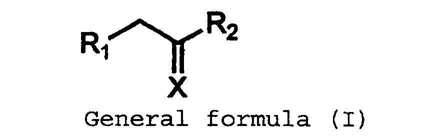 wherein R₁, R₂ and X are as defined above, with either a formic acid ester or an orthoformic acid ester in the presence of a Lewis acid and a base.
(Constitution 2) The process as defined in the constitution 1, wherein the base is a tertiary amine.
(Constitution 3) The process as defined in the constitution 1, wherein the R₁ contained in the general formula (I) represents a group represented by the following formula; 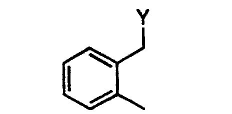 wherein Y represents a group to be eliminated when reacting with a nucleophilic reagent, optionally substituted phenoxy or optionally substituted heteroaryloxy, and R₂ represents a group represented by a formula of OR₁₁, wherein R₁₁ represents lower alkyl.
(Constitution 4) The process defined in the constitution 1, wherein the compound represented by the general formula (I) is methyl 2-[(2-isopropoxy-6-trifluoromethylpyrimidine - 4-yl) oxymethyl ]phenylacetate.
(Constitution 5) The compounds represented by the general formula (I), wherein the R₁ is a group represented by the following formula; 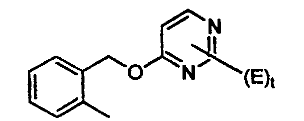 wherein E represents C₁₋₆ alkyl, C₁₋₆ haloalkyl, C₁₋₈ alkoxy, C₁₋₆ haloalkoxy, optionally substituted amino, a group represented by a formula of R₂₆S(O)p, wherein R₂₆ represents alkyl or aryl and p represents 0 or an integer of 1 or 2, optionally substituted aryl, optionally substituted aralkyl, optionally substituted aryloxy, optionally substituted heterocyclic group, optionally substituted heteroaryloxy, a group having an alicyclic structure, nitrile, nitro, alkoxycarbonyl, formyl or carboxyl, t represents 0 or an integer of 1, 2 or 3, provided E each independently represents either the same group or the different group when t is an integer of 2 or more.
(Constitution 6) The compounds represented by the general formula (I), wherein R₁ is a group represented by the following formula; 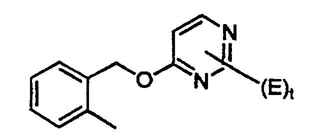 wherein E and t are as defined above.
(Constitution 7) A process for producing acrylic acid derivatives represented a general formula (III); 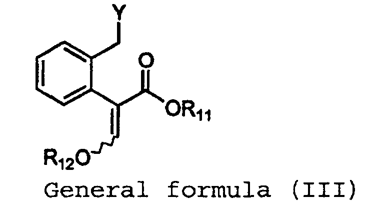 wherein Y and R₁₁ are as defined above, R₁₂ represents lower alkyl, cycloalkyl, haloalkyl, allyl, propargyl or aralkyl, characterized in that the process is constituted by a step to subject a compound represented by the general formula (I), 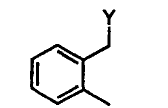 wherein R₁ is a group represented by a formula of OR₁₁, wherein R₁₁ is as defined above, and X is oxygen, to formylation process with either a formic acid ester or an orthoformic acid ester in the presence of a Lewis acid and a base, and the consequent step to convert the obtained formylated product into an alkoxymethylene.
(Constitution 8) The process for producing acrylic acid derivatives as defined in the constitution 7, wherein the base is a tertiary amine.
(Constitution 9) The process for producing acrylic acid derivatives as defined in the constitution 7, wherein the compound represented by the general formula (I) is methyl 2-[(2-isopropoxy-6-trifluoromethylpyrimidine-4-yl) oxymethyl] phenylacetate and the compound represented by the general formula (III) is methyl 3-methoxy-2-[2-{(2-isopropoxy -6-trifluoromethylpyrimidine-4-yl)oxymethyl}phenyl] acrylate .
(Constitution 10) A process for producing compounds represented by a general formula (IV); 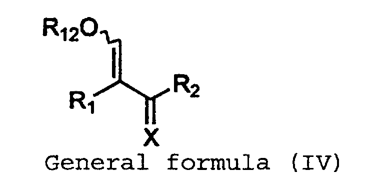 wherein R₁, R₂ and R₁₂ are as defined above, characterized in that the process is constituted by a step to subject a formyl compound represented by a general formula (II); 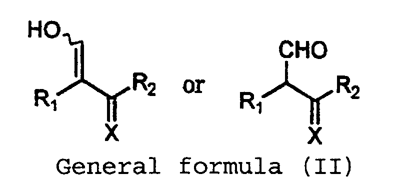 wherein R₁, R₂ and X are as defined above, to a reaction with an alcohol represented by a formula of R₁₂OH, wherein R₁₂ is as defined above, in the presence of an acid catalyst.
(Constitution 11) A process for producing compounds represented by the general formula (IV); 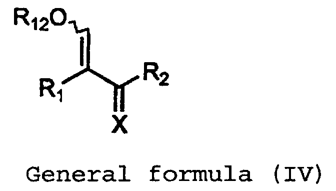 wherein R₁, R₂, R₁₂ and X are as defined above, characterized in that the process is constituted by a step to subject a formyl compound represented by the general formula (II) ; 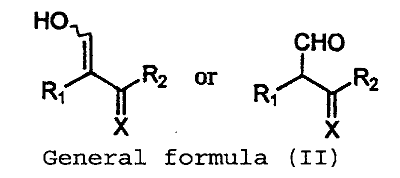 wherein R₁, R₂ and X are as defined above, to a reaction with an alcohol represented by a formula of R₁₂OH, wherein R₁₂ is as defined above, and an ortho ester represented by a formula of R₁₃C(OR₁₂)₃, wherein R₁₂ is as defined above and R₁₃ represents hydrogen, lower alkyl, cycloalkyl, haloalkyl or aralkyl.
(Constitution 12) A process as defined in the constitution 10 or the constitution 11, wherein R₁ in the general formula (II) is a group represented by the following formula; 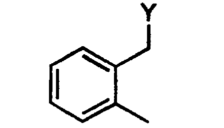 wherein Y is as defined above, and R₂ is a group represented by a formula of OR₁₁, wherein R₁₁ is as defined above.
(Constitution 13) A process as defined in the constitution 10 or the constitution 11, wherein the compound represented by the general formula (II) is methyl 3-hydroxy-2-[2-{(2-isopropoxy -6-trifluoromethylpyrimidine-4-yl)oxymethyl}phenyl] acrylate.
(Constitution 14) A process for producing compounds represented by a general formula (VI-1); 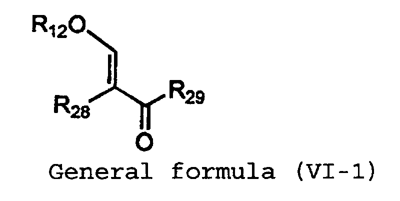 wherein R₂₈ is optionally substituted alkyl,a hydrocarbon group having an optionally substituted alicyclic structure, optionally substituted phenyl or optionally substituted heterocyclic group, R₂₉ is C₁₋₆ alkyl, C₃₋₈ cycloalkyl, hydroxyl, C₁₋₆ alkoxy, amino, a group represented by a formula of NHr₁, wherein r₁ is C₁₋₆ alkyl, C₁₋₆ alkoxy or optionally substituted phenyl, a group represented by a formula of Nr₂r₃, wherein r₂ and r₃ each independently represents C₁₋₆alkyl, C₁₋₆alkoxy or optionally substituted phenyl, a hydrocarbon group having an optionally substituted alicyclic structure, optionally substituted phenyl or optionally substituted heterocyclic group, and R₁₂ is as defined above, containing a step to O-alkylate a compound represented by a general formula (V); 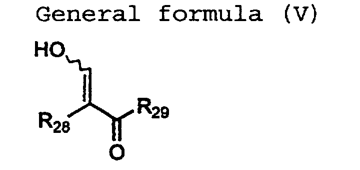 wherein R₂₈ and R₂₉ are as defined above, wherein the step to O-alkylate the compound represented by the general formula (V) contains a step to apply an alkylating agent to the compound represented by the general formula (V) in a bilayer mixed-solvent system consisting of an organic solvent and water and in the presence of a phase-transfer catalyst and any of an alkali metal hydroxide other than the lithium hydroxide, an alkali metal carbonate other than the lithium carbonate, an alkaline earth metal hydroxide and an alkaline earth metal carbonate, while maintaining the concentration of the base in the aqueous solution at 10% by weight or less.
(Constitution 15) A process for producing compounds represented by a general formula (VI-1); 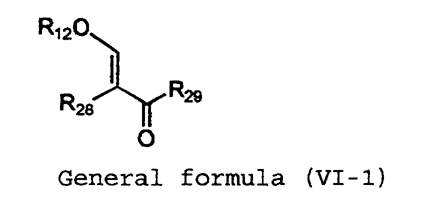 wherein R₂₈, R₂₉ and R₁₂ are as defined above, containing a step to O-alkylate a compound represented by a general formula (V) 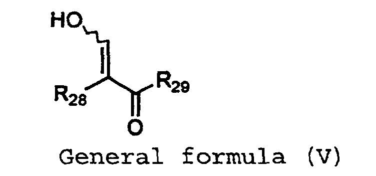 wherein R₂₈ and R₂₉ are as defined above, wherein the step to O-alkylate the compound represented by the general formula (V) contains a step to simultaneously feed dropwise an aqueous solution of any of an alkali metal hydroxide other than the lithium hydroxide, an alkali metal carbonate other than the lithium carbonate, an alkaline earth metal hydroxide and an alkaline earth metal carbonate, and a solution of the compound represented by the general formula (V) in an organic solvent into a bilayer mixed-solution system consisting of an organic solvent and water containing an alkylating agent and a phase-transfer catalyst.
(Constitution 16) A process for producing compounds represented by the general formula (VI-1) as defined in the constitution 15, wherein the step to O-alkylate the compound represented by the general formula (V) is a step to O-alkylate the compound represented by the general formula (V) while maintaining the concentration of the base in the aqueous layer at 10% by weight or less.
(Constitution 17) A process for producing compounds represented by the general formula (VI-1) as defined in the constitution 14 or the constitution 15, wherein the step to O-alkylate the compound represented by the general formula (V) is a step to O-alkylate the compound represented by the general formula (V) while maintaining the concentration of the base in the aqueous layer at 6% by weight or less.
(Constitution 18) A process for producing compounds represented by a general formula (VI-1); 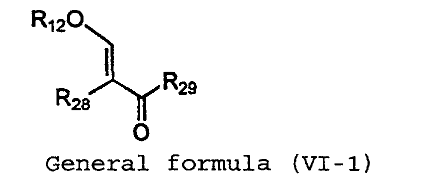 wherein R₂₈, R₂₉ and R₁₂ are as defined above, containing a step to O-alkylate a compound represented by a general formula (V); 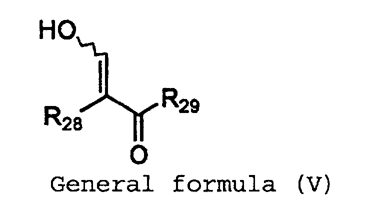 wherein R₂₈ and R₂₉ are as defined above, wherein the step to O-alkylate the compound represented by the general formula (V) contains a step to feed dropwise an aqueous solution of the alkali metal salt or the alkaline earth metal salt other than those lithium salts of the compound represented by the general formula (V) into a bilayer mixed-solvent system consisting of an organic solvent containing an alkylating agent and a phase-transfer catalyst and water.
(Constitution 19) The process for producing the compounds represented by the general formula (VI-1), wherein the step to O-alkylate the compound represented by the general formula (V) is a step to O-alkylate the compound represented by the general formula (V) while maintaining the concentration of the alkali metal salt or the alkaline earth metal salt except the lithium salt of the compound represented by the general formula (V) at 10% by weight or less.
(Constitution 20) The process for producing compounds represented by the general formula (VI-1) as defined in the constitution 14 or the constitution 15, wherein sodium hydroxide or potassium hydroxide is used as the alkali metal hydroxide.
(Constitution 21) A process for producing compounds represented by a general formula (VI-2); 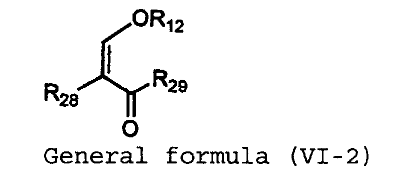 wherein R₂₈, R₂₉ and R₁₂ are as defined above, containing a step to O-alkylate compounds represented by the general formula (V); 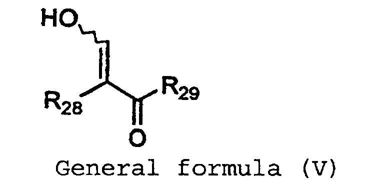 wherein R₂₈ and R₂₉ are as defined above, wherein the step to O-alkylate the compound represented by the general formula (V) contains a step to apply an alkylating agent to the compound represented by the general formula (V) in a bilayer mixed-solvent system in the presence of a phase-transfer catalyst and either lithium hydroxide or lithium carbonate.
(Constitution 22) The process for producing compounds represented by a general formula (VI-2) as defined in the constitution 21, wherein the step to O-alkylate the compounds represented by the general formula (V) is a step to O-alkylate the compounds represented by the general formula (V) while maintaining the concentration of either lithium hydroxide or lithium carbonate in the aqueous solution at 5% by weight or more.
(Constitution 23) The process for producing compounds represented by the general formula (VI-1) as defined in the constitutions 14, 15, 17 or 21, wherein a quaternary ammonium salt is used as the phase-transfer catalyst.
(Constitution 24) The process for producing compounds represented by the general formula (VI-1) as defined in the constitutions 14, 15, 17 or 21, wherein a quaternary ammonium hydroxide is used as the phase-transfer catalyst.
(Constitution 25) A process for producing compounds represented by a general formula (XII); 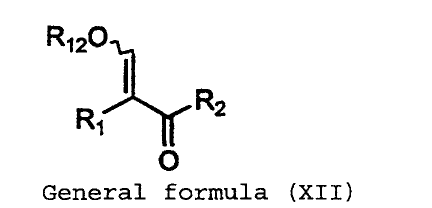 wherein R₁, R₂ and R₁₂ are as defined above, characterized in that the process is constituted by a step to subject a compound represented by a general formula (VII) 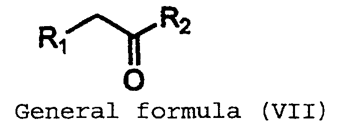 wherein R₁ and R₂ are as defined above, to a reaction with a tertiary amine compound represented by a general formula (VIII); 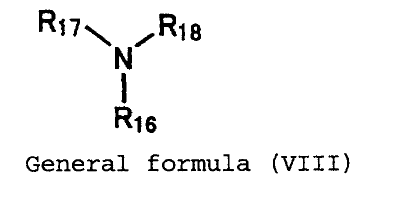 wherein R₁₆, R₁₇ and R₁₈ may be same or different one another and esch represents alkyl, aryl or aralkyl, and an organic silicon compound represented by a general formula (IX); 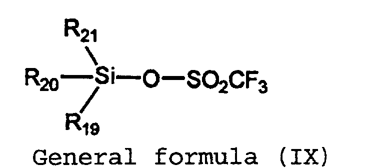 wherein R₁₉, R₂₀ and R₂₁ may be same or different one another and each represents alkyl, aryl or aralkyl, to obtain a silylenol ether represented by a general formula (X); 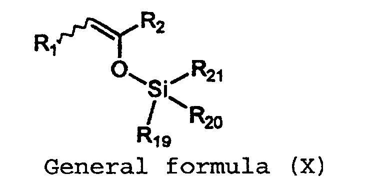 wherein R₁, R₂, R₁₉, R₂₀ and R₁₂ are as defined above, which may be obtained by removing the resulting trifluoromethanesulfonate thereform, and the subsequent step to subject the obtained silylenol ether to a reaction with an orthoformic acid ester compound represented by a general formula of (XI)CH(OR₁₂)₃, wherein R₁₂ is as defined above, in the presence of a Lewis acid.
(Constitution 26) The process as defined in the constitution 25, wherein the group represented by R₁ in the compounds represented by the general formula (VII) is a group represented by the following formula; 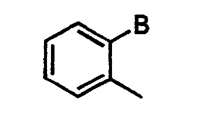 wherein B represents hydrogen, lower alkyl, lower alkoxy, haloalkyl, optionally substituted arylsulfonyloxyalkyl or optionally substituted lower alkylsulfonyloxyalkyl, and the group represented by R₂ in the compound represented by the general formula (VII) represents a group represented by a formula of OR₂₃, wherein R₂₃ represents lower alkyl, and each of B and R₂₃ are a group capable of bonding to jointly form a ring.
(Constitution 27) A process for producing α-alkoxymethylenecarbonyl compounds represented by a general formula (XV) ;
   General formula (XV)
   wherein R₂₄ represents nitro, cyano, halogeno, C₁₋₆ alkyl, C₁₋₆ alkoxy, C₁₋₆ haloalkyl or C₁₋₆ alkoxycarbonyl, n represents 0 or an integer 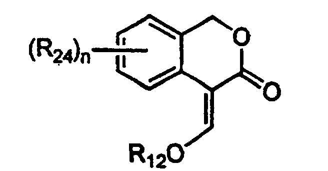 of 1-4, provided R₂₄ may be same or different group when n is 2 or more, and R₁₂ and n are as defined above, characterized in that the process contains a step to obtain α -hydroxymethylenecarbonyl compounds represented by a general formula (XIV); 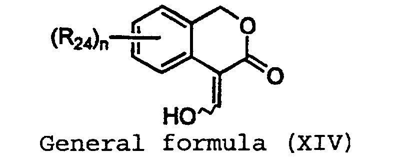 wherein R₂₄ and n are as defined above, by formylating an isochromanone compound represented by a general formula (XIII) ; 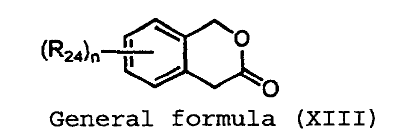 wherein R₂₄ and n are as defined above, and a step to O-alkylate the obtained α-hydroxymethylenecarbonyl compound in a bilayer mixed-solvent system consisting of an organic solvent and water in the presence of a phase-transfer catalyst and a base without isolating the compound represented by the general formula (XIV).
(Constitution 28) The process for producing compounds represented by a general formula (XV) as defined in the constitution 27, wherein the step to formylate the isochromanone compound represented by the general formula (XIII) is a step to formylate the compound represented by the general formula (XIII) by using a formic acid ester.
(Constitution 29) A process for after-treatment, characterized in that the process is constituted by a step to add water following to the addition of an organic acid containing 1-4 carbon atoms to the reaction solution to improve the dispersibility thereof in the after-treatment for the process to produce the compound represented by the general formula (II); 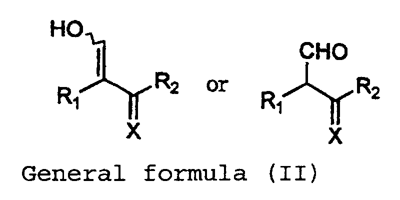 wherein R₁, R₂ and X are as defined above, by reacting a methylene compound represented by the general formula (I); 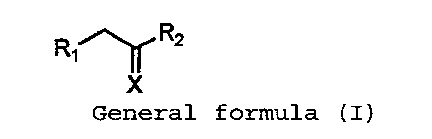 wherein R₁, R₂ and X are as defined above, with either a formic acid ester or an orthoformic acid ester in the presence of a Lewis acid and a base.
(Constitution 30) The process for after-treatment as defined in the constitution 29 characterized by using an organic acid containing 1-4 carbon atoms as the Lewis acid at a rate of 2.5 mole or more.
(Constitution 31) The process for after-treatment as defined in the constitution 29, wherein the organic acid containing 1-4 carbon atoms is acetic acid.
(Constitution 32) The process for after-treatment as defined in the constitution 29, wherein the Lewis acid is titanium tetrachloride.
(Constitution 33) The process for after-treatment as defined in the constitution 29, wherein the base is triethylamine.
(Constitution 34) The process for after-treatment as defined in the constitution 29, wherein the the group represented by R₁ in the compound represented by the general formula (I) is a group represented by the following formula; 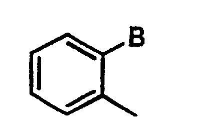 wherein B is as defined above, and the R₂ in the compound of the general formula (I) is a group represented by a formula of OR₂₃, wherein R₂₃ is as defined above, and each of B and R₂₃ may be a group capable of bonding to jointly form a ring.

### Regarding Constitutions 1 through 9:

For the compounds represented by the general formula (I), as examples for the group represented by R₁, hydrogen, halogene, such as chlorine and fluorine, optionally substituted alkyl, such as methyl, isopropyl, methoxymethyl, methylthiomethyl, chloromethyl, trifluoromethyl, trichloromethyl and monofluoromethyl, optionally substituted alkoxy, such as methoxy, isopropoxy, benzyloxy and trifluoromethoxy, a group containing an alicyclic structure, such as cyclopropyl and cyclohexyl, a group represented by a formula of R₃S(O)q, such as methylsulfenyl and methylsulfonyl, a group represented by a formula of R₄R₅N, such as dimethylamino, a group represented by a formula of R₆C(=O), such as acetyl, nitrile, nitro, a group represented by a formula of CH₃C(=NCH₃), optionally substituted aryl, such as phenyl, 4-methoxyphenyl and 2,4-dichlorophenyl, optionally substituted aryloxy, such as phenoxy and 4-chlorophenoxy, optionally substituted heterocyclic group, such as 2-pyridyl, 6-chloro-2-pyridyl and 4-tetrahydropyranyl, optionally substituted heteroaryloxy, such as 2-pyridyloxy and 1,3-dimethyl-5-pyrazoloxy and optionally substituted aralkyl, such as benzyl and 4-chlorobenzyl, can be given.

Whereas, R₆ and R₇ each independently represents optionally substituted alkyl, optionally substituted alkoxy, optionally substituted amino, optionally substituted aryl or optionally substituted aralkyl, R₃, R₄ and R₅ each independently represents optionally substituted alkyl, optionally substituted aryl or optionally substituted aralkyl, R₈ represents optionally substituted alkyl, optionally substituted alkoxy, nitrile, nitro, optionally substituted aryl or optionally substituted aralkyl, and q represents 0 or an integer of 1 or 2.

For the compounds represented by the general formula (I), as examples for the group represented by R₂, optionally substituted alkyl, such as methyl, isopropyl, methoxymethyl, methylthiomethyl, chloromethyl, trifluoromethyl, trichloromethyl and monofluoromethyl, optionally substituted alkoxy, such as methoxy, isopropoxy, benzyloxy and trifluoromethoxy, a group containinh an alicyclic structure, such as cyclopropyl and cyclohexyl, optionally substituted amino, such as dimethylamino, methoxymethylamino and amino, optionally substituted aryl, such as phenyl, 4-methoxyphenyl and 2,4-dichlorophenyl, optionally substituted heterocyclic group, such as 2-pyridyl, 6-chloro-2-pyridyl and 4-tetrahydropyranyl, and optionally substituted aralkyl, such as benzyl and 4-chlorobenzyl, can be given.

In the compounds represented by the general formula (I), X represents oxygen or a group represented by a formula of NR₉R₁₀, and R₉ and R₁₀ each independently represents hydrogen, lower alkyl or optionally substituted aryl.

As the definite examples for the compounds represented by the general formula (I), the following compounds can be given.

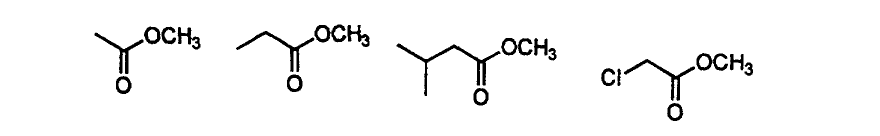

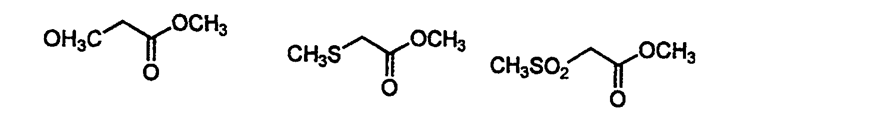

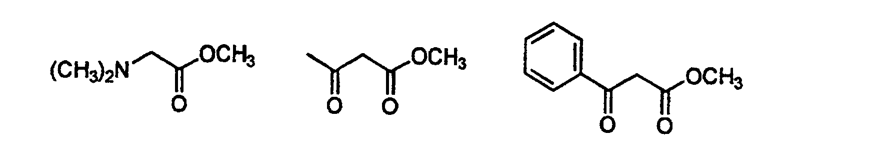

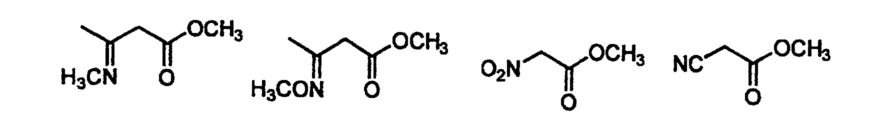

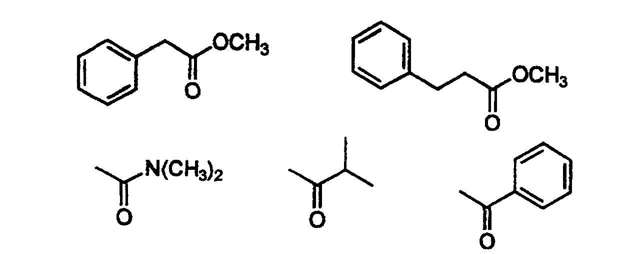

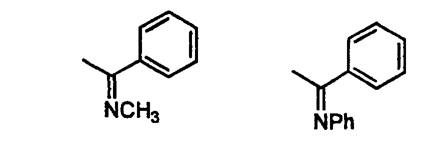

Among the compounds represented by the general formula (I), preferable results can be obtained particularly when using the compounds, in which the group represented by R₁ is a group represented by the following structure; 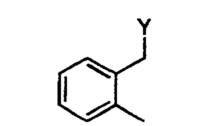 wherein Y represents an eliminating group when it is reacted with a nucleophilic reagent, optionally substituted phenoxy or optionally substituted heteroaryloxy, and the group represented by R₂ is a group represented by a formula of OR₁₁, wherein R₁₁ represents lower alkyl. As described above, the reason to use such compounds is because of the unstable property of the compounds represented by the general formula (I) under a basic condition and the compounds easily induce elimination reaction and dimerization reaction. As the examples for the group represented by Y, halogeno, such as chlorine, bromine and iodine, sulfonyloxy, such as methanesulfonyloxy, p-toluenesulfinyloxy and trifluoromethanesulfonyloxy, trialkylammonium, 4-chlorophenoxy, 2-methylphenoxy, 3-phenoxyphenoxy, 6-trifluoromethyl-2-pyridyloxy and the like can be given. As the definite examples for the compounds represented by the general formula (I), the following compounds can be further given.

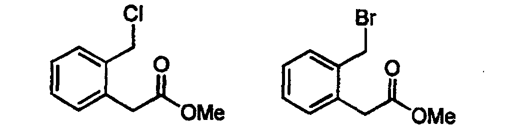

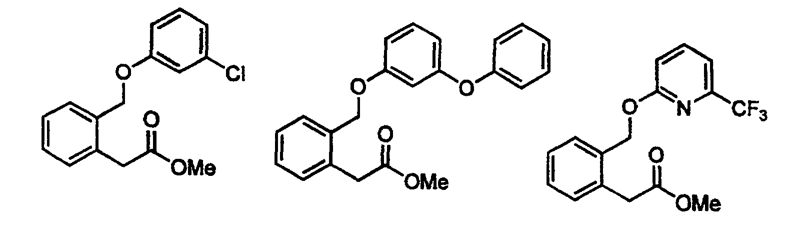

Among the compounds represented by the general formula (I), the ones in which the group represented by R₁ is a group represented by the following structure; 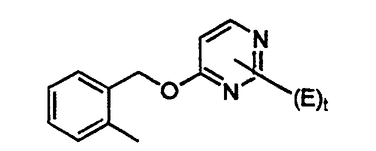 wherein E represents C₁₋₆ alkyl, C₁₋₆ haloalkyl, C₁₋₆ alkoxy, C₁₋₆ haloalkoxy, optionally substituted amino, a group represented by a formula of R₂₆S(O)p, wherein R₂₆ represents alkyl or aryl, and p represents 0, 1 or 2, optionally substituted aryl, optionally substituted aralkyl, optionally substituted aryloxy, optionally substituted heterocyclic group, a group containing an alicyclic structure, nitrile, nitro, alkoxycarbonyl, formyl or carboxyl, and t represents 0 or an integer of 1-3, provided E independently represents same or different group when it is an integer of 2 or more, are novel compounds and are useful as an intermediate to be used for the production of agricultural chemicals.

As the definite examples for the group represented by E in the general formula (I), C₁₋₆ alkyl, such as methyl, ethyl, isopropyl and s-butyl, C₁₋₆ haloalkyl, such as chloroethyl, trichloromethyl, fluoroethyl and trifluoromethyl, C₁₋₆ alkoxy, such as methoxy, ethoxy and isopropoxy, optionally substituted amino, such as amino, dimethylamino and methoxyamino, a group represented by a formula of R₂₆S(O)p, such as methanesulfenyl, methanesulfonyl, phenylsulfeny, and phenylsulfonyl, optionally substituted aryl, such as phenyl and 4-chlorophenyl, optionally substituted aralkyl, such as benzyl and 4-methoxyphenylmethyl, optionally substituted aryloxy, such as phenoxy and 4-methylphenoxy, optionally substituted heterocyclic group, such as 2-pyridyl, 2-imidazolyl, 3-isoxazole and 3-isooxazoline, a group containing an alicyclic structure, such as cyclohexyl and cyclopropyl, nitrile, nitro, formyl, alkoxycarbonyl, such as methoxycarbonyl and t -butoxycarbonyl, and carboxyl can be given. In the examples above, there is no limitation in the number and the position for the substituents, and the definite examples for the compounds represented by the general formula (I) are shown in the following.

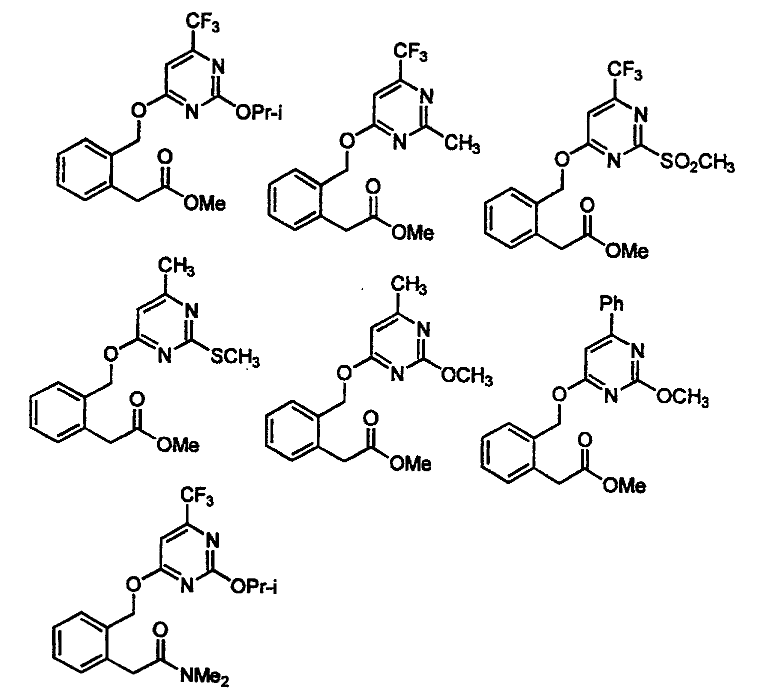

The step to obtain the compound represented by the general formula (II) by formylating the compound represented by the general formula (I) is carried out by admixing a Lewis acid, a substrate for the reaction and either a formic acid ester or an orthoformic acid ester with the solvent and then adding a base into the resulting mixture. The admixing rate of the Lewis acid to the substrate for the reaction is in a range of from 1.0 to 3.0 equivalents and is preferably in a range of from 1.2 to 2.0 equivalents. Although there is no limitation in the type of the Lewis acid, it is preferable to use any of aluminium chloride, aluminium methylchloride, titanium tetrachloride, tin tetrachloride, ferric chloride, zinc chloride, trifluoroboron diethyl ether and the like, and it is most preferable to use titanium tetrachloride.

As the solvent to be used in the step described above, any aprotic solvent containing no active methylene part can be used without limitation, however, it is preferable to use a chlorine-base solvent, such as methylene chloride, chloroform and chlorobenzene. The rate of the solvent to be uses in said step is in a range of from 0 to 10 liter/mole to the reaction substrate, and preferably in a range of from 0.5 to 1.5 liter/mole.

There is no limitation for the part of the formic acid ester or the orthoformic acid ester to be used in the step described above, however, it is preferable to use a formic acid ester or an orthoformic acid ester, which is useful for all purposes. More definitely, methyl formate, ethyl formate, methyl orthoformate, ethyl orthoformate and the like can be given as the example. The rate of the formic acid ester or the orthoformic acid ester to be used to the reaction substrate is in a range of from 1.0 to 10.0 equivalents, and preferably in a range of from 1.2 to 2.0 equivalents.

There is no limitation for the base to be used in the step described above, and any organic and inorganic bases can be used as the base. Particularly, it is preferable to use an organic base for carrying out the reaction in a homogenous system, and it is more preferable to use a tertiary amine among the organic bases. As examples for such tertiary amine, triethylamine, tri(n-butyl)amine, diisopropylethylamine and the like can be given. The rate of the base to be used in the step described above to the reaction substrate is in a range of from 2.0 to 6.0 equivalents, and preferably in a range of from 2.4 to 4.0 equivalents. Particularly, it is preferable to use the base at a rate of 2 equivalents to titanium tetrachloride when titanium tetrachloride is used as a Lewis acid.

The reaction is proceeded under a temperature in a range of from-15 to 20°C, and preferably from-10 to 10°C, following to admixing a reaction substrate, a Lewis acid and either a formic acid ester or an orthoformic acid ester at a temperature in a range of from -20 to 20°C, and preferably from -10 to 10°C, and then adding a base at a temperature in a range of from -15 to 20°C, and preferably from -10 to 10°C. There is no limitation in the method to admix the reaction substrate, the Lewis acid and either of the formic acid ester or the orthoformic acid ester when they are admixed under a low temperature, and a process to add either of the formic acid ester or the orthoformic acid ester into the Lewis acid solution and then to further add the reaction substrate into the solution can be given, for example.

Following to the reaction and subsequent after-treatment, the formy lated product can be obtained. By measuring various spectrums, it is found that the formylated product is an equilibrium mixture in the following structures, and is obtained as a main product in

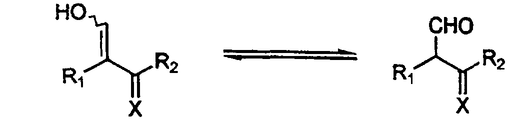

Among the compounds represented by the general formula (II), the compounds in which group represented by R₁ is a group represented by the following formula; 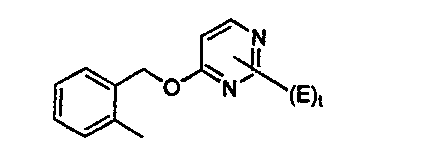 wherein E and t are as defined above, are novel compounds and are useful as an intermediate for agricultural chemicals and pharmaceutical ingredients. As examples for the group represented by E, the ones similar to the functional groups as described above can be given. The compounds represented by the following chemical structures are given as the examples for such compounds.

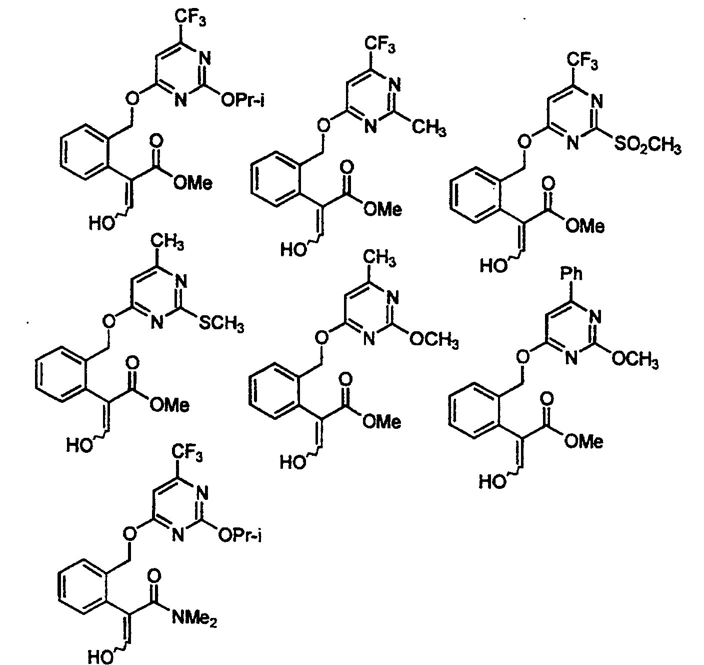

Acrylic acid derivatives useful as an agricultural chemical, a pharmaceutical ingredient and their intermediate and represented by the general formula (III); 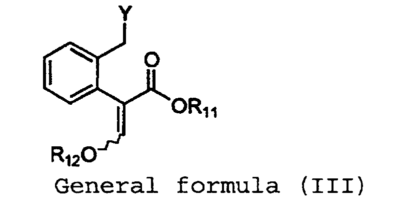 wherein Y and R₁₁ are as defined above, R₁₂ represents a lower alkyl, cycloalkyl, haloalkyl, allyl, propargyl or aralkyl, are efficiently prepared by formylating a compound represented by the general formula (I), wherein the group represented by R₁ is a group represented by the following formula; 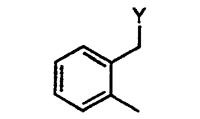 wherein Y is as defined above, the group represented by R₂ is a group represented by a formula of OR₁₁, wherein R₁₁ is as defined above, and X represents oxygen, with either of a formic acid ester or an orthoformic acid ester in the presence of a Lewis acid and a base, and then converting the formylated product into the form of alkoxymethylene. As examples for the group represented by R₂, lower alkyl, such as methyl, ethyl, isopropyl and methoxymethyl; cycloalkyl, such as cyclohexyl and cyclopropyl; haloalkyl, such as chloroethyl and 2,2,2-trifluoroethyl; allyl, propargyl and aralkyl, such as benzyl and 2-pyridylmethyl, can be given.

### Regarding Constitutions 28 through 34;

The after-treatment process for the reaction described above is characterized by adding water following to the addition of an organic acid containing 1-4 carbon atoms into the reacted solution, and the process can improve the separating property of the solution.

The after-treatment process is an effective means as the after-treatment for aldol reaction or Claisen condensation reaction in an organic solvent using a Lewis acid and a Lewis base.

Aldol reaction and Claisen condensation reaction are useful as carbon-carbon bond formation reaction, and particularly the reaction system using a Lewis acid and a Lewis base is well employed in view of good operational easiness and mild reaction condition.

The reaction is normally proceeded by adding a Lewis acid and a Lewis base into an ester to be reacted with an ester or a ketone, a ketone or an aldehyde under a low temperature. In this case, the reacted product is obtained in a form that the Lewis acid worked for the reaction being coordinated to the reacted product due to the reason that the reacted product takes the β -ketoester structure or the *β*-hydroxyketone structure.

Namely, in the process of the reaction described above, the reacted product is hydrolyzed to eliminate the Lewis acid therefrom, then giving the desired final product. However, there are some cases that the reacted product, a complex with the Lewis acid, is hardly hydrolyzed owing to the structure of the reacted product. Furthermore, hydrolysis of the Lewis acid itself is insufficient, and the hydrolyzed product may condense each other to form the oligomer. When adding an organic solvent to extract the desired product from a layer of the added organic solvent under such situation, the solution sometimes makes emulsion, thereby the separation of the solution becomes difficult since the hydrolyzed product may act as a kind of emulsifier. Further, during the after-treatment for the reaction, the side reactions such that the hydroxide group of the obtained aldol be eliminated and the obtained ketoester be deacylated, are caused to lower the yield of the desired product, and the separating property of the reaction system is not always improved depending upon the chemical structure of the reacted product, when making the pH of the reaction system to extremely either acidic or basic in order to accelerate the hydrolysis. From this reason, a phosphate buffer solution or the like being kept at a pH value of 7 have been used for the after-treatment in the past.

However, the cost of the phosphate buffer is expensive and the use of the phosphate buffer in an industrial scale has a limitation in view of waste water treatment issue.

Therefore, it is an object of the present invention to provide a treatment process in an industrial scale capable of accelerating the hydrolysis and improving the separation efficiency in the after-treatment for the aldol reaction and the Claisen condensation reaction, which requires less cost, causing no waste water problem and is easy to operate, and the inventors of the present invention found that the separation efficiency can be remarkably improved by adding acetic acid in a fixed amount into the reacted solution prior to the treatment of the reacted solution with water.

As the substrate for the aldol reaction, various types of ketones can be used, and the definite examples for the ketones, acetophenone, phenyl ethyl ketone, phenylacetoxy methyl ketone, ethyl methyl ketone, n-propyl ethyl ketone, cyclohexanone and the like can be given. Whereas, as the examples for the compound to be reacted with the substrate, various types of ketones and aldehydes, including acetophenone, phenyl ethyl ketone, phenacyl chloride, diethyl ketone, cyclohexanone, benzaldehyde, isopropylaldehyde, n-propylaldehyde and the like, can be given.

For the substrate to be used in the Claisen condensation reaction, various esters of which α -position is methylene can be used. More definitely, phenyl acetate alkyl ester, 3-phenylpropionic acid ester, adipic acid ester and the like can be given as the example. And, for the compound to be subjected to the reaction with the substrate, various esters, particularly the ones of which α -position is not methylene, can be used, and as examples for such esters, benzoic acid esters, formic acid esters, α-chloropropionic acid esters and the like can be given.

Particularly, it is useful to employ the after-treatment process according to the present invention when synthesizing α -formyl-substituted phenylacetic acid ester by employing Claisen condensation reaction with using a substituted phenylacetic acid ester as the substrate and a formic acid ester as the compound to be subjected to the reaction with the substrate.

For the organic solvent to be used for the aldol reaction and the Claisen condensation reaction, any solvents causing no adverse effect on the Lewis acid to be used can be given without limitation, and as examples for the solvent, chlorine-containing solvents, such as methylene chloride, chloroform and chlorobenzene, hydrocarbon-base solvents, such as benzene, toluene, hexane and cyclohexane, can be given. These solvents are usable alone or in combination of 2 or more thereof. However, it is particularly preferable to use methylene chloride or chlorobenzene.

For the Lewis acid used in the aldol reaction and the Claisen condensation reaction, any Lewis acids are usable without limitation, however, it is preferable to use a Lewis acid capable of coordinating at more than two positions. As the definite examples for such Lewis acid, boron trifluoride, aluminium chloride, methyldichloroaluminium, dimethylchloroaluminium, trimethylaluminium, nagnesium chloride, magnesium bromide, titanium tetrachloride, dichlorotitanium bis-triflate, bis-cyclopentadienyltitanium bis-triflate, dichlorotitanium bisfluorosulfonate, tin tetrachloride, tin(II)bistriflate and the like can be given. And, titanium tetrachloride is usable in combination with methanesulfonate trimethylsilyl ether in a catalytic amount.

For the base used for the aldol reaction and the Claisen condensation reaction, any base can be used, and as examples for the base, triethylamine, tributylamine, ethyldiisopropylamine, tetramethylenediamine, N-ethylpiperidine, diazabicyclo[3,3,0]octane and the like can be given.

The combination of the Lewis acid and the base may be appropriately selected corresponding to the compounds used for a reaction, and it is particularly preferable to use titanium tetrachloride and triethylamine in combination.

For the organic acid containing 1-4 carbon atoms to be used for the after-treatment, acetic acid, propionic acid, butyric acid an the like can be used, however, it is more preferable to use acetic acid in view of waste water treatment and the cost when considering the use in an industrial scale.

The aldol reaction and the Claisen condensation reaction in the present invention is proceeded by dissolving a reaction substrate and a compound to be reacted with the substrate in an organic solvent, adding a Lewis acid under a low temperature and further adding a Lewis base to subject the solution to a reaction and subsequently to the after-treatment. Normally, it is preferable to use the compound to be reacted with the substrate at a rate of 1.0-1.2 equivalent to the amount of the substrate, the Lewis acid at a rate of 1-1.5 equivalent to the same, and the Lewis base at a rate of 2-3 equivalent to the same. The reaction is proceeded at a temperature in a range of from -70 to 40°C. The reaction is halted when the raw materials are disappeared, however, it should be noted that the reaction system may be changed to suspension-like state and the separating operation for an organic layer and an aqueous layer in the following step could be difficult if adding water into the system fast or pouring the system into water as done in other ordinary reactions. The characteristic of the present invention is to add an organic acid containing 1-4 carbon atoms at first and then add water, which remarkably improve the separation property of the reaction system in the following separation step. The detailed reason for such improvement is unknown yet, but it is estimated that the subsequent hydrolysis can be easily advanced owing to the substitution with an acetoxy group onto the Lewis acid.

The rate of the organic acid to be used is preferably 2.5 mole or more to the amount of the Lewis acid. The performance to separate the solution will be insufficient when the rate of the organic acid is less than 2.5 mole, and sufficient effect could be gained when the rate is at 2.5 mole or more. However, using the organic acid in an amount more than 2.5 mole would not give substantial difference in the separation performance. The addition of the organic acid may cause some thermal elevation so that it is better to prevent to cause quick temperature elevation. It should be noted that the separation performance is not improved by the addition of an aqueous solution of the organic acid.

### Regarding Constitutions 10 - 11;

The formylated compound represented by the general formula (II) obtainable according to the process described above is converted to the alkoxymethylene form by subjecting it to an alkylation process under a basic condition. For example, the formylated compound can be alkylated by using sodium hydride, t-butoxy potassium, sodium hydroxide and the like as a base and an alkylating agent, such as methyl iodide and dimethylsulfate, in any of an organic solvent, a polar solvent, such as water, alcohol and dimethylformamide, and a mixed solvent of an organic solvent and water.

Then, the alkylated compound can be converted to the alkoxymethylene form by subjecting the alkylated compound to a reaction with either an alcohol having a formula of R₁₂OH or both of an alcohol having a formula of R₁₂OH and an orthoester having a formula of R₁₃C(OR₁₂)₃ not only under a basic condition but also under an acidic condition.

The process described above is effective when it is applied to the compounds represented by the general formula (II) wherein R₁ 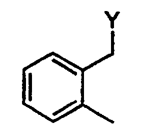 is a group represented by the following formula;
wherein Y is as defined above, and R₂ is a group represented by a formula of OR₁₁, wherein R₁₁ is as defined above, and more specifically to methyl 3-hydroxy-2-[2-{(2-isopropoxy-6 -trifluoromethylpyrimidine-4-yl)oxymethyl}phenyl]acrylate.

The process described above is applicable not only to the formylated compompounds represented by the general formula (II) but also to the acylated compounds as represented by the following chemical formulas; 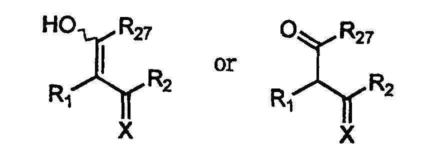 wherein R₁, R₂ and X are as defined above, and R₂₇ represents hydrogen, alkyl, cycloalkyl, alkenyl, alkynyl, aryl, heteroaryl, aralkyl or cycloalkylalkyl.

The examples for the compounds represented by the general formula (II), wherein the group represented by R₁ is a group represented by the following formula; 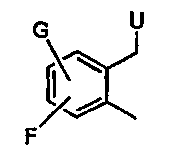 wherein U represents a group that eliminates therefrom when reacting with a nucleophilic reagent, G and F may be same or dofferent and represents hydrogen, halogeno, alkyl, cycloalkyl, alkenyl, alkynyl, haloalkyl, alkoxyalkyl, aryl, heteroaryl, aralkyl, alkoxy, haloalkoxy, allyloxy, propargyloxy, arylalkoxy, aryloxy, heteroaryloxy,, acyloxy, amino, arylazo, acylamino, nitro, cycno, or a group represented by a formula of -CO₄₂R₄₃, -CONR₄₄R₄₅, -COR₄₆, -CR₄₇=NR₄₈ or -N=CR₄₉R₅₀, G and F may form an aliphatic or aromatic condensed ring or a heterocyclic ring containing at least one hetero atom when D and F are adjoining, R₄₃ through R₄₇, R₄₉ and R₅₀ may be same or different and represent hydrogen, alkyl, cycloalkyl, alkenyl, alkynyl, aryl, heteroaryl, aralkyl or cycloalkylalkyl, and R₄₈ represents hydrogen, alkyl, cycloalkyl, alkenyl, alkynyl, aryl, heteroaryl, aralkyl, cycloalkylalkyl or alkoxy, and R₂ represents a group represented by a formula of OR₁₁, wherein R₁₁ is as defined above, are shown in the following.

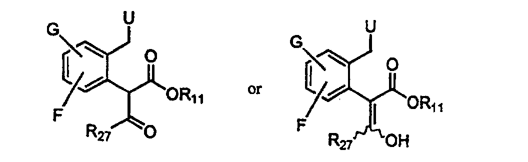

| Compound No. | U | G | F | R₁₁ | R₂₇ |
|---|---|---|---|---|---|
| 1 | Cl | H | H | Me | H |
| 2 | Br | H | H | Me | H |
| 3 | Cl | 3-Me | H | Me | H |
| 4 | Cl | 3-Me | 4-Me | Me | H |
| 5 | Cl | 3-Me | 4-Cl | Me | H |
| 6 | Cl | 3-Me | 4-OMe | Me | H |
| 7 | Cl | 3-CH=CH2 | H | Me | H |
| 8 | Cl | Acetylene | H | Me | H |
| 9 | Cl | 4-CH₂Cl | H | Me | H |
| 10 | Cl | 4-CH₂OCH₃ | H | Me | H |
| 11 | Cl | 4-(4-Cl-C₆H₅) | H | Me | H |
| 12 | Cl | 4-(2-pyridyl) | H | Me | H |
| 13 | Cl | 4-OCH₂CH=CH₂ | H | Me | H |
| 14 | Cl | 4-OCH₂Ph | H | Me | H |
| 15 | Cl | 4-propargyloxy | H | Me | H |
| 16 | Cl | 4-NMe₂ | H | Me | H |
| 17 | Cl | 4-acetyloxy | H | Me | H |
| 18 | Cl | 4-phenylazo | H | Me | H |
| 19 | Cl | 4-acetylamino | H | Me | H |
| 20 | Cl | 4-NO₂ | H | Me | H |
| 21 | Cl | 4-CN | H | Me | H |
| 22 | Cl | 4-CO₂Me | H | Me | H |
| 23 | Cl | 4-CONMe₂ | H | Me | H |
| 24 | Cl | 4-COMe | H | Me | H |
| 25 | Cl | 4-CH=NPh | H | Me | H |
| 26 | Cl | 4-N=CHMe₂ | H | Me | H |
| 27 | Cl | 4-CH=NOH | H | Me | H |
| 28 | Cl | 4,5-(-N=CH=CH=CH-) | | Me | H |
| 29 | Cl | 4,5-(-N=CH-NH-) | | Me | H |
| 30 | Cl | H | H | Me | Me |
| 31 | Cl | 3-Me | H | Me | Me |
| 32 | Cl | 3-Me | 4-Me | Me | Me |
| 33 | Cl | 3-Me | 4-Cl | Me | Me |
| 34 | Cl | 3-Me | 4-OMe | Me | Me |
| 35 | Cl | 3-CH=CH₂ | H | Me | Me |
| 36 | Cl | 3-acetylene | H | Me | Me |
| 37 | Cl | 4-CH₂Cl | H | Me | Me |
| 38 | Cl | 4-CH₂OCH₃ | H | Me | Me |
| 39 | Cl | 4-(4-Cl-C₆H₅) | H | Me | Me |
| 40 | Cl | 4-(2-pyridyl) | H | Me | Me |
| 41 | Cl | 4-OCH₂CH=CH₂ | H | Me | Me |
| 42 | Cl | 4-OCH₂Ph | H | Me | Me |
| 43 | Cl | 4-propargyloxy | H | Me | Me |
| 44 | Cl | 4-NMe₂ | H | Me | Me |
| 45 | Cl | 4-acetyloxy | H | Me | Me |
| 46 | Cl | 4-phenylazo | H | Me | Me |
| 47 | Cl | 4-actylamino | H | Me | Me |
| 48 | Cl | 4-NO₂ | H | Me | Me |
| 49 | Cl | 4-CN | H | Me | Me |
| 50 | Cl | 4-CO₂Me | H | Me | Me |
| 51 | Cl | 4-CO2NMe₂ | H | Me | Me |
| 52 | Cl | 4-COMe | H | Me | Me |
| 53 | Cl | 4-CH=NPh | H | Me | Me |
| 54 | Cl | 4-N=CMe₂ | H | Me | Me |
| 55 | Cl | 4-CH=NOH | H | Me | Me |
| 56 | Cl | 4,5-(-N=CH=CH=CH-) | | Me | Me |
| 57 | Cl | 4,5-(-N=CH-NH-) | | Me | Me |
| 58 | OTos | H | H | Me | H |
| 59 | OTos | 3-Me | H | Me | H |
| 60 | OTos | 3-Me | 4-Me | Me | H |
| 61 | OTos | 3-Me | 4-Cl | Me | H |
| 62 | OTos | 3-Me | 4-OMe | Me | H |
| 63 | OTos | 3-CH=CH₂ | H | Me | H |
| 64 | OTos | 3-acetylene | H | Me | H |
| 65 | OTos | 4-CH₂Cl | H | Me | H |
| 66 | OTos | 4-CH₂OCH₃ | H | Me | H |
| 67 | OTos | 4-(4-Cl-C₆H₅) | H | Me | H |
| 68 | OTos | 4-(2-pyridyl) | H | Me | H |
| 69 | OTos | 4-OCH₂CH=CH₂ | H | Me | H |
| 70 | OTos | 4-OCH₂Ph | H | Me | H |
| 71 | OTos | 4-propargyloxy | H | Me | H |
| 72 | OTos | 4-NMe₂ | H | Me | H |
| 73 | OTos | 4-acetyloxy | H | Me | H |
| 74 | OTos | 4-phenylazo | H | Me | H |
| 75 | OTos | 4-actylamino | H | Me | H |
| 76 | OTos | 4-NO₂ | H | Me | H |
| 77 | OTos | 4-CN | H | Me | H |
| 78 | OTos | 4-CO₂Me | H | Me | H |
| 79 | OTos | 4-CONMe₂ | H | Me | H |
| 80 | OTos | 4-COMe | H | Me | H |
| 81 | OTos | 4-CH=NPh | H | Me | H |
| 82 | OTos | 4-N=CHMe₂ | H | Me | H |
| 83 | OTos | 4-CH=NOH | H | Me | H |
| 84 | OTos | 4,5-(-N=CH=CH=CH-) | | Me | H |
| 85 | OTos | 4,5-(-N=CH-NH-) | | Me | H |
| 86 | OTos | H | H | Me | Me |
| 87 | OTos | 3-Me | H | Me | Me |
| 88 | OTos | 3-Me | 4-Me | Me | Me |
| 89 | OTos | 3-Me | 4-Cl | Me | Me |
| 90 | OTos | 3-Me | 4-OMe | Me | Me |
| 91 | OTos | 3-CH=CH₂ | H | Me | Me |
| 92 | OTos | 3-acethylene | H | Me | Me |
| 93 | OTos | 4-CH₂Cl | H | Me | Me |
| 94 | OTos | 4-CH₂OCH₃ | H | Me | Me |
| 95 | OTos | 4-(4-Cl-C₆H₅) | H | Me | Me |
| 96 | OTos | 4-(2-pyridyl) | H | Me | Me |
| 97 | OTos | 4-OCH₂CH=CH₂ | H | Me | Me |
| 98 | OTos | 4-OCH₂Ph | H | Me | Me |
| 99 | OTos | 4-propargyloxy | H | Me | Me |
| 100 | OTos | 4-NMe₂ | H | Me | Me |
| 101 | OTos | 4-acethyloxy | H | Me | Me |
| 102 | OTos | 4-phenylazo | H | Me | Me |
| 103 | OTos | 4-acetylamino | H | Me | Me |
| 104 | OTos | 4-NO₂ | H | Me | Me |
| 105 | OTos | 4-CN | H | Me | Me |
| 106 | OTos | 4-CO₂Me | H | Me | Me |
| 107 | OTos | 4-CO2NMe₂ | H | Me | Me |
| 108 | OTos | 4-COMe | H | Me | Me |
| 109 | OTos | 4-CH=NPh | H | Me | Me |
| 110 | OTos | 4-N=CMe₂ | H | Me | Me |
| 111 | OTos | 4-CH=NOH | H | Me | Me |
| 112 | OTos | 4,5-(-N=CH=CH=CH-) | | Me | Me |
| 113 | OTos | 4,5-(-N=CH-NH-) | | Me | Me |
| In the table above, OTos represents OSO₂C₆H₄CH₃-p. | | | | | |

As the alcohol represented by the formula of R₁₂OH to be used, methanol, ethanol, isopropanol, 2-chloroethanol, 1,1,1-trifluoroethanol, benzyl alcohol, ally alcohol, cyclohexanol and the like can be given as the examples. Whereas, as the orthoester represented by the formula of R₁₃C(OR₁₂)₃, methyl orthoformate, ethyl orthoformate, orthoacetate and the like can be given as the examples.

The reaction is carried out at a temperature of from an ambient temperature to the reflux temperature for the solvent in the presence of an acid catalyst by using either an alcohol to be introduced as a solvent or an alcohol in an amount of at least 1 equivalent to be introduced into an inert solvent, such as benzene, toluene and chlorobenzene. The obtained acrylic acid derivative (IV) contains almost no stereoisomer thereof but contains only E-isomer which are useful to be used as an intermediate for producing agricultural chemicals. (The E-isomer is herein defined as the steric structure opposing to carbonyl group via the double bond.)

The reaction proceeds to produce the acetal derivative and then to cause the dealcoholization thereof. Therefore, when no alcohol is used as a solvent, the reaction proceeds efficiently in case that the reaction is carried out while distilling out the alcohol from the reaction system. Alternatively, an ortho ester represented by a formula of R₁₃C (OR₁₂)₃ in an amount more than the catalytic amount can be applied to the reaction system in order to accelerate the acetalization reaction.

As the acid to be used in the reaction, any of protonic acid or Lewis acid commonly used can be used without limitation, p-toluenesulfonic acid, methanesulfonic acid, trifluoromethanesulfonic acid, aluminium chloride, zinc chloride, tin chloride, ferric chloride, titanium chloride, alkoxy titanium chloride, titanium alkoxide, brontrifluoride-diethyl ether complex, sulfuric acid, potassium monosulfate and the like can be given. Particularly, it is preferable to use p-toluenesulfonic acid, methanesulfonic acid, etc.

The acetal form which is shown below and is produced as the intermediate can be separated by controlling the reaction condition, such as reaction time and reaction temperature.

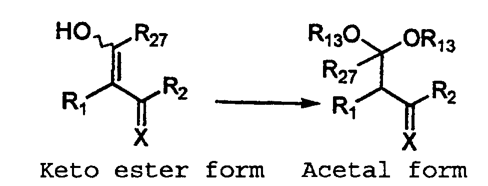

For example, the acetal form is obtainable by maintaining the reaction temperature at around 60°C and completing the reaction in a short time. The acetal form is also synthesized by using an ortho ester in an excess amount or using the ortho ester as a solvent. Moreover, the acetal form is obtained by proceeding the reaction without distilling out the alcohol that is eliminated from the acetal form, from the reaction system. The acetal form is an equivalent of the keto ester form and it has the same reaction property as that of the keto ester form, and the acetal form is an intermediate capable of proceeding the alkylation at the α -position of the keto ester form, which is normally difficult to execute in case of the keto ester form, thereby allowing to execute the modification the α -position. Therefore, the acetal form is highly useful compound as the intermediate for the synthesis of other agricultural chemicals and pharmaceutical ingredients.

The acrylic acid derivatives (III) are also obtainable by using the separated acetal forms according to the similar reaction as described above.

Among the acetal form compounds represented by the formula above and to be used in the present invention, particularly the ones of which group represented by R₁ is a group represented by the following formula; 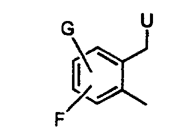 wherein U, G and F are as defined above, are novel compounds and are useful as an intermediate for agricultural chemicals and pharmaceutical ingredients.

In the following, such actal compounds are definitely shown.

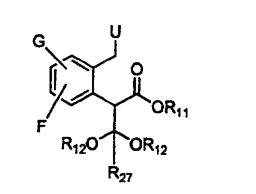

| Compound No. | U | F | G | R₁₁ | R₂₇ | R₁₂ |
|---|---|---|---|---|---|---|
| 1 | Cl | H | H | Me | H | Me |
| 2 | Br | H | H | Me | H | Me |
| 3 | Cl | 3-Me | H | Me | H | Me |
| 4 | Cl | 3-Me | 4-Me | Me | H | Me |
| 5 | Cl | 3-Me | 4-Cl | Me | H | Me |
| 6 | Cl | 3-Me | 4-OMe | Me | H | Me |
| 7 | Cl | 3-CH=CH₂ | H | Me | H | Me |
| 8 | Cl | 3-acetylene | H | Me | H | Me |
| 9 | Cl | 4-CH₂Cl | H | Me | H | Me |
| 10 | Cl | 4-CH₂OCH₃ | H | Me | H | Me |
| 11 | Cl | 4-(4-Cl-C₆H₅) | H | Me | H | Me |
| 12 | Cl | 4-(2-pyridyl) | H | Me | H | Me |
| 13 | Cl | 4-OCH₂CH=CH₂ | H | Me | H | Me |
| 14 | Cl | 4-OCH₂Ph | H | Me | H | Me |
| 15 | Cl | 4-propargyloxy | H | Me | H | Me |
| 16 | Cl | 4-NMe₂ | H | Me | H | Me |
| 17 | Cl | 4-acetyloxy | H | Me | H | Me |
| 18 | Cl | 4-phenylazo | H | Me | H | Me |
| 19 | Cl | 4-acetylamino | H | Me | H | Me |
| 20 | Cl | 4-NO₂ | H | Me | H | Me |
| 21 | Cl | 4-CN | H | Me | H | Me |
| 22 | Cl | 4-CO₂Me | H | Me | H | Me |
| 23 | Cl | 4-CONMe₂ | H | Me | H | Me |
| 24 | Cl | 4-COMe | H | Me | H | Me |
| 25 | Cl | 4-CH=NPh | H | Me | H | Me |
| 26 | Cl | 4-N=CHMe₂ | H | Me | H | Me |
| 27 | Cl | 4-CH=NOH | H | Me | H | Me |
| 28 | Cl | 4,5-(-N=CH=CH=CH-) | | Me | H | Me |
| 29 | Cl | 4,5-(-N=CH-NH-) | | Me | H | Me |
| 30 | Cl | H | H | Me | Me | Me |
| 31 | Cl | 3-Me | H | Me | Me | Me |
| 32 | Cl | 3-Me | 4-Me | Me | Me | Me |
| 33 | Cl | 3-Me | 4-Cl | Me | Me | Me |
| 34 | Cl | 3-Me | 4-OMe | Me | Me | Me |
| 35 | Cl | 3-CH=CH₂ | H | Me | Me | Me |
| 36 | Cl | 3-acetylene | H | Me | Me | Me |
| 37 | Cl | 4-CH₂Cl | H | Me | Me | Me |
| 38 | Cl | 4-CH₂OCH₃ | H | Me | Me | Me |
| 39 | Cl | 4-(4-Cl-C₆H₅) | H | Me | Me | Me |
| 40 | Cl | 4-(2-pyridyl) | H | Me | Me | Me |
| 41 | Cl | 4-OCH₂CH=CH₂ | H | Me | Me | Me |
| 42 | Cl | 4-OCH₂Ph | H | Me | Me | Me |
| 43 | Cl | 4-propargyloxy | H | Me | Me | Me |
| 44 | Cl | 4-NMe₂ | H | Me | Me | Me |
| 45 | Cl | 4-acetyloxy | H | Me | Me | Me |
| 46 | Cl | 4-phenylazo | H | Me | Me | Me |
| 47 | Cl | 4-acetylamino | H | Me | Me | Me |
| 48 | Cl | 4-NO₂ | H | Me | Me | Me |
| 49 | Cl | 4-CN | H | Me | Me | Me |
| 50 | Cl | 4-CO₂Me | H | Me | Me | Me |
| 51 | Cl | 4-CO2NMe₂ | H | Me | Me | Me |
| 52 | Cl | 4-COMe | H | Me | Me | Me |
| 53 | Cl | 4-CH=NPh | H | Me | Me | Me |
| 54 | Cl | 4-N=CMe₂ | H | Me | Me | Me |
| 55 | Cl | 4-CH=NOH | H | Me | Me | Me |
| 56 | Cl | 4,5-(-N=CH=CH=CH-) | | Me | Me | Me |
| 57 | Cl | 4,5-(-N=CH-NH-) | | Me | Me | Me |
| 58 | OTos | H | H | Me | H | Me |
| 59 | OTos | 3-Me | H | Me | H | Me |
| 60 | OTos | 3-Me | 4-Me | Me | H | Me |
| 61 | OTos | 3-Me | 4-Cl | Me | H | Me |
| 62 | OTos | 3-Me | 4-OMe | Me | H | Me |
| 63 | OTos | 3-CH=CH₂ | H | Me | H | Me |
| 64 | OTos | 3-acetylene | H | Me | H | Me |
| 65 | OTos | 4-CH₂Cl | H | Me | H | Me |
| 66 | OTos | 4-CH₂OCH₃ | H | Me | H | Me |
| 67 | OTos | 4-(4-Cl-C₆H₅) | H | Me | H | Me |
| 68 | OTos | 4-(2-pyridyl) | H | Me | H | Me |
| 69 | OTos | 4-OCH₂CH=CH₂ | H | Me | H | Me |
| 70 | OTos | 4-OCH₂Ph | H | Me | H | Me |
| 71 | OTos | 4-propargyloxy | H | Me | H | Me |
| 72 | OTos | 4-NMe₂ | H | Me | H | Me |
| 73 | OTos | 4-acetyloxy | H | Me | H | Me |
| 74 | OTos | 4-phenylazo | H | Me | H | Me |
| 75 | OTos | 4-acetylamino | H | Me | H | Me |
| 76 | OTos | 4-NO₂ | H | Me | H | Me |
| 77 | OTos | 4-CN | H | Me | H | Me |
| 78 | OTos | 4-CO₂Me | H | Me | H | Me |
| 79 | OTos | 4-CONMe₂ | H | Me | H | Me |
| 80 | OTos | 4-COMe | H | Me | H | Me |
| 81 | OTos | 4-CH=NPh | H | Me | H | Me |
| 82 | OTos | 4-N=CHMe₂ | H | Me | H | Me |
| 83 | OTos | 4-CH=NOH | H | Me | H | Me |
| 84 | OTos | 4,5-(-N=CH=CH=CH-) | | Me | H | Me |
| 85 | OTos | 4,5-(-N=CH-NH-) | | Me | H | Me |
| 86 | OTos | H | H | Me | Me | Me |
| 87 | OTos | 3-Me | H | Me | Me | Me |
| 88 | OTos | 3-Me | 4-Me | Me | Me | Me |
| 89 | OTos | 3-Me | 4-Cl | Me | Me | Me |
| 90 | OTos | 3-Me | 4-OMe | Me | Me | Me |
| 91 | OTos | 3-CH=CH₂ | H | Me | Me | Me |
| 92 | OTos | 3-acetylene | H | Me | Me | Me |
| 93 | OTos | 4-CH₂Cl | H | Me | Me | Me |
| 94 | OTos | 4-CH₂OCH₃ | H | Me | Me | Me |
| 95 | OTos | 4-(4-Cl-C₆H₅) | H | Me | Me | Me |
| 96 | OTos | 4-(2-pyridyl) | H | Me | Me | Me |
| 97 | OTos | 4-OCH₂CH=CH₂ | H | Me | Me | Me |
| 98 | OTos | 4-OCH₂Ph | H | Me | Me | Me |
| 99 | OTos | 4-propargyloxy | H | Me | Me | Me |
| 100 | OTos | 4-NMe₂ | H | Me | Me | Me |
| 101 | OTos | 4-acetyloxy | H | Me | Me | Me |
| 102 | OTos | 4-phenylazo | H | Me | Me | Me |
| 103 | OTos | 4-acetylamino | H | Me | Me | Me |
| 104 | OTos | 4-NO₂ | H | Me | Me | Me |
| 105 | OTos | 4-CN | H | Me | Me | Me |
| 106 | OTos | 4-CO₂Me | H | Me | Me | Me |
| 107 | OTos | 4-CO2NMe₂ | H | Me | Me | Me |
| 108 | OTos | 4-COMe | H | Me | Me | Me |
| 109 | OTos | 4-CH=NPh | H | Me | Me | Me |
| 110 | OTos | 4-N=CMe₂ | H | Me | Me | Me |
| 111 | OTos | 4-CH=NOH | H | Me | Me | Me |
| 112 | OTos | 4,5-(-N=CH=CH=CH-) | | Me | Me | Me |
| 113 | OTos | 4,5-(-N=CH-NH-) | | Me | Me | Me |
| In the table above, OTos represents OSO₂C₆H₄CH₃-p. | | | | | | |

### Regarding the constitutions 14 through 24;

It is found that α-alkoxymethylenecarbonyl compound is highly-selectively obtained by choosing a specific base and/or a specific reaction condition at the time of O-alkylating the α -hydroxymethylenecarbonyl compound represented by the general formula (V). The details are explained in the following. In this process, α-hydroxymethylenecarbonyl compound can be converted to α-hydroxyiminocarbonyl compound.

In the present constitutions, α -hydroxymethylenecarbonyl compound represented by the general formula (V) shown above is used as the raw material.

In the compounds represented by the general formula (V), R₂₈ each independently represents a straight-chain or branching optionally substituted alkyl, such as methyl, ethyl, propyl, isopropyl, butyl, isobutyl, sec-butyl, t-butyl, pentyl, hexyl, heptyl, octyl, nonyl, decyl, dodecyl, undecyl, cetyl and lauryl, optionally substituted cycloalkyl, such as cyclopropyl, cyclopentyl and cyclohexyl, phenyl optionally substituted at an arbitrary position of the benzene ring or a heterocyclic group optionally substituted at an arbitrary position of the hetero ring.

The optionally substituted alkyl, the optionally substituted cycloalkyl, the optionally substituted phenyl and the optionally substituted heterocyclic group described above may be substituted with a plurality of substituents which are same or different each other.

As examples for the substituents described above, halogeno, such as fluorine, chlorine and bromine; alkyl, such as methyl, ethyl, propyl, isopropyl, butyl, sec-butyl, t-butyl, pentyl, hexyl, nonyl and decyl; aryloxy, such as phenoxy and tolyloxy; alkoxy, such as methoxy, ethoxy, propoxy, isopropoxy, butoxy and t-butoxy; optionally substituted benzyloxyalkyl, such as benzyloxymethyl, benzyloxyethyl, 4-chlorobenzyloxymethyl, 3-methylbenzyloxymethyl and 2,4-dimethoxybenzyloxymethyl; alkylthio, such as methylthio, ethylthio, propylthio, isopropylthio, butylthio and t-butylthio; alkoxycarbonyl, such as methoxycarbonyl, ethoxycarbonyl, propoxycarbonyl, isopropoxycarbonyl and butoxycarbonyl; alkylsulfenyl, such as methylsulfenyl, ethylsulfenyl, propylsulfenyl and butylsulfenyl; alkylsulfonyl, such as methylsulfonyl, ethylsulfonyl, propylsulfonyl, isopropylsulfonyl, butylsulfonyl and t-butylsulfonyl; alkoxyalkyl, such as methoxymethyl, methoxyethyl, methoxypropyl, ethoxymethyl, ethoxyethyl and ethoxypropyl; optionally substituted aryloxyalkyl, such as phenoxymethyl, phenoxyethyl, tolyloxymethyl and tolyloxyethyl; optionally substituted heteroaryloxyalkyl, such as pyridyloxymethyl, pyridyloxyethyl and pyrimidyloxymethyl; alkylthioalkyl, such as methylthiomethyl, methylthioethyl, methylthiopropyl, ethylthiomethyl, 2-ethylthioethyl and 3-ethylthiopropyl; an optionally substituted arylthioalkyl, phenylthiomethyl, phenylthioethyl, tolylthiomethyl and tolylthioethyl; optionally substituted heterothioalkyl, such as pyridylthiomethyl, pyridylthioethyl and pyrimidylthiomethyl; cycloalkyl, such as cyclopropyl, cyclopentyl and cyclohexyl; optionally substituted phenyl, such as phenyl, 4-chlorophenyl, 2-methylphenyl, 2,4-dichlorophenyl, 2,6-difluorophenyl, 3-bromophenyl, 3-nitrophenyl and 2,4,6-trimethylphenyl; alkenyl, such as vinyl, propenyl, isopropenyl and butenyl; alkynyl, such as ethynyl and propynyl; nitro, cycno, formyl, hydroxy and the like can be given.

As the definite examples for the group represented by R₂₈, haloalkyl, such as chloromethyl, trichloromethyl, difluoromethyl, trifluoromethyl, trifluoroethyl and pentafluoroethyl; alkoxyalkyl, such as methoxymethyl, methoxyethyl, methoxypropyl, methoxybutyl, ethoxymethyl, ethoxyethyl, ethoxypropyl and ethoxybutyl; alkylthioalkyl, such as methylthiomethyl, methylthioethyl, methylthiopropyl, methylthiobutyl, ethylthiomethyl, ethylthioethyl, ethylthiopropyl, ethylthiobutyl, propylthiomethyl and propylthioethyl; alkylsulfenylalkyl, such as methylsulfenylmethyl, methylsulfenylethyl, methylsulfenylpropyl, methylsulfenylbutyl, ethylsulfenylmethyl, ethylsulfenylethyl, ethylsulfenylpropyl, ethylsulfenylbutyl, propylsulfenylmethyl and propylsulfenylethyl; alkylsulfonylalkyl, such as methylsulfonylmethyl, methyl sulfonylethyl, methylsulfonylpropyl, methylsulfonylbutyl, ethylsulfonylmethyl, ethylsulfonylethyl, ethylsulfonylpropyl, ethylsulfonylbutyl, propylsulfonylmethyl and propylsulfonylethyl; alkoxycarbonylalkyl, such as methoxycarbonylmethyl, methoxycarbonylethyl, methoxycarbonylpropyl, ethoxycarbonylmethyl, ethoxycarbonylethyl, ethoxycarbonylpropyl, propoxycarbonylmethyl and propoxycarbonylethyl; cycloalkylalkyl, such as cyclopropylmethyl, cyclopropylethyl, cyclopropylpropyl, cyclopentylmethyl, cyclopentylethyl, cyclohexylmethyl and cyclohexylpropyl; optionally substituted phenylalkyl, such as phenylmethyl, 2 -chlorophenylmethyl, 3-bromophenylmethyl, 2,5-dimethylphenylmethyl, 3-nitrophenylmethyl, 2-fluoroethylphenylmethyl, phenylethyl, 4-chlorophenylethyl, 3,5-dibromophenylethyl and phenylpropyl; alkyl substituted with a heterocycle, such as 2-furylmethyl, 3-furylmethyl, 2-furylethyl, 3-furylethyl, 2-thienylmethyl, 3-thienylmethyl, 2-thiazolylmethyl, 4-thiazolylmethyl, 5-thiazolylmethyl, 2-oxazolylmethyl, 4-oxazolylmethyl, 5-oxazolylmethyl, 2-pyridylmethyl, 3-pyridylmethyl, 4-pyridylmethyl, 5-pyridylmethyl, 5-chloro-2-pyridylmethyl, 2-pyridylethyl, 2-imidazolylmethyl, 4-imidazolylmethyl and 5-imidazolylmethyl; cyanoalkyl, such as 2-cyanomethyl, cyanoethyl and cyanopropyl; naphthylalkyl, such as α -naphthylmethyl, β -naphthylmethyl, α -naphthylethyl and β -naphthylethyl; alkenyl, such as 3-propenyl, 2-butenyl, 3-butenyl, isopropenyl and crotyl; alkynyl, such as 2-propynyl, 2-butynyl and 3-butynyl; optionally substituted cyclohexyl, such as cyclopropyl, 2-fluorocyclopropyl, 2-methylcyclopropyl, 2-chlorocyclopropyl, dichlorocyclopropyl, dibromocyclopropyl, cyclopentyl, methylcyclopentyl, dimethylcyclopentyl, cyclohexyl, methylcyclohexyl and dimethylcyclohexyl; two to six-membered ring heteroyloxymethylphenyl, such as phenyl, 2-methylphenyl, 3-methylphenyl, 4-methylphenyl, 2,3-dimethylphenyl, 2,4-dichlorophenyl, 2,5-dibromophenyl, 3,4-difluorophenyl, 3-methyl -5-nitrophenyl, 2-fluoro-6-chlorophenyl, 2-ethylphenyl, 2-methoxycarbonylphenyl, 2-ethoxycarbonylphenyl, 2-cyanophenyl, 3-cyanophenyl, 2-chloromethylphenyl, 2-methoxycarbonylmethylphenyl, 2-ethoxycarbonylphenyl, 2-dimethylaminocarbonylphenyl, 2-methylaminocarbonylphenyl, 3-ethylaminocarbonylphenyl, 2-phenylmethylphenyl, 2-cyanomethylphenyl, 2-nitromethylphenyl, 3-propenylphenyl, 4-propynylphenyl, 2-dimethoxymethylphenyl, 2-(2'-cinnamyl)phenyl, 2-(5'-phenoxy)phenoxyphenyl, 2-phenoxyphenyl, 2-{[6'-(2"-cyanophenyl)pyrimidine-2'-yl]phenoxy, 2-(2-pyridyloxymethyl) phenyl, 2-(3'-pyridyloxymethylphenyl), 2-(4'-pyridyloxymethyl)phenyl, 2-[(5'-chloropyridine-2'-yl)oxymethyl] phenyl, 2-[(5'-trifluoromethylpyridine-2'-yl)oxymethyl]phenyl, 2-(3'-pyridazinyloxymethyl)phenyl, 2-(4'-pyridazinyloxymethyl) phenyl, 2-[(5'-isopropyl-pyridazine-3'-yl)oxymethyl]phenyl, 2-(4'-pyrimidyloxymethyl)phenyl, 2-(2'-pyrimidyloxymethyl)phenyl and 2-[(2'-isopropoxy-6-trifluoromethyl-pyrimidine-4'-yl) oxymethylphenyl; phenyl optionally substituted with two to five-membered ring heteroyloxymethylphenyl or the like, such as 2-(2'-imidazolyloxymethyl)phenyl, 2-(4'-imidazolyloxymethyl) phenyl, 2-(5'-imidazolyloxymethyl)phenyl, 2-(3'-pyrazolyloxymethyl)phenyl, 2-(4'-pyrazolyloxymethyl)phenyl, 2-(2'-oxazolyloxymethyl)phenyl, 2-(4'-oxazolyloxymethyl)phenyl, 2-(5'-oxazolyloxymethyl)phenyl, 2-(3'-thiazolyloxymethyl)phenyl, 2-(4'-thiazolyloxymethyl)phenyl, 2-(5'-thiazolyloxymethyl) phenyl, 2-(3'-isoxazolyloxymethyl)phenyl, 2-(4'-isoxazolyloxymethyl)phenyl, 2-(5'-isoxazolyloxymethyl)phenyl, 2-(3'-isothiazolyloxymethyl)phenyl, 2- (4'-isothiazolyloxymethyl)phenyl and 2-(5'-isothiazolyloxymethyl) phenyl; a five-membered ring heterocyclic group, such as 2-furyl, 3-furyl, 2-thienyl, 3-thienyl, 2-pyrrolyl, 3-pyrrolyl, 3-pyrazolyl, 4-pyrazolyl, 2-imidazolyl, 4-imidazolyl, 5-imidazolyl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl and 5-isothiazolyl; and a six-membered ring heterocyclic group, such as 2-pyridyl, 3-pyridyl, 4-pyridyl, 5-pyridyl, 2-pyranyl, 3-pyranyl, 4-pyranyl, 5-pyranyl, 2-thianyl, 3-thianyl, 4-thianyl, 5-thianyl, 3-pyridazinyl, 4-pyradazinyl, 2-pyrimidinyl, 3-pyrimidinyl, 4-pyrimidinyl, 2-dioxanyl, 3-dioxanyl, morpholine-2-yl, morpholine-3-yl, piperadine-2-yl andpiperidine-2-yl, canbe given.

The group represented by R₂₉ represents C₁₋₆ alkyl, such as methyl, ethyl, propyl, isopropyl, butyl, sec-butyl, t-butyl, isobutyl, pentyl, neopentyl and hexyl; C₃₋₈ cycloalkyl, such as cyclopropyl, cyclopentyl and cyclohexyl; C₁₋₆ alkoxy, such as methoxy, ethoxy, propoxy, isopropoxy, butoxy and t-butoxy; hydroxy; a group represented by a formula of NHr₁, such as amino, methylamino, ethylamino, propylamino, isopropylamino, butylamino, phenylamino, 4-chlorophenylamino, acetylamino and benzoylamino; a group representedbya formula of Nr₂r₃, such as dimethylamino, diethylamino, dipropylamino, diisopropylamino, dibutylamino, diphenylamino, di(4-chlorophenyl)amino, methylethylamino, methylphenylamino, acetylmethylamino and benzoylmethylamino; phenyl optionally substituted with a substituent at an arbitraryposition of the benzene ring or a heterocyclic group optionally substituted with a substituent at an arbitrary position of the heterocycle. The optionally substituted phenyl and the optionally substituted heterocyclic group described above may be substituted with a plurality of substituents which are same or different each other.

As examples for the substituent for the phenyl and the heterocyclic group described above, the same examples given for the optionally substituted phenyl and the optionally substituted heterocyclic group in the group represented by R₂₈ can be applied as well.

The groups represented by R₂₈ and R₂₉ may jointly form C₁₋₆ saturated or unsaturated ring or a five to six-membered saturated or unsaturated ring heterocycle containing oxygen, nitrogen or sulfur therein.

The compound represented by the general formula (VI-1) is obtainable by O-alkylating the compound represented by the general formula (V) in a bilayer mixed-solvent system consisting of an organic solvent and water in the presence of a phase-transfer catalyst and a predetermined base.

In the general formula (VI-1), R₂₈, R₂₉ and R₁₂ are as defined above.

The reaction described above is preferably carried out I abilayer mixed-solvent system consisting of an organic solvent and water. As the organic solvent, it is preferable to choose one incompatible with water and capable of dissolving the compound represented by the general formula (I) described above. As examples for such solvent, an aromatic hydrocarbon, such as benzene, toluene, xylene and chlorobenzene; a halogenated hydrocarbon, such as chloroform, dichloromethane, carbon tetrachloride and 1,2-dichloroethane; an aliphatic hydrocarbon, such as pentane, hexane, heptane, octane and nonane; an alcohol, such as butanol, pentanol, hexanol and octanol; an ether type solvent, such as diethyl ether, 1,2-dimethoxyethane and methylcersolb; an ester, such as ethyl acetate, propyl acetate and butyl acetate; a ketone, such as methyl isobutyl ketone, methyl ethyl ketone and cyclohexanone, can be given. The rate to mix any of the organic solvent exemplified above and water is preferably in a range of 1:100 to 100:1.

In the reaction described above, any of an alkali metal hydroxide excluding lithium hydroxide, an alkali metal carbonate excluding lithium carbonate, an alkaline earth metal hydroxide and an alkaline earth metal carbonate is used as the base.

As examples for said alkali metal hydroxide, said alkali metal carbonate, said alkaline earth metal hydroxide and said alkaline earth metal carbonate, sodium hydroxide, potassium hydroxide, sodium carbonate, potassium carbonate, calcium hydroxide, magnesium hydroxide, potassium carbonate, magnesium carbonate and the like can be used.

In the reaction described above, the rate of the base to be used is preferably 1-2 mole equivalents to 1 mole equivalent of the compound represented by the general formula (V).

As examples for the alkylating agent used in the reaction, an alkylsulfate represented by a formula of (R₁₂O)₂SO₂, wherein R₁₂ is as defined above, and halogenated alkyl represented by a formula of R₁₂-U, wherein R₁₂ and U are as defined above, are given.

More definitely, sulfuric acid esters, such as dimethyl sulfate and diethyl sulfate, methyl iodide, ethyl iodide, ethyl bromide, propyl iodide, propyl bromide, isopropyl iodide, isopropyl bromide, butyl iodide, butyl bromide, t-butyl bromide, 1-bromopentane, 1-bromohexane and the like can be given as the example.

As the phase-transfer catalyst used in the reaction, a crown ether, such as 18-crown-6, azacrown and thiacrown, a phosphonium salt, such as cryptand, ionophore, tetrabutylphosphonium chloride, tetrabutylphosphonium bromide, benzyltrimethylphosphonium chloride and benzyltrimethylphosphonium bromide, a quaternary ammonium salt, such as benzyltrialkyl ammonium halide and benzyltrialkyl ammonium hydroxide, can be given as example.

Among the examples given for the phase-transfer catalyst exampled above, it is more preferable to use a quaternary ammonium salt. As examples for the quaternary ammonium salt, benzyltrimethyl ammonium chloride, benzyltrimethyl ammonium bromide, benzyltrimethyl ammonium hydroxide, benzyltrimethyl ammonium hydrosulfide, benzyltriethyl ammonium chloride, benzyltriethyl ammonium bromide, benzyltriethyl ammonium hydroxide, benzyltriethyl ammonium hydrosulfide, benzyltripropyl ammonium chloride, benzyltripropyl ammonium bromide, benzyltripropyl ammonium hydroxide, benzyltripropyl ammonium hydrosulfide, benzyltributyl ammonium chloride, benzyltributyl ammonium bromide, benzyltributyl ammonium hydroxide, benzyltributyl ammonium hydrosulfide, tetrabutyl ammonium chloride, tetrabutyl ammonium bromide, tetrabutyl ammonium hydroxide, tetrabutyl ammonium hydrosulfide, trioctylmethyl ammonium chloride, trioctylmethyl ammonium bromide, trioctylmethyl ammonium hydroxide, trioctylmethyl ammonium hydroxide and the like are given.

Among the examples described above, it is particularly preferable to use benzyltrialkyl ammonium hydroxide or tetraalkyl ammonium hydrosulfide since they can be used as a base and a phase-transfer catalyst.

The rate of the phase-transfer catalyst to be used to the compound represented by the general formula (V) in an amount of 1 mole equivalent is preferably around 0.001 to 10 mole equivalent. The reaction is smoothly proceeded at a temperature in a range of from -10°C to a boiling point of the solvent used. The reaction time is normally in a range of from several minutes to 24 hours.

The reaction to O-alkylate the compound represented by the general formula (V) is carried out in a bilayer mixed-solvent system while maintaining the base concentration in the aqueous layer at lower than 10% by weight, more preferably lower than 6% by weight, or maintaining the concentration of the salt of the compound represented by the general formula (V) at lower than 10% by weight.

This reaction is carried out according to anyone of the following methods (a) through (d), for example.
(a) A method to feed dropwise an alkylating agent in a fixed amount while stirring into a mixed solution prepared by mixing an aqueous solution of a base (and a phase-transfer catalyst) in a fixed amount and an organic solvent solution of the compound represented by the general formula (V) in a fixed amount.
(b) A method to feed dropwise an aqueous solution of a base (and a phase-transfer catalyst) in a fixed amount into an organic solvent solution of the compound represented by the general formula (V) in a fixed amount and an alkylating agent (and a phase-transfer catalyst) in a fixed amount.
(c) A method simultaneously feed dropwise an organic solvent solution of the compound represented by the general formula (V) in a fixed amount and an aqueous slution of a base in a fixed amount into a bilayer mixed solvent system consisting of an organic solvent containing both of a phase-transfer catalyst in a fixed amount and an alkylating agent in a fixed amount and water.
(d) A method to feed drowise an organic solvent solution of an alkylating agent in a fixed amount into an aqueous solution of a salt of the compound represented by the general formula (V) in a fixed amount and a phase-transfer catalyst in a fixed amount.

As described above, (E)-alkoxymethylenecarbonyl compounds represented by the general formula (VI-1) can be produced in highly selective manner by controlling the type of the base and the base concentration in the aqueous layer. (Here, for convenience, as expression for the steric structure facing to the double bond in the compounds of formulas (VI-1) and (VI-2), the steric positioning at the site opposite to the carbonyl group is defined as (E) -form, while the steric positioning at the same side with the carbonyl group is defined as (Z)-form.)

The compound represented by the general formula (VI-2) is obtainable by O-alkylating the compound represented by the general formula (V) in a bilayermixed-solvent system consisting of an organic solvent and water in the presence of a phase-transfer catalyst and a base in a fixed amount.

In the general formula (VI-2), R₂₈, R₂₉ and R₁₂ are as defined above.

The reaction described above is preferably carried out in a bilayer mixed-solvent system consisting of an organic solvent and water. Further, it is preferable to use the organic solvent that is incompatible with water but capable of dissolving the compound represented by the general formula (V). For said organic solvent, the same solvents as the ones exampled as the preferable solvent to be used for producing the compound represented by the general formula (VI-1) can be used as well. The rate to mix the organic solvent and water is preferably in a range of from 1:100 to 100:1 in general.

In the reaction described above, lithium hydroxide or lithium carbonate is used as the base. The rate of the base to be used in the reaction is preferably around 1-2 mole equivalent to the compound represented by the general formula (V) in an amount of 1 mole equivalent.

As the alkylating agent to be used in the reaction described above, an alkylsulfate represented by a general formula of (R₁₂O)₂SO₂, wherein R₁₂ is as defined above, a halogenated alkyl represented by a general formula of R₁₂-U, wherein R₁₂ and U are as defined above, and the like can be given.

More particularly, the examples for such alkylating agent and phase-transfer catalyst exampled as usable at the production of the compounds represented by the general formula (VI-1) can be used in the reaction as well. The rate of the alkylating agent to be used in the reaction is preferably around 1-2 mole equivalent to the compound represented by the general formula (I) in an amount of 1 mole equivalent. Further, the rate of the phase-transfer catalyst to be used in the reaction is preferably in a range of around 0.001-10 mole equivalent to the compound represented by the general formula (V) in an amount of 1 mole equivalent. The reaction smoothly proceeds under a temperature ranging from -10°C to a boiling point of a solvent used. The reaction time is normally in a range of from several minutes to 24 hours more or less.

The reaction to O-alkylate the compound represented by the general formula (V) is carried out in a bilayer mixed-solvent system consisting of an organic solvent and water while maintaining the concentration of the base in the aqueous layer at 5% by weight or more.

This reaction is carried out according to any one of the following methods (a) through (d), for example.
(a) A method to feed dropwise an alkylating agent in a fixed amount while stirring into a mixed solution prepared by mixing an aqueous solution of a base (and a phase-transfer catalyst) in a fixed amount and an organic solvent solution of the compound represented by the general formula (V) in a fixed amount.
(b) A method to feed dropwise an aqueous solution of a base (and a phase-transfer catalyst) in a fixed amount into an organic solvent solution of the compound represented by the general formula (V) in a fixed amount and an alkylating agent (and a phase-transfer catalyst) in a fixed amount.
(c) A method simultaneously feed dropwise an organic solvent solution of the compound represented by the general formula (V) in a fixed amount and an aqueous slution of a base in a fixed amount into a bilayer mixed solvent system consisting of an organic solvent containing both of a phase-transfer catalyst in a fixed amount and an alkylating agent in a fixed amount and water.
(d) A method to feed drowise an organic solvent solution of an alkylating agent in a fixed amount into an aqueous solution of a salt of the compound represented by the general formula (V) in a fixed amount and a phase-transfer catalyst in a fixed amount.

As described above, (Z)-alkoxymethylenecarbonyl compounds or (Z)-alkoxyiminocarbonyl compounds, both of which are represented by the general formula (VI-2), can be produced in highly selective manner by controlling the type of the base and the base concentration in the aqueous layer.

### Regarding Constitutions 25 and 26;

The objective compounds represented by a general formula (VII) such as phenylacetic acid ester derivatives are produced in highly-selective manner and at high yield by silylating a compound represented by the general formula (VII) such as phenylacetic acid ester derivatives, and reacting the silylated product to a reaction with an orthoformic acid ester in the presence of a Lewis acid after removing a salt such as trifluoromethanesulfonate which is produced at synthesizing ketenesilylacetal, etc.

In the compounds represented by the general formula (VII) to be used for the production process described above, R₁ and R₂ are as defined above. Particularly, the process is useful when using the compounds represented by the general formula (VII), wherein R₁ is a group represented by the following formula; 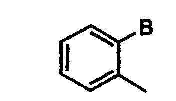 wherein B represents hydrogen, lower alykl, lower alkoxy, haloalkyl, an optionally substituted arylsulfonyloxyalkyl or an optionally substituted lower alkylsulfonyloxyalkyl, R₂ is a group represented by a formula of OR₂₃, wherein R₂₃ represents lower alkyl, and B and R₂₃ may bond to jointly form a ring.

As the group represented by B in the formula above, methyl, methoxy, chloromethyl, methanesulfonyloxymethyl, p-toluenesulfonyloxymethyl and the like can be definitely given as the example. As the group represented by R₂₃, methyl, ethyl, isopropyl, t-butyl and the like are definitely given.

As a process to convert the phenylacetic acid ester derivative or the like represented by the general formula (VII) to the ketenesilylacetal form represented by a general formula (X), a process to convert the phenylacetic acid ester derivative to the ketenesilylacetal form by using a tertiary amine represented by a general formula (IX) as a base and using silyltriflate as a silylating agent is useful particularly when containing reactive functional group such as haloalkyl at B.

In the tertiary amine represented by the general formula (VIII), the groups represented by R₁₆ through R₁₈ may be same or different each other and each represents alkyl, aryl or aralkyl. As definite examples for the group described above, ethyl, propyl, butyl and benzyl are given. Further, for using in combination, triethylamine, tripropylamine, tributylamine, diisopropylethylamine and the like can be given. The rate of the amine to be used is normally 1 equivalent to trifluoromethanesulfonic acid silyl ester.

In the compound represented by the general formula (IX), the groups represented by R₁₉ through R₂₁ may be same or different each other and each represents alykl, aryl or aralkyl. More definitely, ethyl, propyl, isopropyl, butyl, t-butyl, benzyl and the like are given as the examples for such groups. Further, for using in combination, trimethylsilyltriflate, dimethylphenylsilyltriflate, triisopropylsilyltriflate, t-butyldimethylsilyltriflate and the like are given. The rate of the silyl ester to be used is 1-1.5 equivalent to phenylacetate , and preferably 1-1.2 equivalent.

The reaction is carried out under a temperature ranging from 0°C to an ambient temperature, and it is preferable to proceed the reaction at 0°C in view of the stability of the compound. As the solvent used for the reaction, it is preferable to use a hydrocaron, such as toluene, or an ether, such as diethyl ether, for removing a salt of resulting trifluoromethanesulfonate.

As a method to remove a salt of trifluoromethanesulfonate, though any method can be employed without limitation, a method to take out the salt being separated from the reaction system by using an injector and a method to separate a layer containing the salt and then take out are given. The operation of these methods are preferably carried out in an atmosphere of an inactive gas while shutting outdoor air off since silyl ether represented by the general formula (X) is unstable against moisture. However, the operation of such separation work can be also carried out in the air when the silyl group is a bulky substituent like t-butylmethyl.

In the present constitutions, for leading a ketenesilylacetal compound represented by the general formula (X) to an alkoxymethylene compound such as phenylacetic acid ester derivative represented by the general formula (XII), it is preferable to employ a process to condensate the ketenesilylacetal compound with an orthoformic acid ester represented by a general formula (XI) in the presence of a Lewis acid.

As the Lewis acid to be added for accelerating the reaction and improving the selectivity of the reaction, a halogenated metal, such as halogenated titanium, halogenated aluminium, halogenated boron, halogenated iron and halogenated zinc, and a halogenated metal partially substituted with lower alkoxy, such as dichlorotitanium diisopropoxide, can be given as the example. The Lewis acid to be added in the present constitutions is appropriately chosen from the Lewis acids exampled above and is used either solely or in combination. There is no limitation in the amount of the Lewis acid to be used in the present constitution and any amount capable of smoothly proceed the reaction can be applied, the addition of the Lewis acid in an amount of 1 mole relative to 1 mole ketenesilylacetal compound may accelerate the desired reaction, and the use of the Lewis acid in an excess amount may accelerate the reaction to a further extent.

In the orthoformic acid ester represented by the general formula (XI), R₁₂ represents a group as defined above.

As a solvent used in alkoxymethylation reaction, a halogenated hydrocarbon, such as chloroform, dichloromethane and carbon tetrachloride, an aromatic hydrocarbon, such as benzene, toluene, xylene and chlorobenzene, and an ether, such as diethyl ether, dimethyl ether and tetrahydrofuran, are given as the definite examples. These solvents are appropriately selected depending upon the type of reaction and are used either solely or in combination. There is no limitation in the amount of the solvent to be used, an appropriate amount required for carrying on uniform reaction is enough, and there is no need to use the solvent in excess amount. The quantity in liter of the solvent required for the reaction is more or less 2-20 l/kg relative to the total additional amount of whole raw materials in a normal equimolar reaction, and more preferably 4-8 l/kg, for example.

### Regarding Constitutions 27 and 28:

An isochroman compound of the general formula (XIII), which is a starting material in the process according to the present constitution, is commercially available and is easily produced according to the processes disclosed in EP No. 936220 or JP No. 11-279169.

Isochroman compounds represented by a formula (XIII), which may be a starting material to be used in the production process specified in the present constitutions are commercially available and are easily produced according to the processes disclosed in EP No. 936220, JP No. 11-279169 and the like.

In the formula (XIII), R₂₄ represents nitro, cyano, halogeno, C₁₋₆ alkyl, C₁₋₆ alkoxy, C₁₋₆ haloalkyl or C₁₋₆ alkoxycarbonyl, n represents 0 or an integer of 1 through 4, and R₂₄ represents same or different groups when n is 2 or more. There is no limitation in the substituting position for the group represented by R₂₄ when n is 1 or more.

As definite examples for the group represented by R₂₄, nitro, cyano, halogeno, such as fluorine, chlorine and bromine, C₁₋₆ alkyl, such as methyl, ethyl, isopropyl, methoxymethyl and methylthiomethyl, C₁₋₆ alkoxy, methoxy, ethoxy, isopropoxy and trifluoromethoxy, C₁₋₆haloalkyl, such as chloroethyl, fluoroethyl, trichloromethyl, trifluoromethyl and difluoromethyl, C₁₋₆ alkoxycarbonyl, such as methoxycarbonyl and t-butoxycarbonyl, and the like can be given.

α -hydroxymethylenecarbonyl compounds represented by a general formula (XIV) are obtainable by applying a formylating agent to an isochroman compound represented by the general formula (XIII) in an inactive solvent.

As the formylating agent usable for the reaction described above, for example, a formic acid ester and an orthoformic acid ester are given.

As examples for the formic acid ester, methyl formate, ethyl formate, propyl formate, isopropyl formate, butyl formate and t-butyl formate are given. The rate of the formylating agent to be used is approximately 1-2 equivalent relative to the compound represented by the general formula (XIII) in an amount of 1 mole equivalent.

In the formylation reaction using the formic acid ester, it is preferable to carry out the reaction in the presence of a formic acid ester and a base. As examples for the base to be used in the above reaction, an alkali metal alkoxide, such as lithium methoxide, lithium ethoxide, sodium methoxide, sodium ethoxide, potassium methoxide, potassium ethoxide and potassium t-butoxide; an alkaline earth metal alkoxide, such as magnesium methoxide, magnesium ethoxide and calcium ethoxide; an organic lithium, such as lithium diisopropylamide and lithium hexamethyl disilazide; a metal hydride, such as sodium hydride, potassium hydride and calcium hydride; a carbonate salt, such as lithium carbonate, sodium carbonate, potassium carbonate and magnesium carbonate; a hydroxide compound, such as lithium hydroxide, sodium hydroxide, potassium hydroxide, magnesium hydroxide and calcium hydroxide; and an organic base, such as triethylamine, diisopropylethylamine, pyridine, piperadine and DBU, can be given. The rate of the base to be used in the above reaction is preferably in a range of from 1 to 3 equivalents relative to the compound represented by the general formula (XIII) in an amount of 1 mole equivalent.

When the base is used, the reaction proceeds more smoothly by further adding a titanium compound to the reaction system. As examples for the titanium compound to be used in the reaction above, a halogenated titanium, such as titanium tetrachloride, a titanium alkoxide, such as tetramethoxy titanium, tetraethoxy titanium, tetraisopropoxy titanium and tetrabutoxy titanium, and the like can be given. The rate of the titanium compound to be added is approximately 0.01-2 mole equivalent relative to the compound represented by the general formula (XIII) in an amount of 1 mole equivalent.

As examples for the solvent to be used in the reaction, an amide-type solvent, such as N,N-dimethylformamide (DMF) and N,N-dimethylacetoamide, an ether-type solvent, such as tetrahydrofuran (THF), 1,2-dimethoxyethane, diethyl ether and methyl cellosolve, an aromatic hydrocarbon, such as benzene, toluene, xylene, chlorobenzene, dichlorobenzene and benzonitrile, a saturated hydrocarbon, such as pentane, hexane, octane and cyclohexane, a halogenated hydrocarbon, such as dichloromethane, chloroform, carbon tetrachloride and 1,2-dichloroethane, and the like can be given.

Among the solvents exampled above, it is particularly preferable to use an organic solvent, which is incompatible with water and capable of dissolving the compounds represented by the general formula (XIII), such as benzene, toluene, xylene, chlorobenzene and dichlorobenzene, in view of enabling to enter into the subsequent O-alkylation reaction continuously.

Then, the α-alkoxymethylenecarbonyl compound represented by the general formula (XV) is obtainable by applying an alkylating agent to the obtained hydroxymethylene compound represented by the general formula (XIV).

As examples for the organic solvent to be used in this reaction, an aromatic hydrocarbon, such as benzene, toluene, xylene and chlorobenzene, a halogenated hydrocarbon, such as chloroform, dichloromethane, carbon tetrachloride and 1,2-dichloroethane, an aliphatic hydrocarbon, such as pentane, hexane, heptane, octane and nonane, an alcohol, such as butanol, pentanol, hexanol and octanol, an ether-type solvent, such as diethyl ether, 1,2-dimethoxyethane and methyl cellosolve, an ester, such as ethyl acetate, propyl acetate and butyl acetate, methyl isobutyl ketone, methyl ethyl ketone, cyclohexanone and the like can be given.

This reaction is preferably carried out in a bilayer solvent system consisting of water and an organic solvent in the presence of a phase-transfer catalyst and an alkylating agent without separating the compound represented by the general formula (XIV). If the organic solvent used at producing the compound of general formula (XIV) is the one incompatible with water, the organic solvent is straightly used for this reaction.

As the base to be used for this reaction, an alkali metal hydroxide, an alkali metal carbonate, an alkaline earth metal hydroxide and an alkaline earth metal carbonate are given as the example.

As definite examples for the alkali metal hydroxide, the alkali metal carbonate, the alkaline earth metal hydroxide and the alkaline earth metal carbonate as described above, lithium hydroxide, sodium hydroxide, potassium hydroxide, lithium carbonate, sodium carbonate, potassium carbonate, calsium hydroxide, magnesium hydroxide, calcium carbonate, magnesium carbonate and the like can be given.

In the reaction described above, the rate of the base to be used is approximately 1-2 mole equivalent relative to the compound represented by the general formula (XIII) in an amount of 1 mole equivalent.

As the alkylating agent to be used in this reaction, an alkyl sulfate represented by a formula of (R₁₂O)₂SO₂, wherein R₁₂ is as defined above, a halogenated alkyl represented by a formula of R₁₂-U, wherein R₁₂ and U are as defined above, and the like can be given.

As definite examples for the alkylating agent, a sulfuric acid ester, such as dimethyl sulfate and diethyl sulfate, methyl iodide, ethyl iodide, ethyl bromide, propyl iodide, propyl bromide, isopropyl iodide, isopropyl bromide, butyl iodide, butyl bromide, t-butyl bromide, 1-bromopentane, 1-bromohexane and the like can be given.

As examples for the phase-transfer catalyst to be used in the reaction described above, a crown ether, such as 18-brown-6, azacrown, thiacrown, a phosphonium salt, such as cryptand, ionophore, tetrabutyl phosphonium chloride, tetrabutyl phosphonium bromide, benzyltrimethyl phosphonium chloride and benzyltrimethyl phosphonium bromide, a quaternary ammonium salt, such as benzyltrialkyl ammonium halide and benzyltrialkyl ammonium hydroxide, and the like can be given.

Among the phase-transfer catalysts exampled above, it is preferable to use either quaternary ammonium halide or quaternary ammonium hydroxide. As examples for the quaternary ammonium salt, benzyltrimethyl ammonium chloride, benzyltrimethyl ammonium bromide, benzyltrimethyl ammonium hydroxide, benzyltrimethylammonium hydrosulfide, benzyltriethyl ammonium chloride, benzyltriethyl ammonium bromide, benzyltriethyl ammonium hydroxide, benzyltriethyl ammonium hydrosulfide, benzyltripropyl ammonium chloride, benzyltripropyl ammonium bromide, benzyltripropyl ammonium hydroxide, benzyltripropyl ammonium hydrosulfide, benzyltributyl ammonium chloride, benzyltributyl ammonium bromide, benzyltributyl ammonium hydroxide, benzyltributyl ammonium hydrosulfide, tetrabutyl ammonium chloride, tetrabutyl ammonium bromide, tetrabutyl ammonium hydroxide, tetrabutyl ammonium hydrosulfide, trioctylmethyl ammonium chloride, trioctylmethyl ammonium bromide, trioctylmethyl ammonium hydrosulfide, trioctylmethyl ammonium hydroxide and the like can be given.

The rate of the phase-transfer catalyst to be used in the reaction described above is preferably approximately 0.001-10 mole equivalent relative to the compund represented by the general formula (XIV) in an amount of 1 mole equivalent. The reaction smoothly proceeds at a temperature in a range of from -10°C to a boiling point of the solvent used. The reaction time is normally in a range of approximately several minutes to 24 hours.

As a method to O-alkylate the compound represented by the general formula (XIV), (a) a method to admix an aqueous solution of a base (and a phase-transfer catalyst) in a fixed amount and an organic solvent solution of the compound represented by the formula (XIV) in a fixed amount and then feed dropwise an alkylating agent in a fixed amount into the mixture while stirring, (b) a method to feed dropwise an aqueous solution of the base (and the phase-transfer catalyst) in a fixed amount into an organic solvent solution of the compound represented by the general formula (XIV) in a fixed amount and the alkylating agent (and the phase-transfer catalyst) in a fixed amount, and (c) a method to simultaneously feed dropwise an organic solvent solution of the compound represented by the general formula (XIV) in a fixed amount and an aqueous solution of the base in a fixed amount into a bilayer mixed-solvent system consisting of an organic solvent solution containing the phase-transfer catalyst in a fixed amount and the alkylating agent in a fixed amount, are given, for example.

The compound represented by the general formula (XV) is converted to an acrylic acid derivatives of formulas (XVI) and (XVII) as shown below, which are useful for producing agricultural chemicals and pharmaceutical ingredients, by applying a halogenating agent, etc. to the compound.

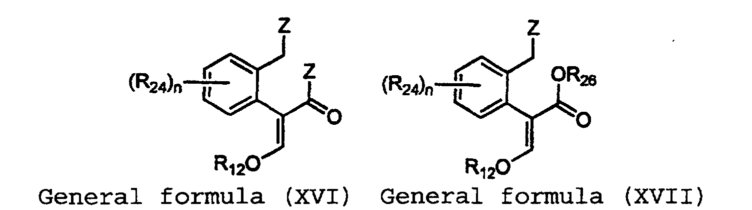

In the general formula (XVI), R₂₄, R₁₂ and n are as defined above, and Z represents halogeno. In the general formula (XVII), R₂₄, R₁₂, n and Z are as defined above, and R₂₆ represents an optionally substituted alkyl. As definite examples for the group represented by R₂₆, methyl, ethyl, isopropyl, cyclohexyl, benzyl, chloroethyl and the like can be given.

The compound represented by the general formula (XVI) is obtainable by applying a halogenating agent to the compound represented by the general formula (XV).

As examples for the halogenating agent, thionyl chloride, thionyl bromide, sulfinyl chloride, oxalyl chloride, phosgene, chlorine, bromine, phosphorus trichloride, phosphorus pentachloride, phosphorous tribromide and the like can be given. The rate of the halogenating agent used in the reaction is preferably approximately 2-10 equivalent relative to the compound represented by the general formula (XV) in an amount of 1 mole equivalent.

Whereas, the reaction may proceed more smoothly when DMF, pyridine or the like in a catalytic amount is addd into the reaction system. The reaction temperature is normally in a range of from an ambient temperature to a boiling point of the solvent used.

In the reaction above, when the alkylating agent remains in the reaction system, the compound represented by the general formula (XVII) is not always obtained at a high yield. In such case, it is preferable to remove the un-reacted alkylating agent beforehand from either the reaction solution (bilayer system) containing the compound represented by the general formula (XV) or the organic solvent solution obtainable by separation from said reaction solution containing the compound represented by the general formula (XV).

The un-reacted alkylating agent can be removed by subjecting the solution containing the compound represented by the general formula (XV) to heating treatment to thermally decompose the alkylating agent. If the alkylating agent has a low boiling point, it is also possible to distilling it out of the reaction solution containing the compound represented by the general formula (XV).

Furthermore, the alkylating agent can be remove by separating the organic solvent solution containing the compound of the formula (XV), condensing the separated solution under reduced pressure, and preparing the condensed solution into the solution of a different organic solvent, then applying a halogenating agent to the solution.

Then, the ester compound represented by the general formula (XVII) is obtainable by applying an alcohol represented by a formula of R₂₆OH to the compound represented by the general formula (XVI) in either an appropriate solvent or an alcohol represented by a formula of R₂₆OH without separating the obtainable compound represented by a formula (XVI).

As examples for the alcohol represented by R₂₆OH, methyl alcohol, ethyl alcohol, propyl alcohol, isopropyl alcohol, butyl alcohol, isobutyl alcohol, sec-butyl alcohol, t-butyl alcohol, pentyl alcohol, hexyl alcohol, and the like can be given.

When appropriate, it is preferable to use the alcohol in combination with a base in an equivalent amount. As examples for such base, an organic base, such as triethylamine and pyridine, a metal alkoxide, such as sodium ethylate and sodium methylate, can be given. When the metal alkoxide is used, it is preferable to use an alkoxide which has the same alkyl part as that of the alcohol to be used. The reaction smoothly proceeds at a temperature in a range of from an ambient temperature to a boiling point of the solvent used.

The compounds represented by the general formula (XVII) obtained as described above are useful as intermediates for producing fungicides, insecticides, etc. for agricultural use disclosed in EP-178826, EP-370629, EP-414153, EP-460575, WO92/18494, WO90/07493, EP-586393, WO94/08968, JP Laid-open 63-216848, JP Laid-open 61-106538, etc.

The compounds represented by the general formula (XVII) are also obtainable by halogenating the corresponding methyl form as shown below.

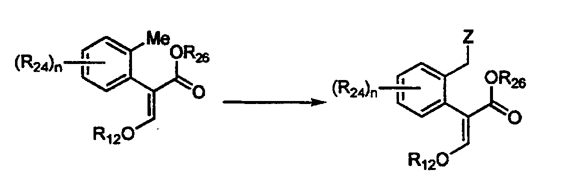

For example, a process to use azobis-isobutylonitrile (hereinafter abbreviated as AIBN) as a starting material and to brominate it with N-bromosuccinimide (See EP-203606) is known. Further, a process to prepare N-bromosuccinimide by using benzoyl peroxide (hereinafter abbreviated as BPO) as a starting material and then to brominate the compound corresponding to the general formula (I) by using AIBN and bromine under a tungsten lamp is also known (See EP-278595). Furthermore, a process to brominate with bromine in a non-aqueous solvent system under light irradiation while using a deacidifying agent polymer as a catalyst is also known (See WO94/05620).

Whereas, as a radical starting material to be used for radical halogenation using bromine, etc., organic peroxides, such as BPO, and azo compounds, such as AIBN, have been known up till now. The organic peroxides are generally unstable against impact and has a problem of danger to cause fire and explosion, while the azo compounds are physically and chemically rather stable, safe for handling during reaction operation, transportation and storing, and easily controlled because of no cause of the self-inducing decomposition as frequently seen in general peroxide compounds and the accurate decomposition at the primary reaction.

In addtion, recently, azo compounds having excellent performance in reaction selectivity, etc. have been developed. In JP laid-open 8-127562, there is a description that a monobromo-form is selectively synthesized even under a relatively low temperature when carrying out bromination reaction of 4'-methyl-2-cyanobiphenyl with bromine by using 2,2'-azobis(4-methoxy-2,4-dimethylvaleronitrile) as the starting material.

However, it is not preferable to employ the process described above in an industrial scale as the process requires N-bromosuccinimide that is very expensive. On the other hand, when using bromine with low quality, only industrially un-desired photoreaction is obtained, or when carrying on bromination reaction by using AIBN or BPO as a radical initiator, disadvantages in safety and bromination efficiency are given since the reaction temperature is higher than the boiling point of bromine, thus the side reaction proceeds to generate dibromo-form, etc. that gives lowering of the yield for the monobromo-form. Even when using 2,2'-azobis(4-methoxy-2,4 -dimethylvaleronitrile), which is a radical initiator capable of proceeding the reaction at relatively low temperature and selectively, there is a problem that a side reaction that hydrogen bromide generating along with the progress of the reaction adds to the double bond in the acrylic acid occurs to lower the yield because the reaction activity of the radical initiator is still so high.

Aprocess to safely and efficiently produce 2-bromomethyl form, which is cheaper in production cost and easy to control, was established by using a radical initiator capable of causing radical decomposition at a lower temperature than the required temperature when using an other radical initiator like AIBN and of which temperature at 10 hours half-life is lower than the boiling point of bromine and halogenating the benzyl position with bromine in a mixed-solvent of an organic solvent and water.

It is found that the compounds represented by a general formula (XIX); 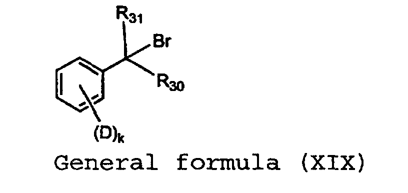 wherein D represents halogeno, optionally substituted alkyl, optionally substituted aralkyl, optionally substituted phenyl, optionally substituted heterocyclic group, optionally substituted alkoxy, hydroxy, nitrile, optionally substituted amino, nitro, alkoxycarbonyl, formyl, optionally substituted acyl or a group represented by a formula of S(O)mR₃₂, wherein R₃₂ represents alkyl, aryl, a heterocyclic group and m represents 0 or an iteger of 1 or 2, or one group selected from the following formulas; 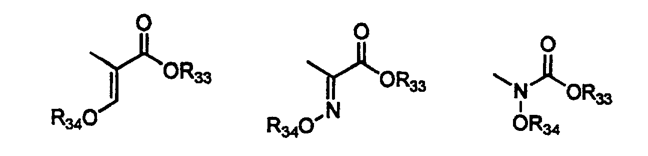 wherein R₃₃ and R₃₄ may be same or different each other and each represents optionally substituted alkyl, optionally substituted allyl, optionally substituted propargyl or optionally substituted aralkyl, R₃₀ and R₃₁ may be same or different each other and each represents hydrogen, halogeno, optionally substituted alkyl, optionally substituted aralkyl, optionally substituted phenyl, optionally substituted heterocyclic group, optionally substituted heterocyclic group, optionally substituted alkoxy, hydroxy, nitrile, optionally substituted amino, nitro, alkoxycarbonyl carboxyl, formyl, optionally substituted acyl or a compound represented by a formula of S(O)mR₃₂, wherein R₃₂ represents alkyl, aryl or a heterocyclic group, and m represents 0, 1 or 2, and k represents 0 or an integer of 1 through 5, is produced in high efficiency by brominating a compound represented by a general formula (XVIII); 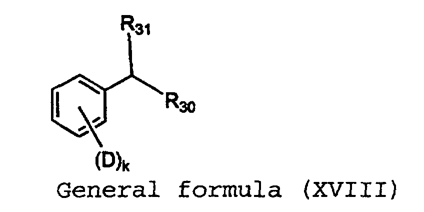 wherein D, R₃₀, R₃₁ and k are as defined above, with bromine and using a radical initiator of which temperature at 10 hours half-life time being lower than the boiling point of bromine in a mixed-solvent of water and an organic solvent.

In the production process described above, it is preferable to use as a radical initiator at least one selected from a group consisting of 2,2'-azobis(4-methoxy-2,4 -dimethylvaleronitrile) and 2,2'-azobis(2,4 -dimethylvaleronitrile).

In the compound represented by the general formula (XVIII), as definite examples for the group represented by D, halogeno, such as fluorine, chlorine and bromine; optionally substituted alkyl, such as methyl, ethyl, isopropyl, methoxymethyl, methylthiomethyl, chloroethyl and trifluoromethyl; optionally substituted aralkyl, such as benzyl, 4-methoxybenzyl and 1-methylbenzyl; optionally substituted phenyl, such as phenyl, 4-chlorophenyl and 2,4-dimethylphenyl; optionally substituted heterocyclic group, such as 2-pyridyl, 6-chloro-2-pyridyl and 2-pyridylmethyl; optionally substituted alkoxy, such as methoxy, ethyoxy, isopropoxy, methoxymethoxy, chloroethoxy and trifluoromethoxy; hydroxy; nitrile; optionally substituted amino, such as amino, dimethylamino and methoxyamino; nitro; alkoxycarbonyl, such as methoxycarbonyl and t-butoxycarbonyl; carboxyl; formyl; optionally substituted acyl, such as acetyl, pivaloyl, phenacyl and 4'-methoxyphenacyl; a group selected from a substituent group consisting of groups represented by a formula of S(O)mR₃₂, wherein R₃₂ represents alkyl, aryl or heterocyclic group, and m represents 0, 1 or 2, or groups represented by the following formulas; 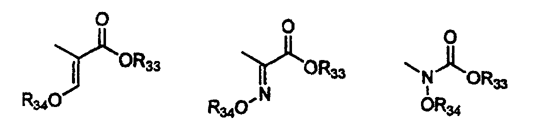 wherein R₃₃ and R₃₄ may be same or different each other and each represents optionally substituted alkyl, optionally substituted arlly, optionally substituted propargyl or optionally substituted aralkyl, can be given. As examples for the group represented by R₃₃ and R₃₄, optionally substituted alkyl, such as methyl, ethyl and isopropyl, optionally substituted allyl, optionally substituted propargyl or aralkyl optionally substituted with benzyl, 4-chlorobenzyl an the like can be given.

As the organic solvent used in the reaction, any solvent being no concern with radical reaction can be used without limitation, but it is preferable to use a solvent incompatible with water, and as the definite examples, an aromatic hydrocarbon, such as benzene and toluene, an aliphatic hydrocarbon, such as octane, a halogenated hydrocarbon, such as chlorobenzene and chloroform, and the like can be given, however, it is particularly preferable to use a halogenated hydrocarbon. The rate of water to use is preferably 10-50% by volume to the volume of the organic solvent used, and more preferably 20-40% by volume.

As the radical initiator used in the reaction, a radical initiator of which temperature at 10 hours half-life time is lower than the boiling point of bromine is used. This means the use of a radical initiator capable of efficiently decomposing at a temperature lower than the boiling point of bromine to generate radical species, and as the definite examples, 2,2'-azobis(4-methoxy-2,4-dimethylvaleronitrile) and 2,2'-azobis(2,4-dimethylvaleronitrile) can be given. These radical initiators are usable either solely or in appropriate combination. The rate of the radical initiator to be used is 0.1-10 mole % to the reaction substrate, and preferably 1-3 mole %.

The reaction according to the present constitution is carried out by dissolving the compound represented by the general formula (XVIII) in an organic solvent, adding water to the solution, heating the solution up to near the reaction temperature, then adding a radical initiator and feeding dropwise a solution of bromine and the radical initiator to the solution. The reaction temperature is set to a temperature lower than the boiling point of bromine and should be higher than a temperature at which the half-life of the radical initiator shows 10 hours, and it is particularly preferable to apply temperature being equal to a value of 10 hours half-life temperature plus 10-40°C. The rate of bromine to use is preferably 1-1.5 equivalent relative to the reaction substrate, and particularly preferable 1-1.3 equivalent.

The reaction is also carried out while controlling the generation of hydrogen bromide during the reaction by adjusting pH with an alkaline solution. As examples for the alkaline solution, an alkali metal hydroxide, such as sodium hydroxide and potassium hydroxide, an alkaline earth metal hydroxide, such as magnesium hydroxide and calcium hydroxide, and an alkali metal carbonate, such as sodium carbonate and potassium carbonate, can be given, however, it is preferable to use an alkali metal hydroxide. This procedure is preferably done in a pH range of from 0 to 8, and and more preferably in a range of from 0 to 3.

### Others:

As one of the raw materials to be used in the processes described in the constitutions 1 through 9, compounds represented by the following chemical structures is given as the example.

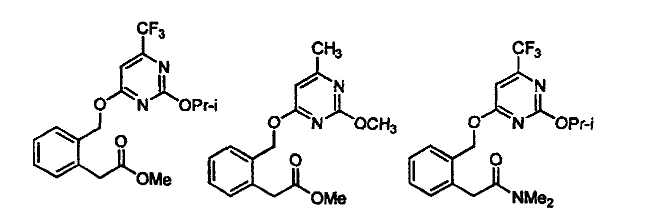

As a process to produce the compounds shown above, the following process can be given. 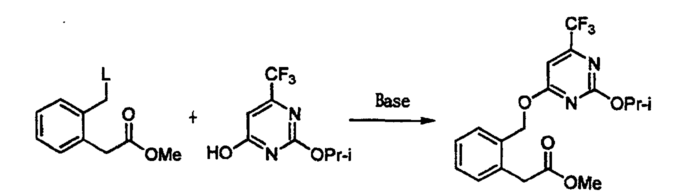 wherein L represents an eliminating group.

However, there is a problem that the yield of the objective O-alkylated compound is low due to the low O, N-selectivity, when the process above is carried out under conventional condition.

Then, it is found that the O-alkylation selectivity is improved by maintaining the concentration of the compound (or the concentration of the salt) containing a partial amide-like structure in the molecule and being used as a raw material for the reaction system at a level lower than a certain concentration.

Therefore, the present production process is to produce a compound containing a partial structure represented by a general formula (XXI); 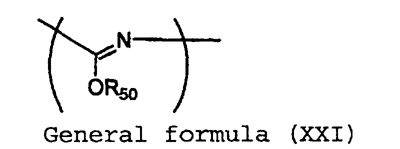 wherein R₅₀ represents optionally substituted alykl, optionally substituted allyl or optionally substituted aralkyl, in the molecule by subjecting a compound represented by a general formula (XX); 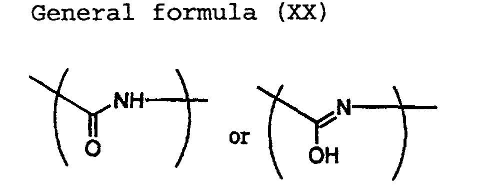 and a compound represented by a formula of R₅₀-L, wherein R₅₀ is as defined above and L represents an eliminating group, in an amide solvent in the presence of an alkali metal carbonate, and is a process to produce a compound containing a partial structure represented by the general formula (XXI) which contains a step to feed dropwise the solution of the compound containing the partial structure represented by the general formula (XX) in the molecule into either an amide solvent solution or a suspension of an alkali metal carbonate described above.

In the present constitutions, the step to feed dropwise a solution of a compound containing a partial structure represented by the general formula (XX) in the molecule into either an amide-type solvent solution or a suspension of the alkali carbonate described above is preferably a step to simultaneously feed dropwise a solution of the compound containing the partial structure represented by the formula (XX) in the molecule and solution of the compound represented by the formula of R₅₀-L into either an amide-type solvent solution or a suspension of the alkali carbonate.

In the production process described above, the compound containing the partial structure represented by the formula (I) in the molecule is preferably any of pyrimidone, pyridone or triazinone compound represented by a general formula (XXII); 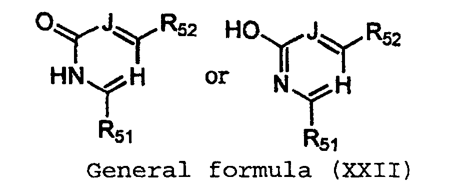 wherein H and J each independently represents CH or N, R₅₁ represents hydrogen, lower alkyl, haloalkyl or lower alkoxy, and R₅₂ represents hydrogen, lower alkyl or trifluoromethyl.

It is more preferable that the compound containing the partial structure represented by the general formula (XX) in the molecule is a group represented by the following general formula; 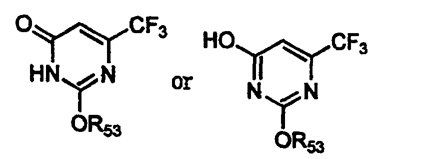 wherein R₅₃ represents lower alkyl.

Further, in the present constitution, it is preferable that the compound represented by the general formula of R₅₀-L is a compound represented by a general formula (XXIV); 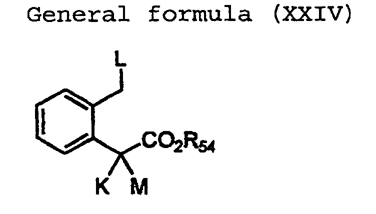 wherein L represents an eliminating group, R₅₄ represents lower alkyl, and K and M both represent hydrogen or combine to be a group of =O, =NOCH3 or =CHOCH3, or a compound represented by a general formula (XXV); 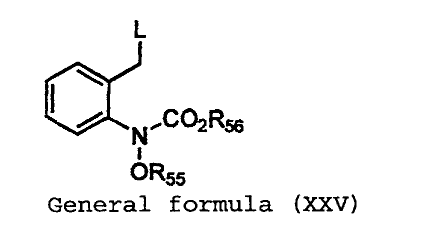 wherein R₅₅ and R₅₆ each independently represents straight-chained or branching lower alkyl, haloalkyl, cycloalkyl or aralkyl, and L is as defined above.

According to the production process described in the present constitution, various pyrimidyloxy derivatives and pyridyloxy derivatives, which are useful as intermediates for producing agricultural chemicals and pharmaceutical ingredients, can be efficiently, economically and advantageously produced at a high yield.

In the production process in the present constitution, there is no limitation for the compound containing the partial structure represented by the general formula (XX) in the molecule to be used as the starting material, namely the compound containing at least one -CONH group or the enol-form thereof in the molecule as the partial structure, if the compound has a structure capable of forming the salt with an alkali metal carbonate. As examples for such compound, in chain or cyclic compounds containing the following basic skeletons can be given.

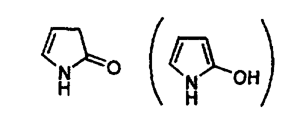

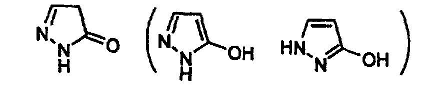

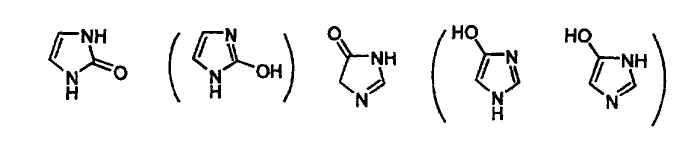

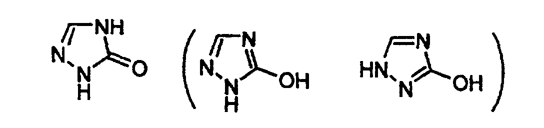

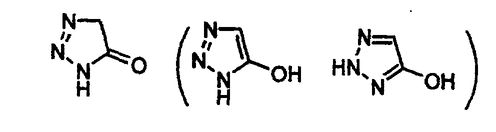

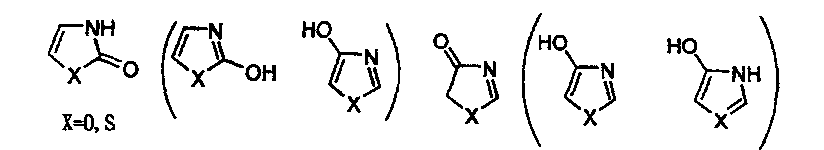

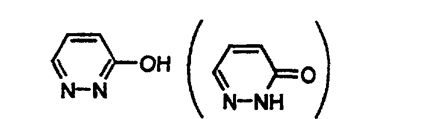

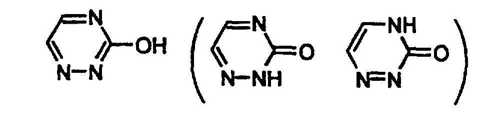

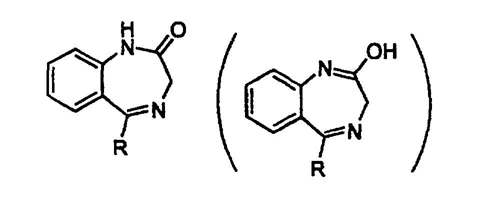

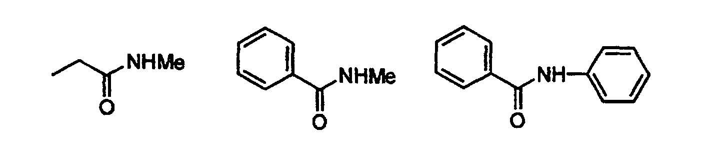

In the structures shown above, the ones in a parenthesis represent tautomers.

Among the examples shown above, by applying the production process of the present constitution to compounds containing pyrimidone skeleton, pyridone skeleton or triazinone skeleton, which are represented by a general formula (XXII); 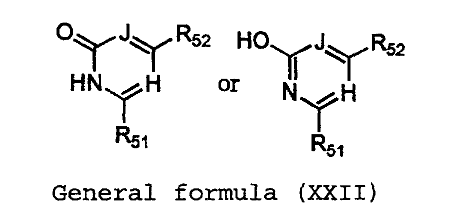 wherein H and J each independently represents CH or N, R₆₁ represents hydrogen, lower alkyl, such as methyl, ethyl, propyl, isopropyl, butyl, sec-butyl, t-butyl, pentyl and hexyl; haloalkyl, such as chloromethyl, dichloromethyl, trichloromethyl, fluoromethyl, difluoromethyl, trifluoromethyl, bromomethyl, dibromomethyl, tribromomethyl, 2,2,2-trifluoroethyl and pentafluoroethyl; or lower alkoxy, such as methoxy, ethoxy, propoxy, isopropoxy, butoxy, sec-butoxy and t-butoxy, and R₅₂ represents hydrogen, loweralkyl, such as methyl, ethyl, propyl, isopropyl, butyl, sec-butyl, t-butyl, pentyl and hexyl, or trifluoromethyl, pyrimidyloxy compounds, pyridyloxy compounds and triazinyloxy compounds useful as intermediates for producing agricultural chemicals or pharmaceutical ingredients can be produced.

As more definite examples for such compounds, the compounds in the following are given.

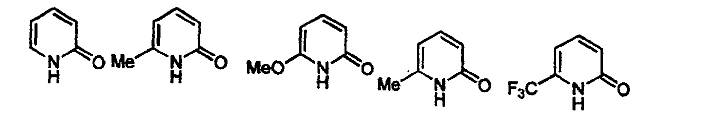

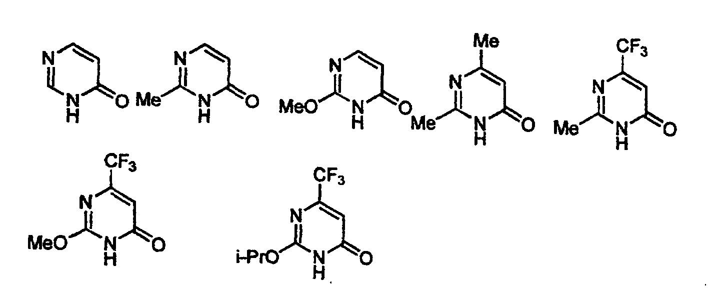

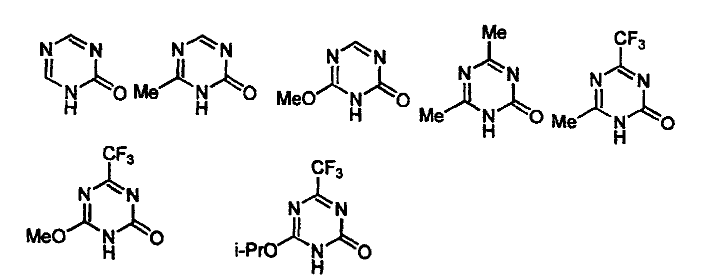

The compound represented by the general formula of R₅₀-L is an alkylating agent containing an eliminating group represented by L. Here, L represents halogeno or an eliminating group, such as optionally substituted arylsulfonic acid residue, and is preferably a group selected from a group consisting of chlorine, bromine, iodine and tosyloxy.

The group represented by R₅₀ is one relating to the structure to be required for the objective compounds, which are pyrimidyloxy derivative and pyridyloxy derivative, and there is no specific limitation for the group. For examples, an optionally substituted alkyl, an optionally substituted allyl and an optionally substituted aralkyl are given depending upon the objective compound to produce.

As the definite examples for the group represented by R₅₀, alkyl, such as methyl, ethyl, propyl, isopropyl, butyl, sec-butyl, t-butyl, pentyl, octyl and decyl; haloalkyl, such as trifluoromethyl and pentafluoromethyl; alkoxymethyl, such as methoxymethyl, methoxyethyl and t-butoxyethyl; alkylthioalkyl, such as methylthiomethyl, methylthioethyl, ethylthiopropyl and propylthiobuty; alkylsulfonylalkyl, such as methylsulfonylmethyl, ethylsulfonylethyl, ethylsulfonylpropyl and propylsulfonylmethyl; alkyl optionally substituted with alkoxycarbonylalkyl or the like including methoxycarbonylmethyl, ethoxycarbonylmethyl, methoxycarbonylethyl, ethoxycarbonylethyl and methoxycarbonylpropyl; optionally substituted allyl, such as allyl, 3-methoxyallyl, 2-methoxyallyl and 3-methoxycarbonylallyl; or aralkyl, such as benzyl, α -methylbenzyl, α, α -dimethylbenzyl and phenacyl, of which arbitrary position of the benzene ring is optionally substituted, can be given.

When using a compound represented by a general formula (XXIV) and a compound represented by a general formula (XXV); 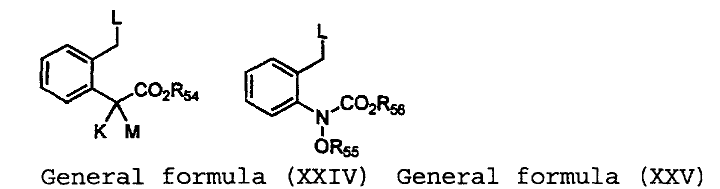 it is particularly useful since intermediates for producing agricultural chemicals and pharmaceutical ingredients can be obtained.

As the definite examples for the compounds represented by the formulas (XXIV) and (XXV), the following compounds are given.

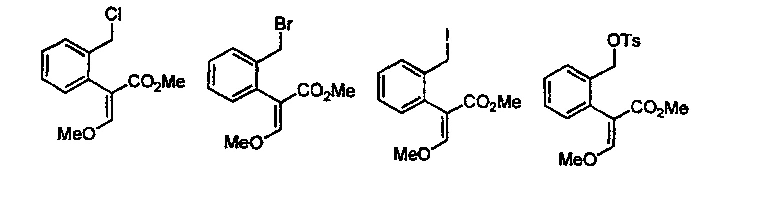

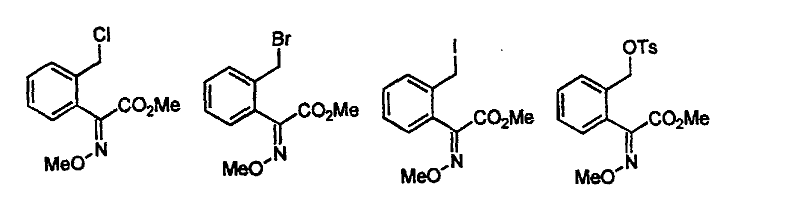

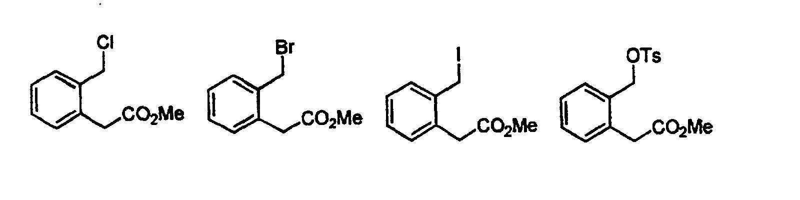

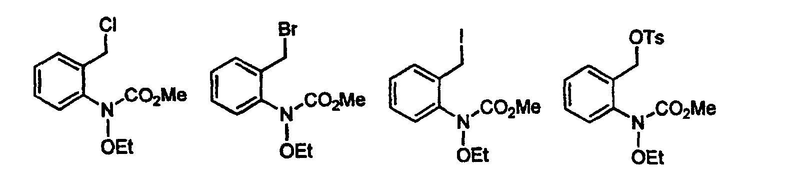

In the formulas shown above, Ts represents p-toluenesulfonyl.

The rate of the compound represented by the formula of R₅₀-L to be used is 1-10 equivalent, and preferably 1-2 equivalent, relative to the compound containing the partial structure represented by the general formula (XX) in the molecule in an amount of 1 mole.

As the amide-type solvent used in the production process according to the present constitution, N, N-dimethylformamide (DMF), N,N-dimethylacetoamide (DMA), N-methylpyrrolidone and the like can be given as the example.

As to the use amount of the solvent, though better O-selectivity is obtainable at more diluted concentration in general, the speed of the reaction could be slow under too much diluted condition, thus more amount of the solvent be required. The rate of the solvent to be used in the reaction is enough with more than 2 liters, and preferably 2-5 liters, relative to the compound containing the partial structure represented by the general formula (XX) in the molecule in an amount of 1 mole.

As examples for the alkali metal carbonate to be used in the present constitution, lithium carbonate, sodium carbonate, calcium carbonate, magnesium carbonate and the like can be given. Among the examples described above, it is particularly preferable to use potassium carbonate in view of general-purpose use, reaction selectivity, solubility to solvents, etc. The rate of the alkali carbonate to be used is in a range of from 1 to 10 equivalent, and preferably from 1 to 5 equivalent, relative to the compound containing the partial structure represented by the general formula (XX) in the molecule in an amount of 1 mole.

The production process of the present constitution is characterized by containing a step to feed dropwise a solution of the compound containing the partial structure represented by the general formula (XX) in the molecule into either an amide-type solvent solution or a suspension of the alkali carbonate.

The production process of the present constitution is carried out definitely as follows.

The first process is to feed dropwise a fixed amount of a solution of the compound containing the partial structure represented by the general formula (XX) in the molecule into a mixed solution (or a suspension) of an alkali carbonate and the compound represented by the formula of R₅₀-L.

In the process above, though there is no limitation for the feeding speed of the compound containing the partial structure represented by the general formula (XX) in the molecule, it is preferable to feed dropwise gradually in order not to elevate the concentration of the compound containing the partial structure represented by the general formula (XX) in the molecule in the reaction system.

The appropriate temperature for the reaction, namely the temperature of the mixed solution or suspension of the alkali carbonate and the compound represented by the formula of R₅₀-L when it is fed dropwise, is in a range of from -10°C to the boiling point of the solvent used, and preferably from an ambient temperature to 100°C.

As the solution for the compound containing the partial structure represented by the general formula (XX) in the molecule, any inert solvent capable of dissolving the said compound can be used without limitation, it is however preferable to use an amide-type solvent.

For improving the reacting performace, it is also preferable to add a small amount of bromine, iodine, potassium bromide, potassium iodide or the like into the reaction system. The rate of such halogen or halogen salt to add is approximately 0.001-1 mole relative to the compound represented by the formula of R₅₀-L in an amount of 1 mole.

The second process is to simultaneously feed dropwise a solution of a fixed amount of the compound containing the partial structure represented by the general formula (I) in the molecule and a solution of the compound represented by the formula of R₅₀-L into a solution or a suspension of the alkali carbonate.

This process is appropriate particularly when the compound represented by the formula of R₅₀-L is unstable to an alkali. This process enables to carry on O-alkylation reaction while controlling the concentration in the reaction system of the compound containing the partial structure represented by the general formula (XX) in the molecule and the compound represented by the formula of R50-L. Therefore, it is preferable to employ this process since the process can improve the O-selectivity while preventing the decomposition owing to the effect of the base in the compound represented by the formula of R₅₀-L. Further, the amount of the solvent to be used can be reduced by means of employing this process.

The third process is to feed dropwise a fixed amount of solution of the compound containing the partial structure represented by the general formula (XX) in the molecule and the compound represented by the formula of R₅₀-L into either a solution or a suspension of an alkali carbonate.

This process is particularly preferable when the compound represented by the formula of R₅₀-L is unstable to alkali. This process enables to carry on O-alkylation reaction while controlling the concentrations of the compound containing the partial structure represented by the general formula (XX) in the molecule and the compound represented by the formula of R₅₀-L in the reaction system, respectively. Namely, this process is preferable as it can improve the O-selectivity while preventing the decomposition of the compound represented by the formula of R₅₀-L caused by the base. Further, according to this process, it is allowed to reduce the amount of the solvent to be used.

Though there is no limitation for the solvent used for the compound containing the partial structure represented by the general formula (XX) in the molecule and the compound represented by the formula of R₅₀-L, any inactive solvent capable of dissolving the compound containing the partial structure represented by the general formula (XX) in the molecule and the compound represented by the formula of R₅₀-L can be used, it is preferable to use an amide-type solvent. The use of the same amide-type solvent throughout the reaction is preferable in view of easy handling, after-treatment operation, reaction yield and O-selectivity.

Further, it is preferable to add a small amount of bromine, potassium bromide, potassium iodide, etc. in the reaction system as it may improve the reaction speed. The rate of such element to add is approximately in a range of 0.001-1 mole relative to the compound represented by the formula of R₅₀-L in an amount of 1 mole.

### Best Mode for carrying out the Invention:

The reactions according to the present invention are explained in detail in the following, however, it should be noted that the present invention is not limited to the reactions described in the examples below. The values obtained by 1HNMR are measured values based on TMS.

### Example 1

Titanium tetrachloride in an amount of 8.54g was added to 20 ml of monochlorobenzene and the resulting mixture was cooled under an atmosphere of nitrogen gas. Methyl formate in an amount of 2.70g was gradually fed dropwise into the mixture at -12.5°C. The dropping was completed while spending 7 minutes, and the temperature inside the mixture elevated to 0°C due to exothermic reaction. After stirring the mixture for 9 minutes, a solution prepared by dissolving methyl 2-((2-isopropoxy-6 -trifluoromethylpyrimidine -4-yl)oxymethyl)phenylacetate in an amount of 11.53g in 10 ml monochlorobenzene was fed dropwise into said mixture at a temperature ranging from -10 to -6°C while spending 6 minutes. After stirring and aging the mixture at -5°C for 31 minutes, triethylamine in an amount of 9.11g was gradually fed dropwise in to the mixture. For this dropping at a temperature in a range of from -10 to -12°C, 27 minutes was required. After aging the mixture by 30 minutes stirring at -5°C, acetic acid in an amount of 1.80g was fed dropwise into the mixture at a temperature of from -5 to 1°C while spending 4 minutes to terminate the reaction, and then monochlorobenzene was added to the mixture to elevate the temperature of the mixture up to 19°C. 47 minutes was required for this temperature elevation. Then, the mixture was added with 12 ml water, then stirred at 19-30°C for 75 minutes and subsequently separated. 15 ml of 2N-hydrochloric acid was then added to the resulting organic solvent layer, and the layer was stirred for 25 minutes, then added with 4 ml monochlorobenzene followed by 5 minutes stirring, then stood in stationary state and separated. The organic solvent layer was then heated under reduced pressure, dehydrated, then distilled and added with monochlorobenzene in an amount of 22.76g to obtain monochlorobenzene solution of methyl 3-hydroxy-2-(2-((2-isopropoxy-6-trifluoromethyl pyrimidine-4-yl)oxymethyl)phenyl)acrylate.

Trimethyl orthoformate in an amount of 4.14g, methanesulfonic acid in an amount of 0.43g and methanol in an amount of 3.84g were added to monochlorobenzene solution of the obtained 3-hydroxy-2-(2-((2-isopropoxy-6 -trifluoromethylpyrimidine -4-yl)oxymethyl)phenyl)acrylic methyl, and the mixture was heated and then subjected to reflux for 2 hours. Then, the mixture was heated to raise the internal temperature up to 111°C while distilling out substance having a low boiling point, aged at 110-111°C for 122 minutes, then further aged at 111°C for 90 minutes following to the addition of methanesulfonic acid in an amount of 0.12g, again aged at 111°C for 90 minutes following to the addition of methanesulfinic acid in an amount of 0.12g, and again aged at 111°C for 30 minutes following to the addition of methanesulfonic acid in an amount of 0.12g. After cooling the temperature to an ambient temperature, the mixture was added with approximately 20 ml of monochlorobenzene to prepare the monochlorobenzene solution of methyl 3-methoxy-2-(2-((2-isopropoxy-6-trifluoromethylpyrimidine-4-yl )oxymethyl) phenyl) acrylate in an amount of 68.33g. The solution in an amount of 3.37g was collected, diluted with acetonitrile to obtain the solution in an amount of 7.20g and then quantitatively analyzed by using high speed liquid chromatography, thereby knowing that the solution contains 4% by weight of methyl 3-methoxy-2-(2-((2-isopropoxy-6 -trifluoromethylpyrimidine-4-yl)oxymethyl)phenyl)acrylate methyl. (Yield 55.9%)

### Example 2

Titanium tetrachloride in an amount of 222g was weighed, 100 ml of methylene chloride was then added thereto and cooled down to -2°C. Then, methyl formate in an amount of 70.3g was gradually fed dropwise into the cooled solution. 28 minutes were required to complete said dropping and the internal temperature of the solution came to in a range of from -2 to 3°C. The solution was further added with 400 ml methylene chloride solution of methyl 2- ((2-isopropoxy-6 -trifluoro methylpyrimidine-4-yl)oxymethyl)phenylacetate in an amount of 300g and stirred at a temperature lower than 0°C for 40 minutes, then the solution was gradually fed dropwise with triethylamine in an amount of 237g. 90 minutes were required to complete said dropping at the internal temperature of the solution was came to a range of from -5 to 1°C. The solution was then stirred and aged for 30 minutes at a temperature lower than 0°C and added with 624 ml of 3N hydrochloric acid. Owing to the generated heat, the internal temperature of the solution was raised to a temperature ranging from -2 to 28°C. The mixture was further heated to raise the temperature thereof up to 30°C for thoroughly dissolving the insoluble matter, then stood in stationary state and separated. The separated organic solvent layer was added with 624 ml of water, then separated again to obtain the methylene chloride solution of methyl 3-hydroxy-2-(2-((2-isopropoxy-6 -trifluoromethylpyrimidine-4-yl) oxy methyl)phenyl)acrylate. The methylene chloride solution was added with magnesium sulfate to dehydrate the solution, then filtrated, and the residue obtained by condensing the filtrate by using an evaporator was added with 624 ml of water to prepare the homogeneous solution. The crystals precipitated from the solution was washed with 117 ml of refrigerated methanol and dried under reduced pressure to obtain methyl 3-hydroxy-2-(2-((2-isopropoxy-6-trifluoromethylpyrimidine-4-yl)oxy methyl) phenyl)acrylare in an amount of 170.6g in crystalline form. (Yield 53.1%)
1HNMR(CDCl₃) δ 1,407(6H,d), 3.723(3H,s), 5.231-5.371(1H,m), 5.366(2H,s), 6.646(1H,s), 7.198(1H,d), 7.174-7.516(4H,m), 11.924(1H,d)

### Example 3

To methyl 3-hydroxy-2-(2-((2-isopropoxy-6-trifluoro methylpyrimidine-4-yl)oxymethyl)phenyl)acrylate in an amount of 6.80g, were added 30 ml of toluene, 7,5 ml of water and aqueous soliton of 50% benzyltributyl ammonium chloride in an amount of 0.32g, and the resulting solution was refrigerated down to 5°C. The solution was added with dimethyl sulfate in an amount of 3.78g and then gradually fed dropwise with an aqueous solution of 25% sodium hydroxide. This dropping required 5 minutes and the internal temperature of the solution was 5-7°C. Following to the dropping, the solution was heated to raise the temperature up to 10°C and stirred for 1 hour at 20°C to carry on reaction. The reacted solution was added with 3 ml of water and separated, and the obtained toluene layer was added with 7.5 ml of water to separate the toluene layer. The resulted organic solvent layer was added with magnesium sulfate to dehydrate it and filtrated to obtain the toluene solution of methyl 3-methoxy-2-(2-((2-isopropoxy-6-trifluoromethyl pyrimidine-4-yl)oxymethyl)phenyl)acrylate in an amount of 44.69g. The solution was quantitatively analyzed by using high speed liquid chromatography, and it is noted that the solution contains the desired compound at a rate of 9.44% by weight. (Yield 60.0%)

### Comparative Example 1

methyl 2-((2-isopropoxy-6-trifluoromethylpyrimidine-4-yl) oxymethyl)phenylacetate in an amount of 1.0g was dissolved in 5.2 ml of dimethylformamide, and the resulting solution was added with 60% sodium hydroxide in an amount of 0.11g at an ambient temperature and then aged at 32-40°C for 20 minutes. At this stage, the peak of the raw material, methyl 2-((2-isopropoxy -6-trifluoromethylpyrimidine-4-yl)oxymethyl)phenylacetate obtainable by high speed liquid chromatography analysis was disappreared, and a peak estimated for 2-isopropoxy-4 -hydroxy-6-trifluoromethylpyrimidine was appeared. The solution was then cooled down to 5°C, fed dropwise with 2.6 ml of methyl formate while spending 2 minutes, stirred and aged at 5-10°C for 183 minutes, and again at 10-20°C for 130 minutes, but no change was observed, and therefore, the desired methyl 3-hydroxy-2-(2-((2-isopropoxy-6-trifluoromethylpyrimidine-4-yl )oxymethyl)phenyl)acrylate was not obtained.

### Example 4

5 ml methylene chloride solution of trimethyl orthoformate in an amount of 1.27g was added to 5 ml methylene chloride solution of titanium tetrachloride in an amount of 2.28g at an ambient temperature. The resulting solution was stirred for 1 hour, then the reacted solution was cooled down to 0°C and was added with 5 ml methylene chloride solution of methyl 2-chloromethylphenylacetate in an amount of 1.58g. 30 minutes later, the solution was added with 5 ml methylene chloride solution of triethylamine in an amount of 2.4g and was subjected to a reaction for 1 hour. The reacted solution was washed with 24 ml of 1N hydrochloric acid and the organic solvent layer was dried with magnesium sulfate. Then, the solvent in the organic solvent layer was distilled out under reduced pressure to obtain the desired methyl 2-(2-chloromethyl phenyl) -3-hydroxyacrylate in an amount of 1.70g. (Yield 94%)

### Example 5

5 ml methylene chloride solution of methyl formate in an amount of 0.72g was added into 5 ml methylene chloride solution of titanium tetrachloride in an amount of 2.28g at an ambient temperature. The reacted solution was cooled down to 0°C and was added with 5 ml methylene chloride solution of methyl 2-chloromethylphenylacetate in an amount of 1.58g. 30 minutes later, the solution was added with 5 ml methylene chloride solution of triethylamine in an amount of 2.4g and was subjedted to a reaction for 1 hour. The reacted solution was washed with 24 ml of 1N hydrochloric acid and the resulting organic solvent layer was dried with magnesium sulfate. The solvent in the dried organic solvent layer was distilled out under reduced pressure to obtain the desired methyl 2-(2-chloromethyl phenyl) -3 -hydroxyacrylate in an amount of 1.72g. (Yield 96%)
1HNMR(CDCl₃) δ 3.74(s,3H,COOMe), 4.52 (s,2H,CH₂), 7.06-7.41(m,5H,Ar CH), 12.0(d,1H,OH)

### Example 6

Trimethyl orthoformate in an amount of 1.59g was added to 10 ml methylene chloride solution of aluminium chloride in an amount of 2.00g at 0°C. Promptly later, mrthyl 2-chloromethyl phenylacetate in an amount of 1.98g was added to the solution. 30 minutes later, the solution ws added with triethylamine in an amount of 3.33g at a temperature lower than 10°C and was subjected to a reaction for 1 hour. The reacted solution was washed with 50 ml of 2N hydrochloric acid and the resulting aqueous layer was extracted twice with each 10 ml of methylene chloride. The resulting organic solvent layer was dried with magnesium sulfate, and the solvent in the said layer was distilled out under reduced pressure to obtain oily substance in an amount of 2.02g. The content of the desired methyl 2-(2-chloromethylphenyl)-3-hydroxyacrylate in the oily substance was 38%, and the remaining was the raw material, which is methyl 2-chloromethylphenylacetate. (Yield 34%)

### Example 7

methyl 2-(2-chloromethylphenyl)-3-hydroxyacrylate in an amount of 1.72g was dissolved in 5 ml methanol, and the resulting solution was added with p-toluenesulfonic acid monohydrate in an amount of 0.2g. The obtained solution was subjected to reflux for 6 hours and was then added with 5 ml of p-xylene. The solution was further subjected to reflux for another 2.5 hours, and the substance therein having low boiling point lower than 30°C was distilled out under reduced pressure at 40 mmHg. The inside of the reaction system was replaced to at normal pressure, then the solution was subjected to reflux for approximately 1 hour. After cooling, the solution in an amount of 23.65g was obtained. By HPLC analysis, it is noted that the desired mrthyl 2-(2-chloromethylphenyl)-3-methoxyacrylate was contained in the solution at a rate of 4.08%. (Yield 52%)

### Example 8

Titanium tetrachloride in an amount of 342.0g, methylformate in an amount of 108.0g and methyl 2-chloro methyl phenylacetate in an amount of 238.4g were added in order of precedence into 1200 ml monochlorobenzene at a temperature lower than 10°C under an atmosphere of nitrogen gas. 30 minutes later, triethylamine in an amount of 363.6g was added to the resulting mixture, and methanol in an amount of 76.8g was then added thereto another 30 minutes later. Then, the reacted solution was added with 15% hydrochloric acid in an amount of 840g and 600 ml of monochlorobenzene in order of precedence. The resulting organic solvent layer was washed twice with 12% hydrochloric acid, and approximately 600 ml monochlorobenzene was distilled out under normal pressure to obtain the solution in an amount of 1564.5g. To said solution, were added p-toluenesulfonic acid monohydrate in an amount of 22.8g, methanol in an amount of 76.9g and trimethyl orthoformate in an amount of 165.6g, and the resulting solution was heated at 65-70°C for 5 hours. A distillation apparatus is equipped onto the reactor to keep the inner temperature of the solution at a temperature lower than 110°C, and the solvent in the solution was distilled out for 3.5 hours. After cooling the solution down to an ambient temperature, the reacted solution was washed with 479 ml of 2N hydrochloric acid, 479 ml of 5% sodium hydrogencarbonate solution and 479 ml of water in order of precedence. Approximately 240 ml chlorobenzene in the obtained water-containing solution was distilled out under reduced pressure at 75 mmHg, and cooling the solution to obtain a solution in an amount of 1488.6g. By HPLC analysis, it is noted that methyl 2-(2-chloromethylphenyl) -3-methoxyacrylate was contained in the solution at a rate of 17.08%. (Yield 88%)

Then, the solvent in the said solution in an amount of 532g was distilled out under reduced pressure. The obtained remain was added with 124 ml of ethyl acetate and 661 ml of hexane, and the mixture was then heated to prepare a homogeneous solution. The solution was then cooled down to 4-6°C, and precipitated crystals were separated by filtration. The crystals were washed with a mixed solvent of ethyl acetate and n-hexane and then dried to obtain methyl 2-(2-chloromethyl phenyl)-3-methoxyacrylate in crystalline form in an amount of 80.8g.
1HNMR(CDCl₃) δ 3.69(s,3H,COOCH₃), 3.80(s,3H,OCH₃), 4.49(s,2H,CH₂), 7.00-7.50(m,4H,Ar), 7.61(s,1H,CH)

### Example 9

To monochlorobenzene solution of methyl 2-(2-chloromethyl phenyl) -3-hydroxyacrylate in an amount of 137.31g, which is prepared according to the same process defined in the example 8 by using methyl 2-chloromethylphenylacetate in an amount of 19.87g, were added methanesulfonic acid an an amount of 12.80g and trimethyl orthoformate in an amount of 13.80g, and the resulting solution was subjected to a reaction for 5.5 hours while keeping the inner temperature of the solution to a range of from 67 to 68°C. The reacted solution was cooled and then washed with 5% sodium hydrogencarbonate solution and water in order of precedence. The resulting organic solvent layer was dried with magnesium sulfate, then, the solvent in the solution was distilled out to obtain yellowish oily substance in an amount of 29.11g. The obtained oily substance was purified by means of silica gel chromatography (Developping solvent: n-hexane/ethyl acetate) to obtain methyl 2-(2-chloromethyl phenyl) -3,3-dimethoxypropionate in an amount of 19.73g in white crystals form with the melting point of 63.5-64.5°C. (Yield 72%)
1HNMR(CDCl₃) δ 3.15(s,3H,OCH₃), 3.50(s,3H,OCH₃), 3.69(s,3H,COOCH₃), 4.34(d,1H,CH), 4.73(dd,2H,CH₂), 5.00(d,1H,CH), 7.25-7.61(m,4H,Ar)

The methyl 2-(2-chloromethylphenyl)-3,3-dimethoxy propionate in crystalline form in an amount of 2.73g was dissolved in 10 ml of monochlorobenzene. The resulting solution was added with methanesulfonic acid in an amount of 0.14g and then subjected to a reaction at 110°C for 40 minutes. The solution was then cooled to obtain a solution in an amount of 17.66g. By HPLC analysis, it is noted that methyl 2-(2-chloromethylphenyl) -3-methoxyacrylate is contained in the solution at a rate of 12.16%. (Yield 89%)

### Example 10

Methyl formate in an amount of 2.70g was added to 15 ml chlorobenzene solution of titanium tetrachloride in an amount of 8.54g under a temperature lower than 10°C. The reacted solution was then added with 15 ml chlorobenzene solution of methyl 2-bromomethylphenylacetatr in an amount of 7.29g while keeping the temperature of the solution at a temperature lower than 10°C. The solution was further stirred for 30 minutes at a temperature lower than 10°C, was added with triethylamine in an amount of 9.108 at a temperature lower than 10°C and subjected to a reaction for 30 minutes. Then, the reacted solution was added with 15 ml of chlorobenzene, acetic acid in an amount of 1.82g and 12 ml of water in order of precedence. Following to stirring the solution for 1 hour at an ambient temperature, the resulting organic solvent layer was condensed by distillation under reduced pressure and then dried. The condensed layer was added with 7.5 ml of chlorobenzene to adjust the volume of chlorobenzene to 30 ml. The monochlorobenzene solution of 2-(2-bromomethylphenyl)-3-hydroxyacrylic methyl obtained as described above was added with methanesulfonic acid in an amount of 0.43g, methanol in an amount of 3.84g and methylorthoformate in an amount of 4.14g in order of precedence, and the resulting solution was subjected a reaction at 55°C for 4 hours. The substance with low boiling point contained in the solution was distilled out while keeping the inner temperature of the solution at 110°C or lower by equipping a distillation apparatus onto the reactor and spending 2 hours. The residue obtained by distilling out the solvent under reduced pressure was purified by means of silica gel chromatography (Developing solvent: n-hexane/ethyl acetate) to obtain methyl 2- (2-bromo methylphenyl) -3-methoxyacrylate in an amount of 4.05g in white crystals form and having a melting point of 94-98°C. (Yield 47%)
1HNMR(CDCl₃) δ 3.70(s,3H, CH₃), 3.83(s,3H,COOCH₃), 4.41(s,2H,CH₂), 7.10-7.55(m,4H,Ar), 7.64(s,1H,CH)

### Example 11

Dimethyl sulfate in an amount of 1.51g was added to 20.37% chlorobenzene solution of methyl 2-[2-(2-isopropoxy-6- tri fluoromethylpyridine-4-yloxymethyl)phenyl]-3-oxypropionate in an amount 20.24g. Then the resulting solution was fed dropwise with a solution of 40% aqueous solution of tetrabutyl ammonium hydroxide in an amount of 8.43g and 12.37 ml of water at 14-16°C while spending approximately 10 minutes. After carrying on the reaction for 30 minutes at an ambient temperature, the reacted solution was analyzed by means of high pressur liquid chromatography, and it is noted that the raw material, propionic acid ester was completely consumed. After decomposing the excess dimethyl sulfate by stirring the reacted solution for 1 hour at the inner temperature of 50°C, the resulting organic solvent layer was separated out. The organic solvent layer was washed with 10 ml of water and dried with anhydrous magnesium sulfate to obtain a chlorobenzene solution in an amount of 28.6g.

The chlorobenzene solution was analyzed by means of high speed liquid chromatography, and it is noted that methyl 2-[2-(2-isopropoxy-6-trifluoromethylpyrimidine-4-yloxymethyl) phenyl]-3-(E)-methoxypropenate and the isomer, the methyl (Z)-methoxypropenate form thereof are obtainable at a yield of 86.9% and 0.6%, respectively.

### Example 12

To 20.37% chlorobenzene solution of methyl 2-[2-(2-isopropoxy-6- trifluoromethylpyrimidine-4-yloxymethyl) phenyl]-3-oxopropionate in an amount of 101.20g, were added 50% aqueous solution of benzyltributylammonium chloride in an amount of 0.95g and dimethyl sulfate in an amount of 7.55g, and the resulting solution was further fed dropwise with 5.85% aqueous solution of potassium hydroxide while spending 10 minutes. The solution was subjected to a reaction for 4 hours at the innner temperature of 50°C and then overnight at an ambient temperature. Then, the reacted solution was heated and stirred at the inner temperature of 50°C to decompose the dimethyl sulfate in the excess amount. The resulting organic solvent layer was taken out by separation and further washed and added with 50 ml of water. The resulting organic solvent layer was dried with anhydrous magnesium sulfate to obtain a chlorobenzene solution in an amount of 95.89g.

The chlorobenzene solution was analyzed by means of high speed liquid chromatography, and it is noted that methyl 2-[2-(2-isopropoxy-6-trifluoromethylpyrimidine-4-yloxymethyl) phenyl]-3-(E)-methoxypropenate and the isomer, the (Z) -methoxypropenic methyl form thereof are obtainable at a yield of 83.3% and 1.98%, respectively.

### Example 13

To a mixture consisting of 15 ml of monochlorobenzene, 15 ml of water, dimethyl sulfate in an amount of 4.54g and 50% aqueous solution of benzyltributylammonium chloride in an amount of 0.57g, were fed dropwise 28.56% chlorobenzene solution of 2-[2-(2-isopropoxy-6-trifluoromethylpyrimidine-4-yloxymethyl)p henyl] -3-oxopropionic methyl in an amount of 43.31g and 3.4% aqueous solution of sodium hydroxide in an amount of 46.4g at 10-15°C while spending 1 hour. The mixture was subjected to a reaction at 15°C for 30 minutes and then stirred for 1 hour following to elevating the inner temperature of the mixture up to 50°C. The resulting organic solvent layer was taken out by separation, then washed with 30 ml of water and dried with anhydrous magnesium sulfate.

The obtained organic solvent layer was analyzed by means of high speed liquid chromatography, and it is noted that methyl 2-[2-(2-isopropoxy-6-trifluoromethylpyrimidine-4-yloxy methyl)phenyl]-3-(E)-methoxypropenate and the isomer, the methyl (Z)-methoxypropenate thereof have been produced in the layer at a ratio of 95.7 : 0.7. The organic solvent layer was condensed under reduced pressure, and the obtained residue was subjected to the recrystalization with 40 ml of methanol and 10 ml of water to obtain pure methyl 2-[2-(2-isopropoxy -6-trifluoromethylpyrimidine-4-yloxymethyl)phenyl]-3-(E)-metho xypropenate in an amount of 10.77g. (Yield 87.5%) 1HNMR(CDCl₃) δ 1.35(d,6H), 3.60(s,3H), 3.70(s,3H), 5.20(dd,2H), 5.25(m,1H), 6.50(s,1H), 7.10-7.40(m,4H), 7.45(s,1H)

### Example 14

Methyl 2-[2-(2-isopropoxy-6-trifluoromethylpyrimidine -4-yloxymethyl)phenyl] -3-oxopropionate in an amount of 591.8g was dissolved in 2200 ml of benzene, and the resulting solution was added with dimethyl sulfate in an amount of 225.5g and benzyltributyl ammonium in an amount of 13.94g in order of precedence. The solution was then fed dropwise with 10% aqueous solution of lithium hydroxide in an amount of 510.28g at 25°C. After the dropping, the solution was stirred overnight at 25°C.

The reacted solution obtained was analyzed by means of high pressure liquid chromatography, it is noted that the raw material, oxopropionic acid ester, was completely consumed. The obtained products were found to be methyl 2-[2-(2-isopropoxy-6-trifluoromethylpyrimidine-4-yloxymethyl)phenyl]-3-(Z)-methoxyp ropenate and the methyl (E)-methoxypropenate form thereof, those which content ratio were 80.4 and 18.2, respectively. The organic solvent layer resulted was separated and washed with 1500 ml of water and then saturated saline solution in order of precedence, then dried with anhydrous magnesium sulfate and condensed under reduced pressure to obtain crude crystals in an amount of 641.1g. The crude crystal were subjected to two times recrystallization processes where a mixture of 3200 ml of n-hexane and 320 ml of ethyl acetate was used to obtain pure methyl 2-[2-(2-isopropoxy-6-trifluoromethylpyrimidine -4-yloxymethyl) phenyl] -3-(Z)-methoxypropenate in an amount of 395.6g.
1HNMR(CDCl₃) δ 1.40(d,6H), 3.65(s,3H), 3.86(s,3H), 5.28(m,1H), 5.37(dd,2H), 6.60(s,1H), 6.62(s,1H), 7.20-7.50(m,4H)

### Example 15

25% aqueous solution of sodium hydroxide in an amount of 6.24g was fed dropwise with 24.74% monochlorobenzene solution of methyl 2-[2-(2-isopropoxy-6-trifluoromethylpyrimidine -4-yloxymethyl) phenyl]-3-oxopropionate in an amount of 50.0g while spending 5 minutes. After the dropping, the resulting solution was stirred for 30 minutes to prepare the O-sodium salt of methyl 2- [2-(2-isopropoxy-6-trifluoromethylpyrimidine-4-yloxymethyl)phenyl] -3-oxopropionate, which salt is hereinafter called as "P-Na solution".

On the other hand, a solution prepared by adding dimethyl sulfate in an amount of 4.54g and benzyltributylammonium chloride in an amount of 0.57g into a mixed solvent of 56.2 ml water and 30 ml monochlorobenzene in order of precedence was prepared.

The P-Na solution prepared beforehand was fed dropwise into the mixture solution described above at 8-12°C while spending 1 hour. After the dropping, the stirring of the mixture solution was carried on, and the obtained reacted solution was analyzed by means of high pressure liquid chromatography, and it is noted that the rawmaterial, oxopropionic acid ester was completely consumed. The obtained products were found to be methyl 2-[2-(2-isopropoxy -6-trifluoromethylpyrimidine-4-yloxymethyl)phenyl]-3-(E)-metho xypropenate and the mathyl (Z)-methoxypropenate thereof and the ratio of these compounds are 235 : 1.

An organic solvent layer was separated from the reacted solution, and the organic solvent in the layer was distilled out under reduced pressure. The obtained residue was subjected to the recrystallization process using a mixture of 50 ml methanol and 9 ml water to obtain pure methyl 2-[2-(2-isopropoxy-6 -trifluoromethylpyrimidine-4-yloxymethyl) phenyl] -3- (E) -methoxypropenate in an amount of 8.8g. (Yield 75.4%)

### Example 16 : Preparation of 2-(2-chloromethylphenyl)-1-methoxy -1-trimethylsilyloxyethylene;

Trimethylsilyltriflate in an amount of 2.22g and triethylamine in an amount of 1.01g were dissolved in 10 ml of diethyl ether, and the resulting solution was stirred at an ambient temperature and relaced into a dropping funnel. The solution was then fed dropwise into 10 ml diethyl ether solution whereto methyl-2-chloromethylphenylacetate in an amount of 1.59g was dissolved. The resulting solution was then stirred for 4 hours at an ambient temperature and stood in stationary state, and the lower layer out of the resulting separated two layers was taken out by using a syringe, and ether in the remaining ether layer was distilled out at 0°C under reduced pressure to obtain the captioned compound in an amount of 2.73g.
1HNMR(CDCl₃) δ 0.19(9H,TMS), 3.68(3H,OCH₃), 4.60(2H,CH₂), 4.76(1H,CH), 7.18-7.26(2H,ArH), 7.68(1H,ArH)

### Example 17 : Preparation of methyl 2-chloromethylphenyl -3-methoxyacrylate;

2-(2-chloromethylphenyl)-1-methoxy-1 -trimethylsilyloxyethylene in an amount of 2.73g, which was obtained in the example 16, was added with 10 ml of dichloromethane, and the resulting solution was fed dropwise into a solution prepared by adding titanium tetrachloride in an amount of 1.9g and methyl orthoformate in an amount of 1.1g into 20 ml of dichloromethane at 0°C.

The resulting solution was subjected to a reaction at an ambient temperature for 2 hours, and the reacted solution was analyzed by means of high pressure liquid chromatography, and it is noted that the desired methoxymethylene form was produced at a content of 65.9%. An extraction process was carried out following to adding water and hydrochloric acid for prparing the reacted mixture obtained into acidic. The extracted organic solvent layer was washed with water and dried with magnesium sulfate, and the solvent in the layer was distilled out to obtain the captioned compound.
1HNMR(CDCl₃) δ 3.22(3H,CH₃), 3.34(3H,CH₃), 4.00(2H,CH₂), 7.24(1H,CH)

### Comparative Example 2 : Preparation of 2--(2-chloromethylphenyl) -1-methoxy-1-trimethylsilyloxyethylene;

Trimethylsilyltriflate in an amount of 2.22g and triethylamine in an amount of 1.01g were dissolved in 10 ml of dichloromethane, and the resulting solution was stirred for 10 minutes and subsequently replaced into a dropping funnel. The solution was then fed dropwise into a solution prepared by dissolving methyl-2-chloromethylphenylacetate in an amount of 1.59g in 10 ml of dichloromethane at 0°C. The solution was stirred for 4 hours at an ambient temperature, and the obtained dichloromethane solution of ketenesilylacetal was fed dropwise into 10 ml dichloromethane solution whereto titanium tetrachloride in an amount of 1.9g and trimethyl orthoformate in an amount of 1.1g were added at 0°C.

The resulting solution was subjected to a reaction for 12 hours at an ambient temperature, and the reacted solution was analyzed by means of high pressure liquid chromatography, by which it is noted that the desired methoxymethylene form is obtained at a yield of 13.0%.

### Example 18 : Preparation of (E)-4-methoxymethylene-3-isochromanone ;

Methyl formate in an amount of 3.61g was added to 60 ml chlorobenzene solution of 3-isochromanone in an amount of 2.96g, and the resulting slurry was gradually added with sodium methylate in powder in an amount of 2.16g at 0-5°C while spending 5 minutes. The reacted slurry was stirred for 1 hour at 0-5°C and then added with 59% aqueous solution of benzyltributylammonium chloride in an amount of 0.37g, 10 ml of water and dimethyl sulfate in an amount of 5.04g in order of precedence. During 2 hours stirring at 25°C, the slurry was gradually dissolving to form a bilayer solution comprising water and chlorobenzene. After completion of the reaction, the organic solvent layer was separated and dried with anhydrous magnesium sulfate. The obtained filtrate was quantitatively analyzed by means of high pressure liquid chromatography, and it is noted that the desired (E)-4-methoxymethylene -3-isochromanone is contained in the filtrate at a content of 89.6%.

### Reference Example 1 : Preparation of methyl (E)-3-methoxy-2-(2-chloromethylphenyl)acrylate;

Methyl formate in an amount of 7.21g was added to 120 ml toluene solution of 3-isochromanone in an amount of 5. 92g, and the resulting slurry was added with sodium methylate in powder in an amount of 4.32g while spending 5 minutes. The reacted slurry was stirred for 1 hour at 0-5°C and then added with 50% aqueous solution of benzyltributylammonium chloride in an amount of 0.75g, 25% aqueous solution of sodium hydroxide in an amount of 8.32g, 20 ml of water and dimethyl sulfate in an amount of 7.57g in oreder of precedence. During stirring the slurry for 2 hours at 21-28°C, the slurry was gradually dissolving to form a bilayer solution comprising water and chlorobenzene. After confirming the completion of the reaction by using high pressure liquid chromatography, the temperature of the reaction system was raised to 50°C and the reaction system was stirred for 1 hour. By analysis of the reacted solution by means of gas chromatography, it is confirmed that no un-reacted dimethyl sulfate is remained in the reacted solution.

After cooling the reacted solution, the organic solvent layer was taken out by separation and was washed with 20 ml of water. The separated organic solvent layer was added with 40 ml of toluene and condensed by dehydration under reduced pressure to obtain toluene solution containing (E)-4-methoxymethylene-3-isochromanone. The toluene solution was quantitatively analyzed by means of high pressure liquid chromatography, and it is noted that the desired (E)-4-methoxymethylene-3-isochromanone is contained in the toluene solution at a concentration of approximately 21%. (Yield 85.5%)

The toluene solution was then added with thionyl chloride in an amount of 27.7g and N,N-dimethylformamide in an amount of 0.12g, and the resulting solution was subjected to a reaction at 75°C. The excess thionyl chloride was distilled out under reduced pressure, and the obtained solution was then added with 8 ml of toluene and continuously subjected to distillation under reduced pressure until the inner temperature of the solution comes to 46°C (75 mmHg). The residue obtained was added with 12 ml of toluene and replaced into a dropping funnel.

Then, the toluene solution was fed dropwise into a mixture of methanol in an amount of 8.57g and triethylamine in an amount of 4.06g at -15°C by using a dropping funnel, and after the dropping, the solution was further subjected to a reaction at -5°C for 2 hours. At this stage, nohalz formation was observed. The resulting organic solvent layer was dried with anhydrous magnesium sulfate, and the filtrate was then quantitatively analyzed by means of high pressure liquid chromatography, which showed that the desired methyl (E)-3-methoxy-2-(2-chloro methylphenyl)acrylate was obtained at a yield of 78.34%.

### Reference Example 2 : Preparation of methyl (E)-3-methoxy-2 - (2-chloromethylphenyl)acrylate;

Titanium tetrachloride in an amount of 2.09g and 15 ml of chloroform were added to the reaction system while flowing nitrogen gas into a 100 ml volume two-inlets type flask and then cooling the reaction system to -5°C. Then, methyl formate in an amount of 0.69g was gradually added to the reaction system while stirring and added with 3-isochromanone in an amount of 1.48g at one go-off. The resulting solution was stirred at -5°C for 30 minutes, and triethylamine in an amount of 2.53g was fed dropwise into to the solution while spending 10 minutes. After the dropping, the solution was stirred at 0°C for 1 hour to complete the reaction. The reacted solution was then gradually fed dropwise with 5 ml aqueous solution of 35% hydrochloric acid in an amount of 1.04g to discontinue the reaction. The resulting organic solvent layer was taken out by separation and washed with 5 ml aqueous solution of 35% hydrochloric acid in an amount of 1.04g, and the obtained chloroform solution was dried with anhydrous magnesium sulfate and then condensed under reduced pressure to obtain reddish oily substance in an amount of 1.6g.

The obtained reddish oily substance was dissolved in 20 ml of toluene, and the resulting solution was added with 50% aqueous solution of benzyltributylammonium chloride in an amount of 0.20g, 15% aqueous solution of sodium hydroxide in an amount of 2.1g and dimethyl sulfate in an amount of 1.90g in order of precedence, then stirred at an ambient temperature for 2 hours. After checking the completion of the reaction by means of high speed liquid chromatography, the reaction system was heated to 50°C and then stirred for 1 hour. The reacted solution was analyzed by means of gas chromatography, and it is noted that no un-reacted dimethyl sulfate was remained therein.

The reacted solution was cooled down to an ambient temperature, and the organic solvent layer was separated and then washed with 20 ml of water. Then the separated organic solvent layer was added with 40 ml of toluene and condensed by dehydration under reduced pressure to obtain the toluene solution containing (E)-4-methoxymethylene-3-isochromanone. The toluene solution was quantitatively analyzed by means of high pressure liquid chromatography, and it is noted that the desired (E)-4-methoxymethylene-3-isochromanone is contained in the toluene solution at a concentration of 21%. (Yield 92%)

Then, the toluene solution was added with thionyl chloride in an amount of 6.0g and N,N-dimethylformamide in an amount of 0.01g in order of precedence, and the resulting solution was subjected to a reaction at 75°C for 5 hours. Excess thionyl chloride was distilled out from the solution under normal pressure and then under reduced pressure, and the solution was then added with 5 ml of toluene and carried on for the distillation under reduced pressure until the inner temperature of the solution comes to 46°C (75 mmHg). The obtained residue was added with 5 ml of toluene and then replaced into a dropping funnel.

The toluene solution was then fed dropwise into amixture solution of methanol in an amount of 1.78g and triethylamine in an amount of 0.85g by using a dropping funnel, and the mixture solution was subjected to a reaction at -5°C for 2 hours following to the dropping. Then, the reacted solution was added with 10 ml of water and 10 ml of toluene and then separated. At this stage, no halz formation was observed. The resulting organic solvent layer was dried with anhydrous magnesium sulfate, and the obtained filtrate was quantitatively analyzed by means of high speed liquid cgromatography, and it is noted that the desired methyl (E)-3-methoxy-2-(2-chloromethyl phenyl)acrylate is obtained in the filterate at a yield of 81%.

### Reference Example 3 : Preparation of methyl (E)-3-methoxy-2-(2-chloromethylphenyl)acrylate;

Methyl formate in an amount of 7.21g was added to 120 ml toluene solution of 3-isochromanone in an amount of 5.93g, and the resulting slurry was added with sodium methylate in powder in an amount of 4.32g at a temperature ranging from -2 to 5°C while spending 5 minutes. The reacted slurry was stirred at 0-5°C for 3 hours and added with 50% aqueous solution of benzyltributylammonium chloride in an amount of 0.75g, 25% aqueous solution of sodium hydroxide in an amount of 7.04g, 20 ml of water and dimethyl sulfate in an amount of 11.10g in order of precedence. During stirring the slurry at 25°C for 2 hours, the slurry was gradually dissolved to produce a bilayer solution of water and chlorobenzene. The toluene layer obtained by separation was washed with 20 ml of water, and the resulting organic layer was added with 40 ml of toluene, condensed by dehydration under reduced pressure to obtain a toluene solution containing (E)-4-methoxymethylene-3-isochromanone. The toluene solution was quantitativelyanalyzedbymeans of high pressure liquid chromatography, and it is noted that the solution contains (E)-4-methoxymethylene-3-isochromanone at a concentration of 24% (Yield 83%). Further, by conducting analysis by means of gas chromatography, it is noted that approximately 40% of the used dimethyl sulfate was remained as un-reacted in the solution.

Then, the toluene solution was added with thionyl chloride in an amount of 27.7g and N,N-dimethylformamide in an amount of 0.12g, and the resulting solution was subjected to a reaction at 75°C for 5 hours. After distilling out excess thionyl chloride in the solution, the obtained solution was added with 8 ml of toluene and subjected to distillation under reduced pressure until the inner temperature of the solution comes to 46°C (75 mmHg). The obtained residue was added with 12 ml of toluene and replaced into a dropping funnel.

The obtained toluene solution was then fed dropwise into a mixture of methanol in an amount of 8.62g and triethylamine in an amount of 3.91g at -5°C by using a dropping funnel, and subjected to a reaction at -5°C for 2 hours following to the dropping. The reacted solution was added with 20 ml of water and 20 ml of toluene and then separated while separating black halz by decantation work. The resulting organic solvent layer was dried with anhydrous magnesium sulfate, and the obtained filtrate was quantitatively analyzed by means of high speed liquid chromatography, and it is noted that the desired methyl (E)-3-methoxy-2-(2-chloromethylphenyl)acrylate can be obtained at a yield of 25%.

### Example 19

Methyl formate in an amount of 2.70g was added to 15 ml chlorobenzene solution of titanium tetrachloride in an amount of 8.54g at a temperature lower than 10°C. The reacted solution was then added with 15 ml chlorobenzene solution of methyl 2-bromomethylphenylacetate in an amount of 7.29g while keeping the temperature of the reacted solution at lower than 10°C. The solution was stirred for 30 minutes at a temperature lower than 10°C, then added with triethylamine in an amount of 9.10g under a temperature lower than 10°C and subjected to a reaction for 30 minutes. The reacted solution was added with 15 ml of chlorobenzene and then fed dropwise with acetic acid in an amount of 1.82g. After adding 12 ml of water, the resulting solution was stirred at an ambient temperature for 1 hour, and the resulting organic solvent layer was taken out by separation. The amount of the water was 270 ml/mol relative to titanium tetrachloride, and separating condition was good. The organic solvent layer was then washed with 18 ml of 2N hydrochloric acid. The organic solvent layer was then condensed by distillation under reduced pressure and dried to obtain monochlorobenzene solution of methyl 2-(2-bromomethylphenyl)-3-hydroxyacrylate. (Yield 94%)

### Example 20

78 ml of chlorobenzene was cooled down to -5°C in an atmosphere of nitrogen gas. The cooled chlorobenzene was added with titanium tetrachloride in an amount of 22.2g and then fed dropwise with methyl formate in an amount of 7.03g. The resulting solution was then added with 41.4% chlorobenzene solution of methyl 2-(2-isopropoxy-6-trifluoromethyl pyrimidine-4-yloxymethyl) phenylacetate in an amount of 72.4g. The solution was stirred for 30 minutes at -5°C and then fed dropwise with triethylamine in an amount of 23.7g. The solution was aged for 30 minutes and then fed dropwise with acetic acid in an amount of 14.1g. Then, the solution was continuously stirred for 30 minutes at an ambient temperature and then gradually added with 31 ml of water. After stirring the solution for 30 minutes at an ambient temperature, the reacted solution was replaced into a 250 ml volume measuring cylinder. Time required for separating into water layer and organic solvent layer was 20 minutes. The organic solvent layer was further washed with 18 ml of 2N hydrochloric acid. The organic layer was then condensed by distillation under reduced pressure and dried to obtain monochlorobenzene solution of methyl 2-(2-isopropoxy-6 -trifluoromethylpyrimidine-4-yloxymethyl)-3-hydroxyacrylate. (Yield 96%)

### Reference Example 4

15.7% by weight chlorobenzene solution of methyl 2-(2-methylphenyl)-3-methoxyacrylate in an amount of 131.12g was added with 40 ml of water and then heated while stirring. When the inner temperature of the solution elevated to 50°C, the solution was added with a radical initiator, 2,2'-azobis(4-methoxy-2,4 -dimethylvaleronitrile) in an amount of 0.31g, and the solution was then heated up to at 55°C. Then, the solution was continuously fed dropwise with a solution prepared by dissolving bromine in an amount of 19.98g and 2,2'-azobis(4-methoxy-2,4 -dimethylvaleronitrile) in an amount of 0.62g in 40 ml of chlorobenzene while keeping the inner temperature of the solution. For this dropping, 75 minutes were required. The solution was stirred and aged for 30 minutes while keeping the temperature, then cooled down to 25°C and separated. The separated organic solvent layer was washed with 40 ml of 5% by weight sodium hydrogencarbonate solution and 40 ml of water, separated and dehydrated with magnesium sulfate. The used magnesium sulfate was separated by filtration, and the insoluble substance was thoroughly washed with 200 ml of chlorobenzene to obtain chlorobenzene solution of 2-(2-bromomethylphenyl)-3-methoxyacrylic methyl. The obtained chlorobenzene solution was quantitatively analyzed by means of high pressure liquid chromatography, and it is noted that the solution contains methyl 2-(2-bromomethylphenyl)-3-methoxy acrylare at a rate of 11.80% by weight. (Yield 79.3%)

### Reference Example 5

15.7% by weight chlorobenzene solution of 2-(2-methyl phenyl)-3-methoxyacrylic methyl in an amount of 131.11g was added with 40 ml of water at an ambient temperature, and the resulting solution was heated while stirring. When the inner temperature of the solution elevated to 50 °C , a radical initiator, 2,2'-azobis(4-methoxy-2,4-dimethylvaleronitrile) in an amount of 0.31g was added to the solution, and after the inner temperature elevated to 55°C, a solution prepared by dissolving bromine in an amount of 19.98g and 2,2'-azobis(4-methoxy -2,4-dimethylvaleronitrile) in an amount of 0.62g into 30 ml of chlorobenzene and 25% aqueous solution of sodium hydroxide were simultaneously fed dropwise into the solution while keeping the inner temperature at 55-56°C and the pH at 2-3. For ths dropping, 66 minutes were required. The solution was stirred and aged for 30 minutes at 55°C, then cooled down and then separated. The separated organic solvent layer was washed with 40 ml of 5% by weight sodium hydrogencarbonate solution and 40 ml of water, separated and dehydrated with magnesium sulfate. The used magnesium sulfate was separated by filtration to obtain chlorobenzene solution of methyl 2-(2-bromomethylphenyl)-3-methoxyacrylate in an amount of 192.87g. The obtained chlorobenzene solution was quantitatively analyzed by means of high pressure liquid chromatography, and it is noted that the solution contains methyl 2-(2-bromomethylphenyl)-3-methoxyacrylate at a rate of 10.63% by weight. (Yield 71.9%)

### Reference Example 6

15.7% by weight chlorobenzene solution of methyl 2-(2-methylphenyl)-3-methoxyacrylate in an amount of 131.11g was added with 10 ml of water, and the resulting solution was heated while thoroughly stirring. When the inner temperature of the solution elevated to 50°C, the solution was added with a radical initiator, 2,2'-azobis(4-methoxy-2,4 -dimethylvaleronitrile) in an amount of 0.31g. After the temperature elevation up to 55°C, the solution was simultaneously fed dropwise with a solution prepared by dissolving bromine in an amount of 19.98g and 2,2'-azobis(4-methoxy-2,4 -dimethylvaleronitrile) in an amount of 0.62g in 30 ml of chlorobenzene and 8.33% aqueous solution of sodium hydroxide while keeping the inner temperature at 55-59°C and the pH of the solution at a range of from 5 to 8. For this dropping, 65 minutes were required. The solution was then stirred and aged for 30 minutes, cooled and then separated. The separated organic solvent layer was washed with 40 ml of 5% by weight sodium hydrogencarbonate solution and 40 ml of water, separated and the dehydrated with magnesium sulfate. The used magnesium sulfate was separated by filtration from the solution and chlorobenzene solution of methyl 2-(2-bromomethylphenyl)-3 -methoxyacrylate in an amount of 191.83g ws obtained from the solution. The obtained chlorobenzene solution was quantitatively analyzed bymeans of high pressure liquid chromatography, and it is noted that the solution contains methyl 2- (2-bromomethylphenyl) -3 -methoxy acrylate at a rate of 9.06% by weight. (Yield 60.1%)

### Reference Example 7

15.7% by weight chlorobenzene solution of methyl 2-(2-methylphenyl)-3-methoxyacrylate in an amount of 131.35g was added with 40 ml of water at an ambient temperature, and the resulting solution was added with a radical intiator, 2,2'-azobis(4-methoxy-2,4-dimethylvaleronitrile) and heated while stirring. When the inner temperature of the solution elevated to 55°C, a solution prepared by dissolving bromine in an amount of 19.98g in 30 ml of chlorobenzene was fed dropwise into the solution while keeping the inner temperature at 55-57°C. For this dropping, 60 minutes were required. The solution was stirred and aged for 30 minutes while keeping the inner temperature, then cooled down to 25°C and then separated. The separated organic solvent layer was washed with 40 ml of 5% by weight sodium hydrogencarbonate solution and 40 ml of water, separated and dehydrated with magnesium sulfate. The magnesium sulfate was separated by filtration from the solution and the solution was washed with 20 ml of chlorobenzene to obtain chlorobenzene solution of methyl 2-(2-bromomethylphenyl)-3-methoxyacrylate in an amount of 201.03g. The obtained chlorobenzene solution was quantitatively analyzed by means of high pressure liquid chromatography, and it is noted that the solution contains methyl 2-(2-bromomethylphenyl)-3-methoxy acrylate at a rate of 9.76% by weight. (Yield 68.8%)

### Reference Example 8

18.06% by weight chlorobenzene solution of methyl 2-(2-methylphenyl)-3-methoxyacrylate in an amount of 262.0g was added with 80 ml of water at an ambient temperature, and the resulting solution was added with AIBN in an amount of 0.33g and then heated while stirring. When the inner temperature of the solution elevated to 85°C, the solution was fed dropwise with a solution prepared by mixing bromine in an amount of 40.0g in 100 ml of chlorobenzene while spending 9 minutes. At his stage, the inner temperature of the solution raised up to 98.5°C and the solution started to reflow. The solution was stirred and aged for 30 minutes under reflux, cooled down to 30°C and then separated. The obtained organic solvent layer was washed with 40 ml of water, then separated and dehydrated with magnesium sulfate. After separating out the magnesium sulfate by filtration from the solution, chlorobenzene solution of methyl 2- (2-bromomethylphenyl)-3-methoxyacrylate in an amount of 417.10g was obtained. The chlorobenzene solution was quantitatively analyzed by means of high speed liquid chromatography, and it is noted that the solution contains methyl 2-(2-bromomethylphenyl)-3-methoxyacrylate at a content of 8.89% by weight. (Yield 65.0%)

### Reference Example 9

15.7% by weight chlorobenzene solution of methyl 2-(2-methylphenyl)-3-methoxyacrylate in an amount of 131.35g was heated while thoroughly stirring. When the inner temperature of the solution elevated to 50°C, the solution was added with a radical initiator, 2,2'-azobis(4-methoxy-2,4 -dimethylvaleronitrile) in an amount of 0.62g, and when the inner temperature further elevated to 55°C, the solution was fed dropwise with a solution prepared by mixing bromine in an amount of 19.98g and 2,2'-azobis(4-methoxy-2,4 -dimethylvaleronitrile) in an amount of 0.62g in 40 ml of chlorobenzene while keeping the inner temperature at 55-56°C. For this dropping, 65 minutes were required. The resulting solution was then stirred and aged for 30 minutes while keeping the inner temperature, colloed down to 25°C and separated after adding 40 ml of water. The separated organic solvent layer was washed with 40 ml of 5 wt% sodium hydrogencarbonate solution and 40 ml of water, then separated and dehydrated with magnesium sulfate. After separating out the magnesium sulfate by filtration from the solution, the solution was washed with 20 ml of chlorobenzene to obtain the chlorobenzene solution of methyl 2- (2-bromomethylphenyl)-3-methoxyacrylate in an amount of 203.36g. The chlorobenzene solution was quantitatively analyzed by means of high pressure liquid chromatography, and it is noted that the solution contains methyl 2-(2-bromomethyl phenyl)-3 -methoxyacrylate at a content of 0.48% by weight. (Yield 3.4%)

### Reference Example 10 : Preparation of methyl 3-methoxy-2-[2-(2-isopropoxy-6-trifluoromethylpyrimidine-4-yloxymethyl)-phenyl]acrylate;

25 ml of DMF solution prepared by dissolving the residue obtained by distilling the solvent contained in the chlorobenzene solution of methyl 2-chloromethylphenyl-3-methoxyacrylate in an amount of 65.58g (16.68 wt%) under reduced pressure and 2-isopropoxy-6-trifluoromethyl-4-hydroxy pyrimidine in an amount of 11.11g in DMF was fed dropwise while spending 2 hours into a solution composed of potassium carbonate in fine powder in an amount of 5.53g, potassium iodide in an amount of 0.25g and 50 ml of DMF having been maintained at 90°C by heating. After the dropping, the resulting solution was stirred at 90°C for 4 hours and then cooled down to an ambient temperature, and the impurity therein was separated out by filtration. The solvent in the solution was distilled out under reduced pressure, and the solution was subjected to crystallization by using a mixed solvent of 69.5 ml of methanol and 13.8 ml of water. The obtained crystals were washed with a mixed solvent of 20.1 ml of methanol and 4.1 ml of water, then collected by filtration and dried at 50°C for 12 hours to obtain the captioned compound in an amount of 13.2g.

The yield was 62.1% based on 2-isopropoxy-6-trifluoromethyl -4-hydroxypyrimidine, and the melting point was 109-110°C.

### Reference Example 11 : Preparation of methyl 3-methoxy-2-[2-(2 -isopropoxy-6-trifluoromethylpyrimidine-4-yloxymethyl) phenyl]acrylate;

A residue obtained by distilling under reduced pressure the solvent in chlorobenzene solution of methyl 2-chloromethyl phenyl -3-methoxyacrylate in an amount of 65.58g (16.68 wt%) was dissolved in 25 ml of DMF. On the other hand, 2-isopropoxy -6-trifluoromethyl-4-hydroxypyrimidine in an amount of 11.11g was dissolved in 25 ml of DMF. These two solutions were simultaneously fed dropwise while spending 2 hours into a solution prepared by mixing potassium carbonate in fine powder in an amount of 5.53g and potassium bromide in an amount of 0.18g with 25 ml of DMF and maintained at 110°C by heating. The resulting solution was stirred at 110°C for 4 hours and then cooled down to an ambient temperature, and the insoluble materials therein were separated out by filtration. Then, the solution was subjected to the same procedure described in the example 1 to obtain the captioned compound in an amount of 12.9g. (Yield 60.5%)

### Reference Example 12 : Preparation of methyl 3-methoxy-2-[2-(2 -isopropoxy-6-trifluoromethylpyrimidine-4-yloxymethyl) phenyl]acrylate;

methyl 2-Chloromethylphenyl-3-methoxyacrylate in an amount of 14.97g containing toluene at a rate of 26.6 wt%, 2-isopropoxy-6-trifluoromethyl-4-hydroxypyrimidine in an amount of 11.11g and potassium carbonate in fine power in an amount of 5.53g were admixed with DMF, and the resulting mixture was stirred for 7.5 hours at 120°C. The reacted mixture was cooled down to an ambient temperature, then the insoluble materials therein were removed by filtration. The captioned compound in an amount of 10.4g was obtained after subjecting the mixture to the same procedure as described in the Reference Example 11, however, the crystals of said compound was found to be colored brownish. (Yield 49.0%)

### Industrial Use of the Invention:

The production process for the compounds according to the present invention is as follows.

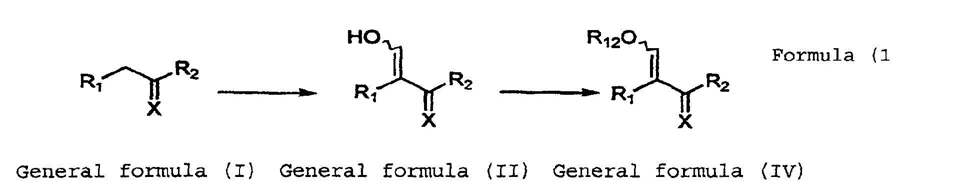

In the production process for the compounds represented by the general formula (IV) shown above, a step to formylate the compound represented by the general formula (I) (Step 1) and a step to convert the -OH group in the compound represented by the general formula (II) to OR" (Step 2) are included.

The step 1 is constituted by a step to react a compond represented by the general formula (I) with either a formic acid ester or an orthoformic acid ester in the presence of a Lewis acid and a base.

The step 2 is constituted with ① a step to react a compond represented by the genral formula (II) with either an alcohol represented by a formula of R"OH or with an alcohol represented by a formula of R"OH and an alkoxymethane represented by a formula of CH(OR")₃ in an acid condition and ② a step to stereoselectively synthesize a compond represented by the general formula (IV) in a bilayer solvent system and using a phase-transfer catalyst by means of specifying a type of the base and the concentration.

The process described above is more advantageous in comparison with the processes in the past in view of ① applicability for unstable compounds in a basic condition and capability for production in high efficiency, ② capability to selectively produce desired stereoisomers, and ③ capability to efficiently produce the compound represented by the general formula (IV) without requiring isolation of the compound represented by the general formula (II). Particularly, the processes described above are applicable for the production of various acrylic acid derivatives useful as agricultural chemicals, pharmaceutical ingredients and their intermediates and are highly useful for industrial uses.

## Claims

1. A process to produce compounds represented by a general formula (II) ; 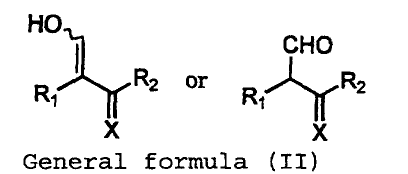 wherein R₁ represents hydrogen, halogeno, optionally substituted alkyl, optionally substituted alkoxy, a group having an alicyclic structure, a group repsented by R₃S(O)q, a group represented by R₄R₅N, a group represented by R₆C(=O), nitrile, nitro, a group represented by R₇C(=NR8), optionally substituted aryl, optionally substituted aryloxy, or optionally substituted aralkyl, R₂ represents optionally substituted alykl, optionally substituted alkoxy, a group having an alicyclic structure, optionally substituted amino, optionally substituted aryl, optionally substituted heterocyclic group or optionally substituted aralkyl, R₃, R₄ and R₅ each independently represents optionally substituted alkyl, optionally substituted aryl, optiobally-substituted heterocyclic group or optionally substituted aralkyl, R₆ and R₇ each independently represents optionally substituted alykl, optionally substituted alkoxy, a group having an alicyclic structure, optionally substituted amino, optionally substituted aryl, optionally substituted heterocyclic group or optionally substituted aralkyl, R₈ represents optionally substituted alkyl, optionally substituted alkoxy, nitrile, nitro, optionally-substitued aryl, optionally substituted heterocyclic group or optionally substituted aralkyl, q represents 0, 1 or 2, and R₉ and R₁₀ each independently represents hydrogen, lower alkyl or optionally substituted aryl, and R₁ and R₂ each represents a group which may bond to jointly form a ring,and X represents oxygen or a group represented by a formula of NR₉R₁₀, **characterized in that** the compound is subjected to a reaction with a methylene compound represented by a general formula (I); 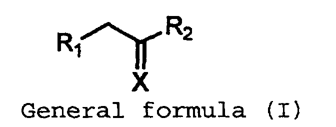 wherein R₁, R₂ and X are as defined above, with either a formic acid ester or an orthoformic acid ester in the presence of a Lewis acid and a base.

2. The production process according to claim 1, wherein the base is a tertiary amine.

3. The production process according to claim 1, wherein the group represented by R₁ in the general formula (I) is a group represented by the following formula; 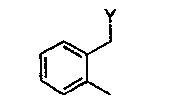 wherein Y represents a group to be eliminated when it is reacted with a nucleophilic reagent, optionally substituted phenoxy or optionally substituted heteroaryloxy, and the group represented by R₂ is a group represented by a formula of OR₁₁, wherein R₁₁ represents lower alkyl.

4. The production process according to claim 1, wherein the compound represented by the general formula (I) is methyl 2-[(2-isopropoxy-6-trifluoromethylpyrimidine-4-yl)oxymethyl] phenylacetate.

5. Compound represented the general formula (I), wherein the group represented by R₁ is a group represented by the following formula;
wherein E represents C₁₋₆ alkyl, C₁₋₆ haloalkyl, C₁₋₈ alkoxy, C₁₋₆ 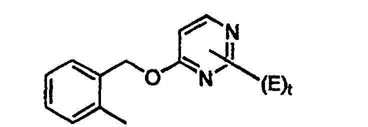 haloalkoxy, optionally substituted amino, a group represented by a formula of R₂₆S(O)p, wherein R₂₆ represents alkyl or aryl and p represents 0, 1 or 2, optionally substituted aralkyl, optionally substituted aryloxy, optionally substituted heterocyclic group, optionally substituted heteroaryloxy, a group having an alicyclic structure, nitrile, nitro, alkoxycarbonyl, formyl or carboxyl, t represents 0, 1, 2 or 3, provided E each represents a same or different group when t is 2 or more integer.

6. Compounds represented by the general formula (II), wherein the group represented by R₁ is a group represented by the following formula; 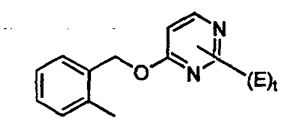 wherein E and t are as defined above.

7. A process for producing acrylic acid derivatives represented by a general formula (III); 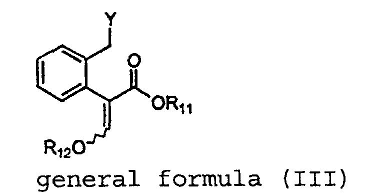 wherein Y and R₁₁ are as defined above, **characterized in that** the compound represented by the general formula (I), wherein R₁ represents a group represented by the following formula; 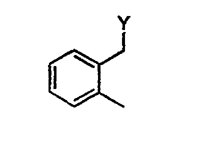 wherein Y is as defined above, R₂ is a group represented by a formula of OR₁₁, wherein R₁₁ is as defined above, and X represents oxygen, is formylated by using either a formic acid ester or an orthoformic acid ester in the presence of a Lewis acid and a base and then converted to the alkoxymethylene form.

8. The process for producing acrylic acid derivatives according to claim 7, wherein the base is a tertiary amine.

9. The process for producing acrylic acid derivatives according to claim 7, wherein the compound represented by the general formula (I) is methyl 2-[(2-isopropoxy-6 -trifluoromethylpyrimidine-4-yl)oxymethyl]phenylacetate and the compound represented by the general formula (III) is 3-methoxy-2-[2-{(2-isopropoxy-6 -trifluoromethylpyrimidine-4-yl)oxymethyl}phenyl]acrylic methyl.

10. A process for producing compounds represented by a general formula (IV); 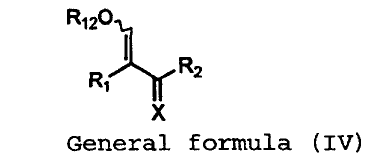 wherein R₁, R₂ and R₁₂ are as defined above, **characterized in that** the compounds are produced by reacting a formyl form represented by a general formula (II); 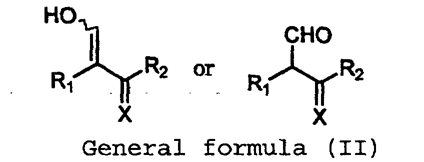 wherein R₁, R₂ and X are as defined above, with an alcohol represented by a formula of R₁₂OH, wherein R₁₂ is as defined above, in the presence of an acid catalyst

11. A process for producing compounds represented by a general formula (IV); 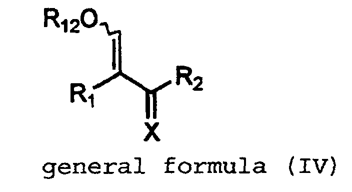 wherein R₁, R₂, R₁₂ and X are as defined above, **characterized in that** a formyl form represented by a general formula (II); 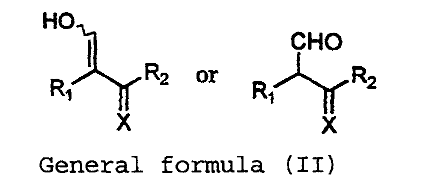 wherein R₁, R₂ and X are as defined above, is racted with an alcohol represented by a formula of R₁₂OH, wherein R₁₂ is as defined above, and an orthoformic acid ester represented by a formula of R₁₃C(OR₁₂)₃, wherein R₁₂ is as defined above and R₁₃ represents hydrogen, lower alkyl, cycloalkyl, haloalkyl or aralkyl, in the presence of an acid catalyst.

12. The production process according to claim 9 and claim10, wherein the group represented by R₁ in the compound represented by the general formula (II) is a group represented by the following formula: 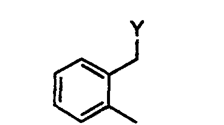 wherein Y is as defined above, and the group represented by a R₂ in the general formula (II) is a group represented by a formula of OR₁₁, wherein R₁₁ is as defined above.

13. The production process according to claim 10 and claim 11, wherein the compound represented by the general formula (II) is 3-hydroxy-2-[2-{(2-isopropoxy-6 -trifluoromethylpyrimidine-4yl)oxymethyl}phenyl]acrylic methyl.

14. A process for producing compounds represented by a general formula (VI-1); 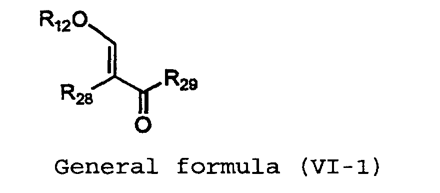 wherein R₂₈ represents optionally substituted alkyl, optionally substituted hydrocarbon containing an alicyclic structure, optionally substituted phenyl or optionally substituted heterocyclic group, R₂₉ represents C₁₋₆ alkyl, C₃₋₈ cycloalkyl, hydroxy, C₁₋₆ alkoxy, amino, a group represented by a formula of NHr₁, wherein r₁ represents C₁₋₆ alkyl, C₁₋₆ alkoxy or optionally substituted phenyl, a group represented by a formula of Nr₂r₃, wherein r₂ and r₃ each independently represents C₁₋₆ alkyl, C₁₋₆ alkoxy or optionally substituted phenyl, optionally substituted hydrocarbon containing an alicyclic structure, optionally substituted phenyl or optionally substituted heterocyclic group, and R₁₂ is as defined above, containing a step to O-alkylate a compound represented by a general formula (V); 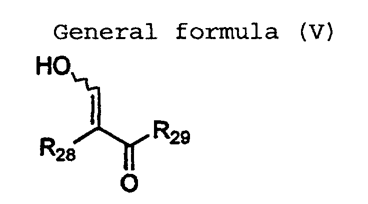 wherein R₂₈ and R₂₉ are as defined above, **characterized in that** the step to O-alkylate the compound represented by the general formula (V) contains a step to apply an alkylating agent to the compound represented by the general formula (V) in a bilayer mixed-solvent system consisting of an organic solvent and water in the presence of a phase-transfer catalyst and any of an alkali metal hydroxide excluding the lithium salt, an alkali metal carbonate excluding the lithium salt, an alkaline earth metal hydroxide and an alkaline earth metal carbonate while maintaining the concentration of the base in the aqueous solution at 10 wt% or lower.

15. A process for producing compounds represented by a general formula (VI-1); 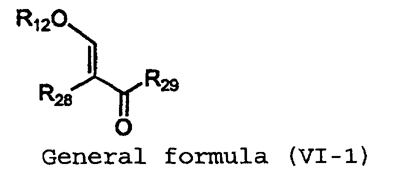 wherein R₂₈, R₂₉ and R₁₂ are as defined above, containing a step to O-alkylate a compound represented by a general formula (V) ; 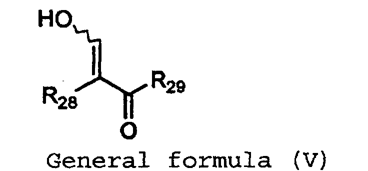 wherein R₂₈ and R₂₉ are as defined above, **characterized in that** the step to O-alkylate the compound represented by the general formula (V) contains a step to simultaneously feed dropwise an aqueous solution of any of an alkali metal hydroxide excluding the lithium salt, an alkali metal carbonate exclusing the lithium salt, an alkaline earth metal hydroxide and an alkaline earth metal carbonate, and an organic solvent solution of the compound represented by the general formula (V) into a bilayer mixed-solvent system consisting of an organic solvent containing an alkylating agent and a phase-transfer catalyst.

16. The process for producing compounds represented by the general formula (VI-1) according to claim 15, wherein the step to O-alkylate the compound represented by the general formula (V) is to O-alkylate a compound represented by the general formula (V) while maintaining the concentration of the base in the aqueous layer at 10 wt% or lower.

17. The process for producing compounds represented by the general formula (VI-1) according to claim 14 and claim 15, **characterized in that** the step to O-alkylate a compound represented by the genral formula (V) is to O-alkylate a compound represented by the general formula (V) while maintaining the concentration of the base in the aqueous layer at 6 wt% or lower.

18. A process for producing compounds represented by a general formula (VI-1); 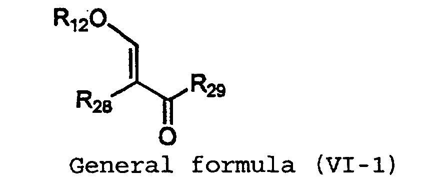 wherein R₂₈, R₂₉ and R₁₂ are as defined above, containing a step to O-alkylate a compound represented by a general formula (V); 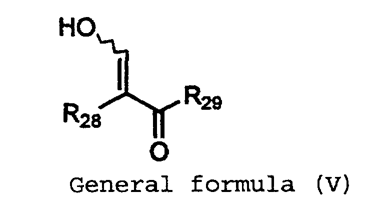 wherein R₂₈ and R₂₉ are as defined above, **characterized in that** the step to O-alkylate a compound represented by the general formula (V) contains a step to feed dropwise a solution of either the alkali metal salt or the alkaline earth meatl salt excluding the lithium salt of the compound represented by the general formula (V) into a bilayer mixed-solvent system consisting of an organic solvent solution, which contains an alkylating agent and a phase-transfer catalyst, and water.

19. The process for producing compounds represented by the general formula (VI-1) according to claim 18, **characterized in that** the step to O-alkylate a compound represented by the general formula (V) is a step to O-alkylate a compound represented by the general formula (V) while maintaining the concentration of the alkali metal salt or the alkaline earth metal salt excluding the lithium salt of the compound represented by the general formula (V) at 10 wt% or lower.

20. The process for producing compounds represented by the general formula (VI-1) according to any of claim 14 and claim15, **characterized in that** either sodium hydroxide or potassium hydroxide is used as the alkali metal hydroxide.

21. A process for producing compounds represented by a general formula (VI-2); 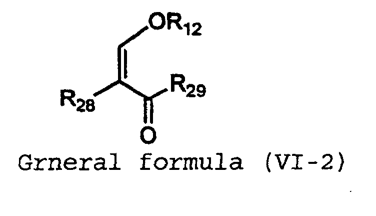 wherein R₂₈, R₂₉ and R₁₂ are as defined above, containing a step to O-alkylate a compound represented by a general formula (V); 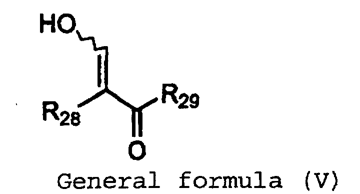 wherein R₂₈ and R₂₉ are as defined above, **characterized in that** the step to O-alkylate a compound represented by the general formula (V) contains a step to apply an alkylating agent to the compound represented by the general formula (V) in a bilayer mixed-solvent system consisting of an organic solvent and water in the presence of a phase-transfer catalyst and either lithium hydroxide or lithium carbonate.

22. The process for producing compounds represented by the general formula (VI-2) according to claim 21, **characterized in that** the step to O-alkylate a compound represented by the general formula (V) is a step to O-alkylate the compound represented by the general formula (V) while maintaining the concentration of either the lithium hydroxide or the lithium carbonate in the aqueous layer at 5 wt% or higher.

23. The process for producing compounds represented by the general formula (VI-1) according to any of claims 14, 15, 17 and 21, wherein a quartenary ammonium salt is used as the phase-transfer catalyst.

24. The process for producing compounds represented by the general formula (VI-1) according to any of claims 14, 15, 17 and 21, wherein a quartenary ammonium hydroxide is used as the phase-transfer catalyst.

25. A process for producing compounds represented by a general formula (XII); 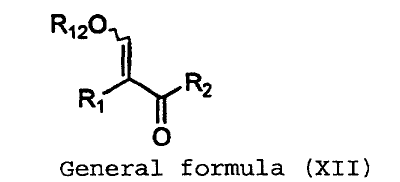 wherein R₁, R₂ and R₁₂ are as defined above, **characterized in that** the compound represented by a general formula (XII) is produced by reacting a tertiary amine compound represented by a general formula (VIII); 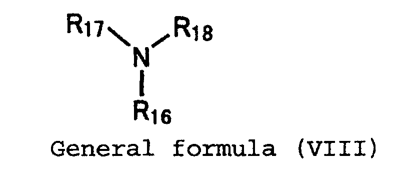 wherein R₁₆, R₁₇ and R₁₈ may be same or different and represents alkyl, aryl or aralkyl, and an organic silica compound represented by a general formula (IX); 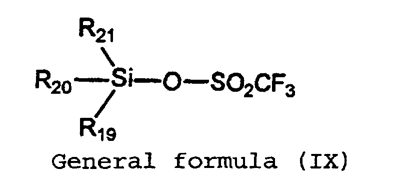 wherein R₁₉, R₂₀ and R₂₁ may be same or different and represents alkyl, aryl or aralkyl with a compound represented by a general formula (VII); 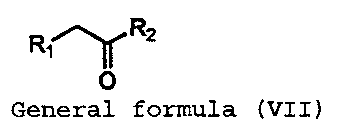 where in R₁ and R₂ are as defined above, and then reacting an orthoformic acid ester compound represented by a general formula of (XI)CH(OCR₁₂)₃, wherein R 12 is as defined above, with a silylenol ether represented by a general formula (X); 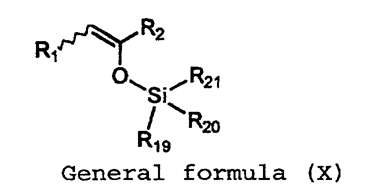 wherein R₁, R₂, R₁₉, R₂₀ and R₂₁ are as defined above, in the presence of a Lewis acid.

26. The production process according to claim 25, wherein the group represented by R₁ in the compound represented by the general formula (VII) is a group represented by the following formula; 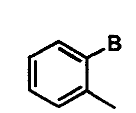 wherein B represents hydrogen, lower alkyl, lower alkoxy, haloalkyl, optionally substituted arylsulfonyloxyalkyl or optionally substituted lower alkylsulfonyloxyalkyl, and the group represented by R₂ is a group represented by a formula of OR₂₃, wherein R₂₃ represents lower alkyl, and B and R₂₃ are a group which may bond to jointly form a ring.

27. A process for producing α -alkoxymethylenecarbonyl compounds represented by a general formula (XV); 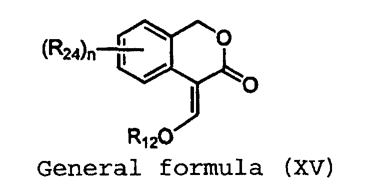 wherein R₂₄ represents nitro, cycno, halogeno, C₁₋₆ alkyl, C₁₋₆ alkoxy, C₁₋₆ haloalkyl, or C₁₋₆ alkoxycarbonyl, R₁₂ is as defined above and n represents 0 or an integer of 1 to 4, and each of R₂₄ may be same or different when n is 2 or more integer, containing a step obtain an α-hydroxymethylenecarbonyl compound represented by a general formula (XIV); 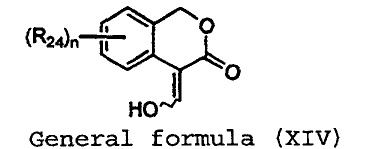 wherein R₂₄ and n are as defined above, by formylating an isochromanone compound represented by a general formula (XIII); 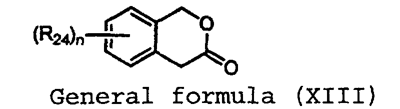 wherein R₂₄ and n are as defined above, and a step to O-alkylate a compound represented by the general formula (XIV) in a bilayer mixed-solvent system consisting of an organic solvent and water in the presence of a phase-transfer catalyst and a base, without isolating the compound represented by the general formula (XIV).

28. The process for producing compounds represented by the general formula (XV) according to claim 27, **characterized in that** the step to formylate the isochromanone compound represented by the general formula (XIII) is a step to formylate the compound represented by the general formula (XIII) by using an formic acid ester.

29. An after-treatment process in a step to produce compounds represented by a general formula (II); 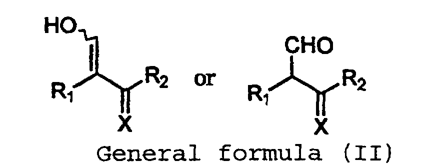 wherein R₁, R₂ and X are as defined above, by reacting a methylene compound represented by a general formula (I); 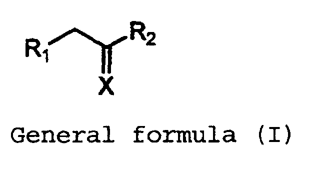 wherein R₁, R₂ and X are as defined above, with eithe an formic acid ester or an orthoformic acid ester in the presence of a Lewis acid and a base, **characterized in that** the after-treatment process contains a step to add water following to an addition of C₁₋₄ organic acid into the reacted solution to improve the separating property of the solution.

30. The after-treatment process according to claim 29 **characterized by** using the C₁₋₄ organic acid in an amount of 2.5 times mole or more of the the Lewis acid to be used.

31. The after-treatment process according to claim 29, wherein the C₁₋₄ organic acid is acetic acid.

32. The after-treatment process according to claim 29, wherein the Lewis acid is titanium tetrachloride.

33. The after-treatment process according to claim 29, wherein the base is triethylamine.

34. The after-treatment process according to claim 29, wherein the group represented by R₁ in the compound represented by the general formula (I) is a group represented by the following formula; 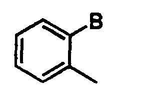 wherein B is as defined above, and the group represented by R₂ is a group represented by a formula of OR₂₃, wherein R₂₃ is as defined above, and each of B and R₂₃ are a group which may bond to jointly form a ring.
